# EUROPEAN PATENT APPLICATION

(11) **EP 4 840 884 A1**
(43) Date of publication of application: **18.11.2026**
(21) Application number: 25738556.7
(22) Date of filing: 10.01.2025
(51) Int. Cl.: C07K 16/28, A61K 39/00, A61P 35/00

(54) **ANTI-CD28 ANTIBODY AND USE THEREOF**

(30) Priority: 12.01.2024 CN 202410047645
(71) Applicant: Innovent Biologics (Suzhou) Co., Ltd., Suzhou, Jiangsu 215123 (CN)
(72) Inventor: XIONG, Huizhong, Suzhou, Jiangsu 215123 (CN); ZHANG, Xiguang, Suzhou, Jiangsu 215123 (CN); LI, Li, Suzhou, Jiangsu 215123 (CN); ZHOU, Shuaixiang, Suzhou, Jiangsu 215123 (CN)
(74) Representative: Sagittarius IP
(86) International application number: PCT/CN2025/071730
(87) International publication number: WO 2025/149029

(57) **Abstract**

An antibody or an antigen-binding fragment thereof. The antibody or an antigen-binding fragment thereof can specifically bind to a CD28 protein, such as human CD28 protein. Also provided is a multispecific binding molecule, such as a multispecific antibody, that specifically binds to CD28 and one or more tumor-associated antigens. Further provided are a nucleic acid molecule encoding the antibody, an expression vector for expressing the antibody, a host cell and a preparation method therefor. Also provided are methods of diagnosis and treatment using the antibody.

## Description

### Cross-Reference to Related Applications

The present application is based on and claims priority to Chinese Patent Application No. 202410047645.4 filed on Jan. 12, 2024, which is incorporated herein by reference in its entirety.

The present invention provides an antibody or an antigen-binding fragment thereof that can specifically bind to a CD28 protein, e.g., a human CD28 protein. The present invention also provides a multispecific binding molecule, e.g., a multispecific antibody, that specifically binds to CD28 and one or more tumor-associated antigens. The present invention further provides a nucleic acid molecule encoding the antibody, an expression vector for expressing the antibody, a host cell, and a preparation method therefor. The invention also provides methods of diagnosis and treatment using the antibodies of the invention.

### Background of the Invention

T cell activation involves stimulation of highly specific T cell receptors (TCRs) by antigen-presenting cells such as dendritic cells that present their specific antigens on class II major histocompatibility (MHC) complexes, and can also be promoted by costimulatory molecules such as CD28. CD28 is a 44 KDa disulfide-linked homodimeric receptor glycosylated at five different sites. CD28 is expressed on T cells (95% of resting CD4+ cells and 50% of resting CD8+ T cells in human peripheral blood) and plasmablasts, and provides costimulatory signals required for T cell activation and survival. Co-stimulation occurs when CD28 on the T cells surface binds to CD80 (B7-1) and CD86 (B7-2) on antigen-presenting cells. CD80 expression is upregulated on antigen-presenting cells (APCs) upon their activation, whereas CD86 is constitutively expressed on APCs. CD28 co-stimulation of T helper cells enhances transcription of IL2R and IL2, leading to T cell proliferation; induces expression of Bcl XL, leading to T cell survival; and increases production of IL-4 (leading to Th2 differentiation), IFNγ, IL-1, TNF, IL-5, various chemokines and their receptors.

In addition, CD28 induces the expression or upregulation of several other co-stimulatory and regulatory molecules, including ICOS, 4-1BB, and CTLA-4, as well as the CD40L molecule that is essential for T-cell and B-cell interactions.

Several monoclonal antibodies against CD28 are known in the art, such as TGN1412 (Tegenero, US20060188493A1). It is a CD28 superagonist monoclonal antibody that preferentially activates regulatory T (Treg) cells in the absence of TCR co-stimulation. However, when this antibody was used for therapeutic purposes involving T cell activation, the phase I clinical trial resulted in rapid multiorgan failure, i.e., a severe cytokine release syndrome (cytokine storm), in all six volunteers.

Therefore, there remains a need in the art to develop safe anti-CD28 antibodies suitable for use as medicaments, as well as multispecific antigen-binding molecules constructed based thereon that specifically bind to CD28 and other target antigens, for use in therapy.

### Summary of the Invention

One aspect of the present invention relates to an anti-CD28 antibody or an antigen-binding fragment thereof.

In some embodiments, the anti-CD28 antibody or the antigen-binding fragment thereof of the present invention specifically binds to CD28 (e.g., human CD28). In some embodiments, the anti-CD28 antibody or the antigen-binding fragment thereof of the present invention binds to CD28 (e.g., human CD28) with a binding affinity (K_{D}) of less than or equal to about 50 nM, 45 nM, 40 nM, 35 nM, 30 nM, 25 nM, 20 nM, 18 nM, 15 nM, 10 nM, 9 nM, 8 nM, 7 nM, 6 nM, 5 nM, 4 nM, 3 nM, 2 nM, 1 nM, 0.9 nM, 0.8 nM, 0.7 nM, or 0.6 nM, or a value therebetween. In some embodiments, the anti-CD28 antibody or the antigen-binding fragment thereof of the present invention binds to CD28 (e.g., human CD28) with a binding affinity (K_{D}) of greater than or equal to about 0.5 nM.

In some embodiments, the anti-CD28 antibody or the antigen-binding fragment thereof of the present invention is capable of activating the CD28 signaling pathway.

In some embodiments, the anti-CD28 antibody or the antigen-binding fragment thereof of the present invention is capable of activating T cells (e.g., human peripheral T cells, such as CD4 or CD8 T cells), for example, activating T cells to release cytokines, e.g., to release TNF (e.g., TNFα) or interleukins (e.g., IL2).

In some embodiments, the anti-CD28 antibody or the antigen-binding fragment thereof of the present invention is capable of activating human T cells, e.g., human peripheral T cells, to kill tumor cells.

Another aspect of the present invention relates to a multispecific binding molecule (e.g., a multispecific antibody, such as a bispecific antibody) constructed based on the CD28 antibody of the present invention, comprising a target-binding region from the anti-CD28 antibody of the present invention, and optionally one or more additional target-binding regions (antigen-binding regions), e.g., those specifically bind to one or more tumor-associated antigens (TAAs).

In one embodiment, the present invention relates to a multispecific antibody, e.g., a bispecific antibody, that specifically binds to CD28 and one or more TAAs. In some embodiments, the TAA is CLDN18.2 or PSMA, e.g., human CLDN18.2 or human PSMA.

In one embodiment, the present invention relates to a bispecific antibody specifically binding to CD28 (e.g., human CD28) and CLDN18.2 (e.g., human CLDN18.2).

In one embodiment, the present invention relates to a bispecific antibody specifically binding to CD28 (e.g., human CD28) and PSMA (e.g., human PSMA).

In some embodiments, the bispecific antibody of the present invention is capable of effectively activating the CD28 signaling pathway.

In some embodiments, the bispecific antibody of the present invention is capable of activating human peripheral T cells, e.g., activating T cells (e.g., CD4 or CD8 T cells) to release cytokines, e.g., TNF(such as TNFα) or interleukins(e.g., IL2).

In some embodiments, the bispecific antibody of the present invention is capable of activating human T cells, e.g., activating human peripheral T cells, to kill tumor cells.

In some embodiments, the bispecific antibody of the present invention can specifically bind to CD28 (e.g., human CD28) and TAA (e.g., human TAA, e.g., CLDN18.2 or PSMA).

In some embodiments, the bispecific antibody of the present invention, e.g., a bispecific antibody specifically binding to CD28 and CLDN18.2 or to CD28 and PSMA, has anti-tumor activity, e.g., *in vivo* anti-tumor activity.

In some embodiments, the bispecific antibody of the present invention can be combined with other therapeutic agents (e.g., other antibodies such as anti-PD1 antibodies) for anti-tumor use.

### Brief Description of the Drawings

FIG. 1: Assay results for Jurkat-NF-κB activation activity of anti-CD28 chimeric antibodies;
FIG. 2: Activation results of human peripheral T cells by anti-CD28 humanized antibodies, A-B: in the absence of CD3 antibody, C-D: in the presence of CD3 antibody;
FIG. 3: Schematic diagram of a CD28 bispecific antibody;
FIG. 4: Activation results of NFkB Jurkat Reporter by CLDN18.2×CD28 bispecific antibodies;
FIG. 5: Results of CLDN18.2×CD28 bispecific antibody-mediated killing of tumor cells by human peripheral T cells;
FIG. 6: Results of of cytokine release from peripheral T cells activated by CLDN18.2×CD28 bispecific antibodies , A: in the absence of CD3 antibody, B: in the presence of CD3 antibody;
FIG. 7: Results of cell surface affinity assay of PSMA×CD28 bispecific antibodies, A: PSMA affinity, B: CD28 affinity;
FIG. 8: Activation results of NFkB Jurkat Reporter by PSMA×CD28 bispecific antibodies, A: with addition of CD3 antibody, B: with addition of PSMA×CD3 bispecific antibody;
FIG. 9: Results of the PSMA×CD28 bispecific antibody-mediated killing of tumor cells by human peripheral T cells;
FIG. 10: The Results of cytokine IL2 release from peripheral T cells activated by PSMA×CD28 bispecific antibodies , A: in the absence of CD3 antibody (-signal 1), B: in the presence of CD3 antibody (+signal 1);
FIG. 11: Results of *in vivo* single-drug efficacy study of PSMA ×CD28 bispecific antibodies as monotherapy, A: dosing regimen; b: assay results;
FIG. 12: Results for the *in vivo* efficacy study of the PSMA×CD28 bispecific antibody in combination with an anti-PD1 antibody, A: dosing regimen; b: assay results.

### Detailed Description of the Invention

It is to be understood that the present invention is not limited to the particular methods, protocols, examples, and reagents described herein, as these may vary. It is also to be understood that the terminology used herein are merely intended to describe particular embodiments rather than limiting the scope of the present invention, which will be limited only by the appended claims.

### I. Definitons

Unless otherwise defined, all technical and scientific terms used herein have the same meaning as commonly understood by those of ordinary skill in the art to which the present invention belongs.

For the purpose of explaining this specification, the following definitions will be used, and wherever appropriate, terms used in the singular form may also include the plural form, and vice versa. It is to be understood that the terminology used herein is for the purpose of describing particular embodiments only and is not intended to be limiting.

The term "about" used in combination with a numerical value is intended to encompass a range of numerical values having a lower limit that is 5% (e.g., 4%, 3%, 2% or 1%) less than the specified numberical value and an upper limit that is 5% (e.g., 4%, 3%, 2% or 1%) greater than the specified numerical value.

As used herein, the term "and/or" refers to any one of the options, or any two or more of the options, or all of the options.

When referring to "first" and "second" herein, it is only to distinguish two domains or two chains, and does not indicate the location of the two domains in any way.

As used herein, the term "comprise" or "include" is intended to mean that the described elements, integers or steps are included, but not to the exclusion of any other elements, integers or steps. The term "comprise" or "include" used herein, unless otherwise indicated, also encompasses instances consisting of the described elements, integers or steps. For example, when referring to an antibody variable region "comprising" a particular sequence, the term is also intended to encompass an antibody variable region consisting of the particular sequence.

The term "CD28" as used herein refers to an antigen expressed on T cells as a costimulatory receptor. Human CD28 comprises, for example, the amino acid sequence set forth in NCBI accession No. NP_006130.1. In some embodiments, CD28 is human CD28.

As used herein, an "antibody binding to CD28", "anti-CD28 antibody" or "antibody specifically binding to CD28" refers to an antibody capable of binding to monomeric CD28 or an extracellular domain thereof, or binding to dimeric CD28 or an extracellular domain thereof, with an appropriate affinity.The antibody and the antigen-binding fragment of the present invention can bind to soluble CD28 and/or CD28 expressed on the cell surface.Soluble CD28 includes native CD28 proteins as well as recombinant CD28 protein variants, such as monomeric and dimeric CD28 constructs, which lack a transmembrane domain or are otherwise independent of the cell membrane.

In some aspects, the anti-CD28 antibody described herein also encompasses multispecific antibodies such as bispecific antibodies that specifically bind to both CD28 and other target antigens.

General information on the amino acid sequences and nucleotide sequences of the human immunoglobulin light and heavy chains is given in Kabat, E.A. et al., Sequences of Proteins of Immunological Interest, 5th Ed., Public Health Service, National Institutes of Health, Bethesda, MD (1991).

The multispecific antibody of the present invention may comprise a linker. The term "linker" as used herein refers to any molecule that enables direct connection of different portions of a multispecific antibody. Examples of linkers to establish covalent linkages between different portions of a multispecific antibody include peptide linkers and non-proteinaceous polymers including, but not limited to, polyethylene glycol (PEG), polypropylene glycol, polyalkylene oxide, or copolymers of polyethylene glycol and polypropylene glycol. In some embodiments, the term "peptide linker" according to the present invention refers to an amino acid sequence, wherein the sequence links the amino acid sequences of various portions of a multispecific antibody together. Preferably, the peptide linker is of a length sufficient to link two entities in such a way that they maintain their conformation relative to each other without interfering with the desired activity. The peptide linker may or may not predominantly comprise the following amino acid residues: Gly, Ser, Ala or Thr.

The term "Fc domain" or "Fc region" is used herein to define a C-terminal region of an immunoglobulin heavy chain that contains at least a portion of the constant region. The term includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and third constant domains (CH2 and CH3 domains) derived from two heavy chains of IgG, IgA, and IgD antibodies; or comprises the second, third, and fourth constant domains (CH2, CH3, and CH4 domains) derived from two heavy chains of IgM and IgE antibodies. Unless otherwise stated herein, amino acid residues in the Fc region or heavy chain constant region are numbered according to the EU numbering scheme (also known as the EU index) described in, for example, Kabat et al., Sequences of Proteins of Immunological Interes, 5th Ed., Public Health Service, National Institutes of Health, Bethesda, MD, 1991. However, the C-terminal lysine (Lys447) of the Fc region may or may not be present. Two Fc regions can form a dimeric Fc by dimerization, and two different Fc form a heterodimeric Fc by heterodimerization. Herein, the terms "Fc region", "Fc portion", and "dimeric Fc (e.g., heterodimeric Fc)" do not comprise a heavy chain variable region VH and a light chain variable region VL as well as a heavy chain constant region CH1 and a light chain constant region CL of an immunoglobulin, but in some cases, they may comprise a hinge region at the N-terminus of the heavy chain constant region. In one embodiment, a human IgG heavy chain Fc region extends from Asp221 or from Cys226 or from Asp231 to the carboxyl-terminus of the heavy chain.

In one embodiment, the human IgG1 Fc region polypeptide (comprising a portion of hinge region) comprises or consists of the following amino acid sequence: 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 94);
 or

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK (SEQ ID NO: 95).

In one embodiment, the Fc region is derived from a human Fc region. In one embodiment, the Fc region comprises all or part of a human constant region. The antibody Fc region is directly involved in complement activation, C1q binding, C3 activation, and Fc receptor binding. In one embodiment, the Fc region is a human Fc region. In one embodiment, the Fc region belongs to a human IgG4 subclass. In one embodiment, the Fc region belongs to a human IgG1 subclass. In one embodiment, the Fc region is an Fc region of human IgG1, IgG2, IgG3, or IgG4.

Herein, a "heterodimeric Fc scaffold" refers to a scaffold comprising two different Fc regions or formed by the dimerization of two different Fc regions, which may be linked to an antigen-binding domain at its N-terminus or C-terminus (e.g., a heavy and/or light chain variable region of an antibody or an antigen-binding fragment of an antibody that can bind to a target molecule, or a soluble moiety of a ligand or receptor that can bind to a target molecule) to constitute a multispecific antibody, e.g., a bispecific antibody.

The term "CH1 region" refers to a portion of an antibody heavy chain polypeptide that extends from EU position 118 to EU position 220 (EU numbering scheme). In one embodiment, the CH1 domain comprises or consists of an amino acid sequence of

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSC (SEQ ID NO: 74).

In some embodiments, the CH1 may comprise a portion of hinge region. In some embodiments, the Fc region may comprise a portion of hinge region.

Herein, specific amino acid residues of antibody IgG constant regions are described according to the EU numbering scheme. For example, "S364" refers to a serine at EU position 364. An amino acid mutation at a specific position in the constant region is indicated by "original amino acid, amino acid position, mutated amino acid". For example, "S364R" refers to a substitution of serine (S) at EU position 364 with arginine (R). When describing combinations of mutations, the combined mutations are connected by a plus (+) or "-" or "and". "S364R+D399K", "S364R-D399K", or "S364R and D399K" indicates that the Fc region comprises both mutations S364R and D399K. When there is a possibility of multiple mutations at a particular position, it is indicated herein by the symbol "/". For example, the mutation "K370T/S" indicates that residue K at position 370 may be substituted with T or S residue.

For polypeptide sequences, "conservative alterations" include substitutions, deletions, or additions to the polypeptide sequence that do not substantially alter the desired functional activity of the polypeptide sequence. For example, conservative substitutions often involve in the replacement of an amino acid with a chemically similar one. Tables of conservative substitution providing functionally similar amino acids are well known in the art. Listed below are eight groups of amino acids that are conservative substitutionsfor each other : 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence alteration" is used to refer to an amino acid modification that does not significantly affect or change the binding characteristics for an antigen of interest of the antibody molecule or binding protein molecule of the present invention comprising the amino acid sequence. For example, conservatively modified variants retain at least 80%, 85%, 90%, 95%, 98%, 99% or higher, e.g., 100%-110% or higher, binding affinity for an antigen of interest relative to the parent antibody or binding protein.

The term "vector" as used herein refers to a nucleic acid molecule capable of proliferating another nucleic acid to which it is linked. The term includes vectors that serve as self-replicating nucleic acid structures as well as vectors that incorporate into the genome of a host cell into which they have been introduced. Some vectors are capable of directing the expression of a nucleic acid to which they are operably linked. Such vectors are referred to herein as "expression vectors."

The term "binding molecule" refers to any molecule capable of specifically binding to a target, e.g., an antibody or an antigen-binding fragment thereof or a fusion protein.

The term "target" refers to the bound substance against which the binding molecule is directed. The target may be an antigen, or may be a ligand or receptor. The term "antigen" refers to a molecule that elicits an immune response. This immune response may involve antibody production or activation of specific immune cells, or both. The skilled artisan will appreciate that any macromolecule, including substantially all proteins or peptides, can be used as an antigen. Further, antigens may be derived from recombinant or genomic DNA. As used herein, the term "epitope" refers to a moiety of an antigen that specifically interacts with an antibody molecule. "Target" and "antigen" are used interchangeably when the binding molecule of the present invention relates to a target-binding region derived from an antibody. In some embodiments, the antigen is a tumor-associated antigen (TAA).

The term "tumor-associated antigen (TAA)" described herein preferably relates to a protein that is specifically expressed in a limited number of tissues and/or organs under normal conditions or in a specific developmental stage, and is expressed or abnormally expressed in one or more tumor or cancer tissues. In the context of the present invention, tumor-associated antigens are preferably associated with the cell surface of cancer cells and are preferably not expressed or only rarely expressed in normal tissues. In some embodiments, the tumor-associated antigen is a human tumor-associated antigen. In some embodiments, the tumor-associated antigen is CLDN18.2, such as human CLDN18.2, or PSMA, such as human PSMA.

The term "target-binding region" as used herein refers to a portion of a binding molecule, e.g., a multispecific binding molecule or a bispecific binding molecule, that binds to a particular target or antigen. The target-binding region may be, for example, an antibody or immunoglobulin per se or an antibody fragment. Such target-binding regions may or may not have a tertiary structure independent of the remainder of the binding molecule, and may or may not bind to their targets as separate entities. The target-binding region may also be a receptor or a ligand, or a domain of a receptor capable of binding to a ligand. In the case of multispecific antibodies or bispecific antibodies, the "target-binding region" is also referred to as the "antigen-binding region".

The term "antigen-binding region" as used herein refers to any portion of an antibody or an antigen-binding fragment thereof, e.g., a multispecific antibody or a bispecific antibody, that binds to a particular target or antigen. The antigen-binding region may be, for example, an antibody or immunoglobulin per se or an antibody fragment. Such antigen-binding region may or may not have a tertiary structure independent of the remainder of the multispecific antibody or bispecific antibody, and may or may not bind to its antigen/epitope as a separate entity. When the binding molecule of the present invention relates to a target-binding region derived from an antibody, "target-binding region" and "antigen-binding region" are used interchangeably.

The term "multispecific binding molecule" refers to a multispecific binding molecule that is at least bispecific, e.g., a bispecific binding molecule, that is, the molecule comprises at least a first target-binding region and a second target-binding region, wherein the first target-binding region binds to one target or antigen and the second target-binding region binds to another antigen or target. The multispecific binding molecule according to the present invention also encompasses a multispecific binding molecule comprising a plurality of target-binding regions/sites. In some embodiments, the multispecific binding molecule of the present invention is a multispecific antibody. In some embodiments, the bispecific binding molecule of the present invention is a bispecific antibody.

As used herein, the term "monospecific" refers to a polypeptide/protein molecule having one or more target-binding regions, each of which binds to the same site or structure of the same target or the same epitope of the same antigen.

As used herein, the term "multispecific" binding molecule, e.g., an antibody, refers to a binding molecule having at least two target-binding regions, each of which binds to a different site/structure of the same target or a different target. A multispecific binding molecule is a binding molecule having binding specificities for at least two different targets or sites/structures. In one embodiment, provided herein is a bispecific binding molecule having binding specificity for a first target and a second target.

As used herein, the term "multispecific" antibody refers to an antibody having at least two antigen-binding regions, each of which binds to a different epitope of the same antigen or a different epitope of a different antigen. A multispecific antibody is an antibody having binding specificity for at least two different antigens or epitopes. In one embodiment, provided herein is a bispecific antibody having binding specificity for a first antigen and a second antigen.

When referring to a "first antigen-binding region" in a multispecific antibody or a bispecific antibody, it refers to a binding region that binds to a first antigen and is not intended to limit the number of such antigen-binding regions contained in the antibody. For example, the multispecific antibody or the bispecific antibody may comprise one or more first antigen-binding regions. For example, the bispecific antibody comprises a first antigen-binding region and a second antigen-binding region, but may comprise one or more first antigen-binding regions and one or more second antigen-binding regions.

When referring to "first target-binding region" in a binding molecule, it refers to a binding region that binds to a first target and is not intended to limit the number of such target-binding regions contained in the binding molecule. For example, the multispecific binding molecule or bispecific binding molecule may comprise one or more first target-binding regions. For example, the bispecific binding molecule comprises a first target-binding region and a second target-binding region, and may comprise one or more first target-binding regions and one or more second target-binding regions.

When it is mentioned that "a target or an antigen-binding region is derived from an antibody", it is meant that the binding domain constituting the target/antigen-binding region is or is derived from a binding domain of the antibody that specifically binds to an antigen. For example, a fragment of the antigen-binding region that specifically binds to an antigen, such as a Fab, is or is derived from a corresponding fragment of the antibody, such as a Fab; or the heavy chain variable region and/or the light chain variable region of the antigen-binding region is or is derived from a heavy chain variable region and/or a light chain variable region of the antibody; or 1, 2, 3, 4, 5, or 6 CDRs of the antigen-binding region are CDRs of the antibody.

The term "derived from" means that the fragment in the antigen-binding region is substantially identical to the fragment of the antibody from which it is derived, but has a mutation, such as a substitution, deletion, or addition, at one or more sites. In one specific embodiment, the mutation is not in a CDR of the antibody.

The terms "whole antibody" and "full-length antibody" are used interchangeably herein to refer to an antibody molecule having the structure of a native immunoglobulin molecule. In the case of a conventional four-chain IgG antibody, a full-length antibody comprises two heavy chains (H) and two light chains (L) interconnected by disulfide bonds. In the case of a heavy chain antibody having only heavy chains but lacking light chains, a full-length antibody comprises two heavy chains (H) interconnected by disulfide bonds. For a conventional four-chain IgG antibody, the full-length antibody heavy chain generally consists of a heavy chain variable region (abbreviated herein as VH) and a heavy chain constant region, wherein the heavy chain constant region comprises at least 3 domains CH1, CH2, and CH3. The full-length antibody light chain consists of a light chain variable region (abbreviated herein as VL) and a light chain constant region, wherein the light chain constant region consists of one domain CL. Each heavy chain variable region VH or each light chain variable region consists of three CDRs and 4 FRs arranged from amino-terminus to carboxyl-terminus in the following order: FR1, CDR1, FR2, CDR2, FR3, CDR3, and FR4. The term "antibody fragment" includes a portion of an intact antibody. In a preferred embodiment, the antibody fragment is an antigen-binding fragment.

The term "antigen-binding fragment" of an antibody refers to a molecule different from a full-length antibody, wherein the antigen-binding fragment comprises a portion of the full-length antibody, but is capable of binding to an antigen of the full-length antibody or competing with the full-length antibody (i.e., a full-length antibody from which the antigen-binding fragment is derived) for binding to an antigen. An antigen-binding fragment may be prepared by recombinant DNA techniques, or by enzymatic or chemical cleavage of an intact antibody. The antigen-binding fragment includes, but is not limited to, a Fab, a Fab', a F(ab')₂, a Fv, a single-chain Fv, a diabody, a single-domain antibody (sdAb), and a nanobody. For example, a Fab fragment can be obtained by papain digestion of a full-length antibody. In addition, the F(ab')₂, a dimer of the Fab', is a bivalent antibody fragment produced by pepsin digestion at a site below the disulfide bonds in a hinge region of an intact antibody. The F(ab')₂ can be reduced by disrupting the disulfide bonds in the hinge region under neutral conditions, thereby converting the F(ab')₂ dimer into a Fab' monomer. The Fab' monomer is essentially a Fab fragment with a hinge region. The Fv fragment consists of the VL and VH domains of a single arm of an antibody. The two domains VL and VH of the Fv fragment can be encoded by separate genes, but the two domains can also be linked, using recombinant methods, via a synthetic linker peptide so that they are produced as a single protein chain in which the VL and VH regions pair to form a single-chain Fv (scFv).

"Fab fragment" and "Fab" are used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. Herein, a Fab polypeptide chain comprising a heavy chain constant region CH1 is also referred to as a "Fab heavy chain"; correspondingly, a Fab polypeptide chain comprising a light chain constant region CL is also referred to as a "Fab light chain".

"Complementarity-determining region" or "CDR region" or "CDR" is a region in an antibody variable domain that is highly variable in sequence and forms a structurally defined loop ("hypervariable loop") and/or comprises antigen-contacting residues ("antigen contact site"). The CDRs are primarily responsible for binding to antigenic epitopes. The CDRs of heavy and light chains are generally referred to as CDR1, CDR2, and CDR3, and are numbered sequentially from the N-terminus. The CDRs located in the heavy chain variable domain of the antibody are referred to as CDRH1, CDRH2, and CDRH3, while the CDRs located in the light chain variable domain of the antibody are referred to as CDRL1, CDRL2, and CDRL3. In a given amino acid sequence of a light chain variable region or a heavy chain variable region, the exact amino acid sequence boundary of each CDR can be determined using any one or a combination of many well-known antibody CDR assignment schemes including, e.g., Chothia based on the three-dimensional structure of antibodies and the topology of the CDR loops (Chothia et al. (1989) Nature 342: 877-883; Al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (www.imgt.cines.fr/), and North CDR definition based on the affinity propagation clustering using a large number of crystal structures. Unless otherwise stated, the term "CDR" or "CDR sequence" used herein encompasses CDR sequences determined by any one of the schemes described above or a combination thereof. CDRs can also be determined based on having the same Kabat numbering positions as a reference CDR sequence (e.g., any of the exemplary CDRs of the present invention).

In some embodiments, the CDRs of the heavy chain variable region of the antibody of the present invention are each defined according to Kabat or Chothia, or a combination of Kabat and Chothia (hereinafter referred to as "Kabat & Chothia"; corresponding to H26-H32 under the Kabat numbering system). In some embodiments, the CDRs of the light chain variable region of the antibodies of the present invention are determined according to Kabat.

In one embodiment, in the anti-CD28 antibody of the present invention, CDRH1 is defined according to the Kabat & Chothia scheme, CDRH2 and CDRH3 are each defined according to the Kabat scheme, and CDRLs are each defined according to the Kabat scheme.

| CDR | Kabat scheme | Chothia scheme | Kabat & Chothia |
|---|---|---|---|
| **CDRL**1 (Kabat numbering system) | L24-L34 | | |
| **CDRL**2 (Kabat numbering system) | L50-L56 | | |
| **CDRL**3 (Kabat numbering system) | L89-L97 | | |
| **CDRH**1 (Kabat and Chothia numbering systems) | H31-H35 | H26-H32 | H26-H35 |
| **CDRH**2 (Kabat numbering system) | H50-H65 | | |
| **CDRH**3 (Kabat numbering system) | H95-H102 | | |

The percent identity between sequences is calculated as follows.

To determine the percent identity of two amino acid sequences or two nucleic acid sequences, the sequences are aligned for optimal comparison purposes (e.g., gaps can be introduced into one or both of the first and second amino acid sequences or nucleic acid sequences for optimal alignment, or non-homologous sequences can be disregarded for comparison). In a preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. The amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

"Humanized antibody" is an antibody that retains the antigen-specific reactivity of a non-human antibody and has lower immunogenicity when administered to humans as a therapeutic agent. This can be achieved, for example, by retaining non-human antigen-binding sites and substituting the remainder of the antibodies with their human counterparts (i.e., the portions of the variable regions not involved in binding are substituted with the corresponding parts of human antibodies).

As used herein, the term "anti", "binding", or "specific binding" means that the binding effect is selective for targets or antigens and can be distinguished from unwanted or non-specific interactions. The ability of a binding site to bind to a particular target or antigen may be determined by flow cytometry or enzyme-linked immunosorbent assay (ELISA) or conventional binding assays known in the art, such as radioimmunoassay (RIA), bio-layer interferometry, MSD assay, or surface plasmon resonance (SPR).

"Affinity" or "binding affinity" refers to the inherent binding affinity that reflects the interaction between members of a binding pair. The affinity of a molecule X for its partner Y can be represented generally by the dissociation constant (K_{D}), which is the ratio of the dissociation rate constant to the association rate constant (K_{dis} and Kₒₙ, respectively). Affinity can be measured by common methods known in the art. One specific method for measuring affinity is the ForteBio kinetic binding assay described herein.

The term "host cell" refers to a cell into which an exogenous polynucleotide has been introduced, including progeny of such cells. Host cells include "transformants" and "transformed cells", which include primary transformed cells and progeny derived therefrom.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, the subject is a human.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, condition, or disorder.

The term "prevention" (or "prevent" or "preventing") includes the inhibition of the development or progression of symptoms of a disease or disorder, or a specific disease or disorder.

The term "therapeutic agent" described herein encompasses any substance effective in preventing or treating a tumor, e.g., cancer, including a chemotherapeutic agent, a cytokine, a cytotoxic agent, an additional antibody (e.g., an immune checkpoint molecule antibody), a small molecule drug, or an immunomodulatory agent (e.g., an immunosuppressant or an agonist).

The term "combination therapy" refers to the administration of two or more therapeutic agents or modalities to treat the diseases described herein. Such administration includes co-administration of these therapeutic agents in a substantially simultaneous manner, e.g., in a single capsule having a fixed ratio of active ingredients. Alternatively, such administration includes co-administration of the active ingredients in a variety of or separate containers (e.g., tablets, capsules, powder and liquid). The powder and/or liquid may be reconstituted or diluted to a desired dose prior to administration. In addition, such administration also includes using each type of therapeutic agent in a sequential manner at approximately the same time or at different times. In either case, the therapeutic regimen will provide the beneficial effect of the drug combination in treating the disorders or conditions described herein.

The term "pharmaceutical combination or combination product" refers to a non-fixed combination product or a fixed combination product, including, but not limited to, a kit and a pharmaceutical composition. The term "non-fixed combination" means that the active ingredients (e.g., (i) the antibody of the present invention, and (ii) an additional therapeutic agent) are administered to a patient as separate entities, either simultaneously or sequentially (without specific time constraint or at the same or different time intervals), wherein such administration provides two or more prophylactically or therapeutically effective active agents in the patient. The term "fixed combination" means that two or more active agents are administered to a patient simultaneously as a single entity. The dosage and/or time interval of the two or more active agents is preferably selected such that the combined use of the components can result in a therapeutic effect on the disease or disorder greater than that achieved by the use of either component alone. The ingredients may each be in a separate formulation, which may be the same or different.

The term "pharmaceutical composition" refers to a composition that exists in a form allowing the biological activity of active ingredients contained therein to be effective and does not contain additional ingredients having unacceptable toxicity to a subject to which the composition is administered.

The term "pharmaceutical supplementary material" refers to diluents, adjuvants (e.g., Freund's adjuvants (complete and incomplete)), excipients, carriers, stabilizers, or the like, which are administered with the active substance.

"Subject/patient/individual sample" refers to a collection of cells or fluids obtained from a patient or a subject. The source of the tissue or cell sample may be solid tissue as from a fresh, frozen and/or preserved organ or tissue sample or biopsy or puncture sample; bodily fluids such as tears, vitreous humor, cerebrospinal fluid, amniotic fluid, peritoneal fluid, or interstitial fluid.

### II. Anti-CD28 antibody

In some embodiments, the anti-CD28 antibody or the antigen-binding fragment thereof of the present invention comprises three complementarity-determining regions from a heavy chain variable region (CDRHs): CDRH1, CDRH2, and CDRH3. In some embodiments, the anti-CD28 antibody or the antigen-binding fragment thereof of the present invention comprises three complementarity-determining regions from a light chain variable region (CDRLs): CDRL1, CDRL2, and CDRL3. In some embodiments, the anti-CD28 antibody or the antigen-binding fragment thereof of the present invention comprises three complementarity-determining regions from a heavy chain variable region (CDRHs) and three complementarity-determining regions from a light chain variable region (CDRLs).

In some aspects, the anti-CD28 antibody or the antigen-binding fragment thereof of the present invention comprises a heavy chain variable region (VH). In some aspects, the anti-CD28 antibody or the antigen-binding fragment thereof of the present invention comprises a light chain variable region (VL). In some aspects, the anti-CD28 antibody or the antigen-binding fragment thereof of the present invention comprises a heavy chain variable region (VH) and a light chain variable region (VL). In some embodiments, the heavy chain variable region comprises three complementarity-determining regions (CDRHs) from the heavy chain variable region: CDRH1, CDRH2, and CDRH3. In some embodiments, the light chain variable region comprises three complementarity-determining regions (CDRLs) from the light chain variable region: CDRL1, CDRL2, and CDRL3.

In some embodiments, the heavy chain variable region VH of the anti-CD28 antibody of the present invention
(i) comprises or consists of an amino acid sequence having at least 90%, 91%, 92%, 93%, 94%, 95%, 96%, 97%, 98%, or 99% identity to the amino acid sequence of SEQ ID NO: 4, 5, 13, 23, 24, 69, 72, or 75; or
(ii) comprises or consists of the amino acid sequence of SEQ ID NO: 4, 5, 13, 23, 24, 69, 72, or 75; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with the amino acid sequence of SEQ ID NO: 4, 5, 13, 23, 24, 69, 72, or 75, wherein preferably, the amino acid alterations do not occur in the CDRs.

In some embodiments, the light chain variable region VL of the anti-CD28 antibody of the present invention
(i) comprises or consists of an amino acid sequence having at least 90%, 91%, 92%, 93%, 94%, 95%, 96%, 97%, 98%, or 99% identity to the amino acid sequence of SEQ ID NO: 9, 17, 19, 28, 70, 73, or 78; or
(ii) comprises or consists of the amino acid sequence of SEQ ID NO: 9, 17, 19, 28, 70, 73, or 78; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence of SEQ ID NO: 9, 17, 19, 28, 70, 73, or 78, wherein preferably, the amino acid alterations do not occur in the CDRs.

In some embodiments, the three complementarity-determining regions from a heavy chain variable region (CDRHs) of the anti-CD28 antibody of the present invention, CDRH1, CDRH2, and CDRH3, are
(i) three complementarity-determining regions CDRH1, CDRH2, and CDRH3 contained in a VH set forth in SEQ ID NO: 4, 5, 13, 23, 69, 72, or 75, or
(ii) a sequence comprising, collectively across the three CDRH regions, at least one and no more than 5, 4, 3, 2, or 1 amino acid alterations (preferably amino acid substitutions, and more preferably conservative substitutions) relative to the sequence of (i) ;
   wherein the CDRHs may each be defined according to any scheme for determining CDRs, e.g., according to the Kabat, AbM, Chothia, Contact, or IMGT schemes, or a combination thereof;
   preferably, the CDRH1 is defined according to the Kabat & chothia scheme, and CDRH2 and CDRH3 are each defined according to the Kabat scheme.

In some embodiments, the three complementarity-determining regions from a light chain variable region (CDRLs) of the anti-CD28 antibody of the present invention, CDRL1, CDRL2, and CDRL3, are
(i) three complementarity-determining regions CDRL1, CDRL2 and CDRL3 contained in a VL set forth in SEQ ID NO: 9, 17, 19, 28, 70, 73 or 78, or
(ii) a sequence comprising, collectively across the three CDRL regions, at least one and no more than 5, 4, 3, 2, or 1 amino acid alterations (preferably amino acid substitutions, and more preferably conservative substitutions) relative to the sequence of (i);
   wherein the CDRLs may each be defined according to any scheme for determining CDRs, e.g., according to the Kabat, AbM, Chothia, Contact, or IMGT scheme, or a combination thereof;
   preferably, the CDRL1-3 are each determined according to Kabat.

In some embodiments, the CDRH1 comprises or consists of an amino acid sequence set forth in SEQ ID NO: 1, 10, or 20 or an amino acid sequence having 1, 2, or 3 amino acid alterations (preferably amino acid substitutions, and more preferably conservative substitutions) compared thereto.

In some embodiments, the CDRH2 comprises or consists of an amino acid sequence set forth in SEQ ID NO: 2, 11, 21, or 76 or an amino acid sequence having 1, 2, or 3 amino acid alterations (preferably amino acid substitutions, and more preferably conservative substitutions) compared thereto.

In some embodiments, the CDRH3 comprises or consists of an amino acid sequence set forth in SEQ ID NO: 3, 12, 22, or 77 or an amino acid sequence having 1, 2, or 3 amino acid alterations (preferably amino acid substitutions, and more preferably conservative substitutions) compared thereto.

In some embodiments, the CDRL1 comprises or consists of an amino acid sequence set forth in SEQ ID NO: 6, 14, 18, 25, or 71 or an amino acid sequence having 1, 2, or 3 amino acid alterations (preferably amino acid substitutions, and more preferably conservative substitutions) compared thereto.

In some embodiments, the CDRL2 comprises or consists of an amino acid sequence set forth in SEQ ID NO: 7, 15, or 26 or an amino acid sequence having 1, 2, or 3 amino acid alterations (preferably amino acid substitutions, and more preferably conservative substitutions) compared thereto.

In some embodiments, the CDRL3 comprises or consists of an amino acid sequence set forth in SEQ ID NO: 8, 16, or 27 or an amino acid sequence having 1, 2, or 3 amino acid alterations (preferably amino acid substitutions, and more preferably conservative substitutions) compared thereto.

In some embodiments, in the anti-CD28 antibody of the present invention,
the CDRH1 comprises or consists of the amino acid sequence of SEQ ID NO: 1; the CDRH2 comprises or consists of the amino acid sequence of SEQ ID NO: 2; the CDRH3 comprises or consists the an amino acid sequence of SEQ ID NO: 3; the CDRL1 comprises or consists of the amino acid sequence of SEQ ID NO: 6; the CDRL2 comprises or consists of the amino acid sequence of SEQ ID NO: 7; and/or the CDRL3 comprises or consists of an amino acid sequence of SEQ ID NO: 8;
the CDRH1 comprises or consists of the amino acid sequence of SEQ ID NO: 10; the CDRH2 comprises or consists of the amino acid sequence of SEQ ID NO: 11; the CDRH3 comprises or consists of the amino acid sequence of SEQ ID NO: 12; the CDRL1 comprises or consists of the amino acid sequence of SEQ ID NO: 14, 18, or 71; the CDRL2 comprises or consists of the amino acid sequence of SEQ ID NO: 15; and/or the CDRL3 comprises or consists of the amino acid sequence of SEQ ID NO: 16;
the CDRH1 comprises or consists of the amino acid sequence of SEQ ID NO: 20; the CDRH2 comprises or consists of the amino acid sequence of SEQ ID NO: 21 or 76; the CDRH3 comprises or consists of the amino acid sequence of SEQ ID NO: 22 or 77; the CDRL1 comprises or consists of the amino acid sequence of SEQ ID NO: 25; the CDRL2 comprises or consists of the amino acid sequence of SEQ ID NO: 26; and/or the CDRL3 comprises or consists of the amino acid sequence of SEQ ID NO: 27;
the CDRH1 comprises or consists of the amino acid sequence of SEQ ID NO: 20; the CDRH2 comprises or consists of the amino acid sequence of SEQ ID NO: 21; the CDRH3 comprises or consists of the amino acid sequenceof SEQ ID NO: 22; the CDRL1 comprises or consists of the amino acid sequence of SEQ ID NO: 25; the CDRL2 comprises or consists of the amino acid sequence of SEQ ID NO: 26; and/or the CDRL3 comprises or consists of the amino acid sequence of SEQ ID NO: 27; or
the CDRH1 comprises or consists of the amino acid sequence of SEQ ID NO: 20; the CDRH2 comprises or consists of the amino acid sequence of SEQ ID NO: 76; the CDRH3 comprises or consists of the amino acid sequence of SEQ ID NO: 77; the CDRL1 comprises or consists of the amino acid sequence of SEQ ID NO: 25; the CDRL2 comprises or consists of the amino acid sequence of SEQ ID NO: 26; and/or the CDRL3 comprises or consists of the amino acid sequence of SEQ ID NO: 27.

In some particular embodiments of the present invention, the anti-CD28 antibody or the antigen-binding fragment thereof of the present invention comprises a VH and a VL, wherein
the VH comprises or consists of an amino acid sequence set forth in SEQ ID NO: 4 or 5 or 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or 73 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 4 or 5 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 73 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 13 or 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or 70 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 13 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 70 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 23 or 24 or 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or 78 or an amino acid sequence having at least 90%, 91%, 92%, 93%, 94%, 95%, 96%, 97%, 98%, or 99% identity thereto;
the VH comprises or consists of an amino acid sequence set forth in SEQ ID NO: 23 or 24 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or an amino acid sequence having at least 90%, 91%, 92%, 93%, 94%, 95%, 96%, 97%, 98%, or 99% identity thereto; or
the VH comprises or consists of an amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 78 or an amino acid sequence having at least 90%, 91%, 92%, 93%, 94%, 95%, 96%, 97%, 98%, or 99% identity thereto.

In some particular embodiments of the present invention, the anti-CD28 antibody or the antigen-binding fragment thereof of the present invention comprises:
(i). three complementarity-determining regions CDRH1, CDRH2, and CDRH3 contained in a VH set forth in SEQ ID NO: 4, 5, or 72, and three complementarity-determining regions CDRL1, CDRL2, and CDRL3 contained in a VL set forth in SEQ ID NO: 9 or 73;
(ii). three complementarity-determining regions CDRH1, CDRH2 and CDRH3 contained in a VH set forth in SEQ ID NO: 13 or 69, and three complementarity-determining regions CDRL1, CDRL2 and CDRL3 contained in a VL set forth in SEQ ID NO: 17, 19 or 70; or
(iii). three complementarity-determining regions CDRH1, CDRH2, and CDRH3 contained in a VH set forth in SEQ ID NO: 23, 24, or 75, and three complementarity-determining regions CDRL1, CDRL2, and CDRL3 contained in a VL set forth in SEQ ID NO: 28 or 78;
preferably, wherein the CDRH and CDRL may be determined according to any scheme for determining CDRs, e.g., according to Kabat, AbM, Chothia, Contact, or IMGT schemes, or a combination thereof; for example, the CDRH1 is determined according to the Kabat & Chothia scheme, and CDRH2 and CDRH3 are each determined according to Kabat; and the CDRL1-3 are each determined by the Kabat scheme.

In some particular embodiments of the present invention, the anti-CD28 antibody or the antigen-binding fragment thereof of the present invention comprises CDRH1, CDRH2, CDRH3, CDRL1, CDRL2, and CDRL3, wherein
the CDRH1 consists of the amino acid sequence of SEQ ID NO: 1; the CDRH2 consists of the amino acid sequence of SEQ ID NO: 2; the CDRH3 consists of the amino acid sequence of SEQ ID NO: 3; the CDRL1 consists of the amino acid sequence of SEQ ID NO: 6; the CDRL2 consists of the amino acid sequence of SEQ ID NO: 7; and the CDRL3 consists of the amino acid sequence of SEQ ID NO: 8;
the CDRH1 consists of the amino acid sequence of SEQ ID NO: 10; the CDRH2 consists of the amino acid sequence of SEQ ID NO: 11; the CDRH3 consists of the amino acid sequence of SEQ ID NO: 12; the CDRL1 consists of the amino acid sequence of SEQ ID NO: 14 or 18 or 71; the CDRL2 consists of the amino acid sequence of SEQ ID NO: 15; and the CDRL3 consists of the amino acid sequence of SEQ ID NO: 16;
the CDRH1 consists of the amino acid sequence of SEQ ID NO: 20; the CDRH2 consists of the amino acid sequence of SEQ ID NO: 21; the CDRH3 consists of the amino acid sequence of SEQ ID NO: 22; the CDRL1 consists of the amino acid sequence of SEQ ID NO: 25; the CDRL2 consists of the amino acid sequence of SEQ ID NO: 26; and the CDRL3 consists of the amino acid sequence of SEQ ID NO: 27; or

The CDRH1 consists of the amino acid sequence of SEQ ID NO: 20; the CDRH2 consists of the amino acid sequence of SEQ ID NO: 76; the CDRH3 consists of the amino acid sequence of SEQ ID NO: 77; the CDRL1 consists of the amino acid sequence of SEQ ID NO: 25; the CDRL2 consists of the amino acid sequence of SEQ ID NO: 26; and the CDRL3 consists of the amino acid sequence of SEQ ID NO: 27.

In some particular embodiments of the present invention, the anti-CD28 antibody or the antigen-binding fragment thereof of the present invention comprises a VH and a VL, wherein
the VH comprises or consists of an amino acid sequence set forth in SEQ ID NO: 4, and the VL comprises or consists of an amino acid sequence set forth in SEQ ID NO: 9;
the VH comprises or consists of an amino acid sequence set forth in SEQ ID NO: 5, and the VL comprises or consists of an amino acid sequence set forth in SEQ ID NO: 9;
the VH comprises or consists of an amino acid sequence set forth in SEQ ID NO: 13, and the VL comprises or consists of an amino acid sequence set forth in SEQ ID NO: 17;
the VH comprises or consists of an amino acid sequence set forth in SEQ ID NO: 13, and the VL comprises or consists of an amino acid sequence set forth in SEQ ID NO: 19;
the VH comprises or consists of an amino acid sequence set forth in SEQ ID NO: 23, and the VL comprises or consists of an amino acid sequence set forth in SEQ ID NO: 28;
the VH comprises or consists of an amino acid sequence set forth in SEQ ID NO: 24, and the VL comprises or consists of an amino acid sequence set forth in SEQ ID NO: 28;
the VH comprises or consists of an amino acid sequence set forth in SEQ ID NO: 72, and the VL comprises or consists of an amino acid sequence set forth in SEQ ID NO: 73;
the VH comprises or consists of an amino acid sequence set forth in SEQ ID NO: 69, and the VL comprises or consists of an amino acid sequence set forth in SEQ ID NO: 70; or
the VH comprises or consists of an amino acid sequence set forth in SEQ ID NO: 75, and the VL comprises or consists of an amino acid sequence set forth in SEQ ID NO: 78.

In one embodiment of the present invention, the amino acid alterations described herein include amino acid substitutions, insertions, or deletions. In a preferred embodiment, the amino acid alterations described herein occur in regions outside the CDRs (e.g., in FRs). More preferably, the amino acid alterations described herein occur in a region outside the heavy chain variable region and/or outside the light chain variable region. Preferably, the amino acid alterations described herein are amino acid substitutions, preferably conservative substitutions.

In some embodiments, the anti-CD28 antibody or the antigen-binding fragment thereof of the present invention is an antibody in the form of IgG1, IgG2, IgG3, or IgG4, for example, an antibody in the form of IgG1 or an antigen-binding fragment thereof.

In some embodiments, the anti-CD28 antibody or the antigen-binding fragment thereof of the present invention further comprises an antibody heavy chain constant region HC. In some embodiments, the anti-CD28 antibody or the antigen-binding fragment thereof of the present invention further comprises an antibody light chain constant region LC. In some embodiments, the anti-CD28 antibody or the antigen-binding fragment thereof of the present invention further comprises a heavy chain constant region HC and a light chain constant region LC. In some embodiments, the heavy chain constant region of the present invention comprises an Fc region.

In some embodiments, the heavy chain constant region of the anti-CD28 antibody of the present invention is a constant region of (human) IgG1, IgG2, IgG3, or IgG4, e.g., a constant region of IgG1.

In some embodiments, the IgG1 heavy chain constant region suitable for use in the antibody molecule of the present invention
(i) comprises or consists of an amino acid sequence having at least 85%, 90%, 91%, 92%, 93%, 94%, 95%, 96%, 97%, 98%, or 99% identity to the amino acid sequence of SEQ ID NO: 29 or 79;
(ii) comprises or consists of the amino acid sequence of SEQ ID NO: 29 or 79; or
(iii) comprises or consists of an amino acid sequence having one or more (preferably no more than 10 or 10, more preferably no more than 5, 4, 3, 2, or 1) amino acid alterations (preferably amino acid substitutions, more preferably conservative amino acid substitutions) compared with the amino acid sequence of SEQ ID NO: 29 or 79.

In some embodiments, the light chain constant region of the anti-CD28 antibody of the present invention is a (human) Kappa or Lambda light chain constant region, e.g., a Kappa light chain constant region.

In some embodiments, the light chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 30;
(ii) comprises or consists of the amino acid sequence of SEQ ID NO: 30; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 30.

In some embodiments, the anti-CD28 antibody or the antigen-binding fragment thereof of the present invention comprises an antibody heavy chain. In some embodiments, the anti-CD28 antibody or the antigen-binding fragment thereof of the present invention comprises an antibody light chain. In some embodiments, the anti-CD28 antibody or the antigen-binding fragment thereof of the present invention comprises a heavy chain and a light chain. In some embodiments, the anti-CD28 antibody or the antigen-binding fragment thereof of the present invention comprises two heavy chains and two light chains. In some embodiments, the heavy chain comprises or consists of a heavy chain variable region and a heavy chain constant region. In some embodiments, the light chain comprises or consists of a light chain variable region and a light chain constant region.

In some embodiments, the anti-CD28 antibody or the antigen-binding fragment thereof of the present invention has one or more of the following properties:
(i) showing the same or similar binding affinity and/or specificity for CD28 as the antibody of the present invention;
(ii) inhibiting (e.g., competitively inhibiting) the binding of the antibody of the present invention to CD28;
(iii) binding to the same or overlapping epitope as the antibody of the present invention;
(iv) competing with the antibody of the present invention for binding to CD28;
(v) having one or more biological properties of the antibody of the present invention.

In some embodiments, the anti-CD28 antibody is a monoclonal antibody.

In some embodiments, the anti-CD28 antibody is humanized. Humanization can be achieved by replacing one or more amino acid residues, particularly framework region sequences, in the heavy and light chain variable regions of a non-human natural antibody with residues from the corresponding positions in the variable regions of a human conventional antibody. Methods of humanizing antibodies are well known in the art. Typically, the humanization substitutions are made in a manner that preserves the favorable binding properties of the antibody. Assays for determining biological properties of the humanized antibody, such as binding affinity, are well known in the art to determine and select suitable mutations or combinations of mutations of humanized residues.

In one embodiment, the anti-CD28 antibody of the present invention is a full-length antibody. In one embodiment, the anti-CD28 antibody of the present invention also encompasses an antibody fragment (e.g., an antigen-binding fragment) thereof, preferably an antibody fragment selected from: a Fab, a Fab', a Fab'-SH, a Fv, a single-chain antibody (e.g., scFv), (Fab')₂, a single domain antibody (e.g., VHH), a domain antibody (dAb), a heavy-chain antibody, or a linear antibody. In one embodiment, the anti-CD28 antibody fragment of the present invention is a Fab comprising the VH and VL described herein, as well as the CH1 and CL.

In one embodiment, the anti-CD28 antibody of the present invention may also be a bispecific antibody or a multispecific antibody specifically binding to CD28 and one or more additional antigens (e.g., TAA).

### III. Multispecific Binding Molecule

In one aspect of the present invention, the present invention relates to a multispecific binding molecule capable of specifically binding to CD28 and specifically binding to one or more additional targets or antigens. In some embodiments, the additional antigen is a tumor-associated antigen (TAA), such as CLDN18.2 or PSMA. In some embodiments, the multispecific binding molecule is a multispecific antibody, e.g., a bispecific antibody.

In some embodiments, the antibody of the present invention is a bispecific antibody. The term "bispecific antibody" refers to an antibody comprising a first antigen-binding region and a second antigen-binding region, wherein the first antigen-binding region binds to one antigen or epitope and the second antigen-binding region binds to another antigen or another epitope. Therefore, the bispecific antibody according to the present invention comprises specificity for two different antigens, or for two different epitopes of one antigen. Bispecific antibody formats comprise IgG-like antibodies (Fan et al. (2015) Journal of Hematology & Oncology. 8: 130). The most common type of IgG-like antibodies comprises two Fab regions and two Fc regions, and the heavy and light chains of each Fab may be from a separate monoclonal antibody. The bispecific antibody of the present invention can be prepared using bispecific antibody formats or techniques known in the art. Specific exemplary bispecific formats that can be used in the context of the present invention can be found, for example, in Labrijn, et al., Bispecific antibodies: a mechanistic review of the pipeline, Nature Reviews Drug Discovery, 2019, 18(8):1-24.

Therefore, one aspect of the present invention relates to a bispecific antibody, which comprises
a first antigen-binding region and a second antigen-binding region, wherein the first antigen-binding region specifically binds to CD28, and the second antigen-binding region specifically binds to a tumor-associated antigen. In some embodiments, the tumor-associated antigen is selected from PSMA or CLDN18.2.

In some embodiments, the first antigen-binding region is from the anti-CD28 antibody or the antigen-binding fragment thereof described herein, e.g., the Fab of the anti-CD28 antibody described herein. In some embodiments, the first antigen-binding region specifically binding to CD28 comprises 1, 2, 3, 4, 5, or 6 CDRs of the anti-CD28 antibody described herein. In some embodiments, the first antigen-binding region comprises 1, 2, or 3 heavy chain variable region CDRs, i.e., CDRH1, CDRH2, and CDRH3, of the anti-CD28 antibody described herein. In some embodiments, the first antigen-binding region comprises 1, 2, or 3 light chain variable region CDRs, i.e., CDRL1, CDRL2, and CDRL3, of the anti-CD28 antibody described herein. In some embodiments, the first antigen-binding region comprises 3 heavy chain variable region CDRs and 3 light chain variable region CDRs of the anti-CD28 antibody described herein. In some embodiments, the first antigen-binding region comprises the heavy chain variable region of the anti-CD28 antibody described herein. In some embodiments, the first antigen-binding region comprises the light chain variable region of the anti-CD28 antibody described herein. In some embodiments, the first antigen-binding region comprises the heavy chain variable region and the light chain variable region of the anti-CD28 antibody described herein. In some embodiments, the first antigen-binding region comprises the Fab of the anti-CD28 antibody described herein.

In some embodiments, the second antigen-binding region is from an anti-TAA (e.g., CLDN18.2 or PSMA, e.g., human CLDN18.2 or human PSMA) antibody or an antigen-binding fragment thereof, e.g., an anti-CLDN18.2 antibody or an antigen-binding fragment thereof, or an anti-PSMA antibody or an antigen-binding fragment thereof.

The first antigen-binding region suitable for use in the bispecific antibody of the present invention may comprise or consist of the anti-CD28 full-length antibody or the antigen-binding fragment thereof of the present invention, as long as it is capable of specifically binding to CD28, including, but not limited to, for example, a full-length antibody, a half-antibody, a Fab, a Fab', a Fab'-SH, a Fv, a single-chain antibody (e.g., scFv), or a (Fab')₂, a single-domain antibody (e.g., VHH), a domain antibody (dAb), a heavy-chain antibody, or a linear antibody specifically binding to CD28, or the like.

The second antigen-binding region suitable for use in the bispecific antibody of the present invention may comprise or consist of an anti-TAA antibody or an antigen-binding fragment thereof, as long as it is capable of specifically binding to TAA, including, but not limited to, for example, a full-length antibody, a half-antibody, a Fab, a Fab', a Fab'-SH, a Fv, a single-chain antibody (e.g., scFv), and a (Fab')₂, a single-domain antibody (e.g., VHH), a domain antibody (dAb), a heavy-chain antibody, or a linear antibody specifically binding to TAA, or the like.

In some embodiments, the second antigen-binding region suitable for use in the bispecific antibody of the present invention may comprise or consist of an anti-CLDN18.2 antibody or an antigen-binding fragment thereof (e.g., the anti-CLDN18.2 antibody or the antigen-binding fragment thereof disclosed in CN202010570517.X (WO2021254481A1), e.g., an HB37A6 antibody or an antigen-binding fragment thereof, as long as it is capable of specifically binding to CLDN18.2, including, but not limited to, for example, a full-length antibody, a half-antibody, a Fab, a Fab', a Fab'-SH, a Fv, a single-chain antibody (e.g., scFv), and a (Fab')₂, a single-domain antibody (e.g., VHH), a domain antibody (dAb), a heavy-chain antibody, or a linear antibody specifically binding to CLDN18.2, or the like.

In some embodiments, the second antigen-binding region suitable for use in the bispecific antibody of the present invention may comprise or consist of an anti-PSMA antibody or an antigen-binding fragment thereof (e.g., the anti-PSMA antibody or antigen-binding fragment thereof disclosed in EP1851250B1, e.g., an 1C3 antibody or antigen-binding fragment thereof), as long as it is capable of specifically binding to PSMA, including, but not limited to, for example, a full-length antibody, a half-antibody, a Fab, a Fab', a Fab'-SH, a Fv, a single-chain antibody (e.g., scFv), and a (Fab')₂, a single-domain antibody (e.g., VHH), a domain antibody (dAb), a heavy-chain antibody, or a linear antibody specifically binding to PSMA, or the like.

In some embodiments, the bispecific antibody of the present invention is an IgG-like bispecific antibody. In some embodiments, the IgG-like bispecific antibody of the present invention refers to a bispecific antibody comprising an Fc dimer (e.g., a heterodimeric Fc scaffold). Thus, in some embodiments, the bispecific antibody of the present invention comprises an Fc dimer, e.g., a heterodimeric Fc scaffold.

In one embodiment, the bispecific antibody may comprise one or more first antigen-binding regions. In one embodiment, the bispecific antibody may comprise one or more second antigen-binding regions.

### III-1 Second antigen-binding region

In some embodiments, the second antigen-binding region specifically binds to a tumor-associated antigen (TAA), or an immune checkpoint molecule.

In some embodiments, the tumor-associated antigen is CLDN18.2.

In some embodiments, the second antigen-binding region is an antigen-binding region specifically binding to CLDN18.2. In some embodiments, the second antigen-binding region specifically binding to CLDN18.2 is derived from an antibody specifically binding to CLDN18.2, e.g., the antibody specifically binding to CLDN18.2 disclosed in CN202010570517.X (WO2021254481A1) (incorporated herein in its entirety), e.g., HB37A6 disclosed therein.

In some embodiments, the second antigen-binding region specifically binding to CLDN18.2 comprises 1, 2, 3, 4, 5, or 6 CDRs of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises 1, 2, or 3 heavy chain variable region CDRs (i.e., CDRH1, CDRH2, and CDRH3) of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises 1, 2, or 3 light chain variable region CDRs (i.e., CDRL1, CDRL2, and CDRL3) of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises 3 heavy chain variable region CDRs and 3 light chain variable region CDRs of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises the heavy chain variable region of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises the light chain variable region of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises the heavy chain variable region and the light chain variable region of an antibody known to specifically bind to CLDN18.2 (e.g., the CLDN18.2 antibody in CN202010570517.X (WO2021254481A1), such as HB37A6 disclosed therein). In some embodiments, the second antigen-binding region comprises a Fab of an antibody known to specifically bind to CLDN18.2 (e.g., the CLDN18.2 antibody in CN202010570517.X (WO2021254481A1), such as HB37A6 disclosed therein).

In some embodiments, the second antigen-binding region specifically binding to CLDN18.2 comprises three complementarity-determining regions from a heavy chain variable region (CDRHs): CDRH1, CDRH2, and CDRH3, wherein the CDRH1, the CDRH2, and the CDRH3 are the CDRH1, CDRH2, and CDRH3 of the heavy chain variable region set forth in SEQ ID NO: 86; preferably, wherein the CDRHs may be determined according to any scheme for determining CDRs, e.g., according to the Kabat, AbM, Chothia, Contact, or IMGT scheme, or a combination thereof. In some embodiments, the second antigen-binding region specifically binding to CLDN18.2 comprises three complementarity-determining regions from a light chain variable region (CDRLs): CDRL1, CDRL2, and CDRL3, wherein the CDRL1, CDRL2, and CDRL3 are the CDRL1, CDRL2, and CDRL3 of the light chain variable region set forth in SEQ ID NO: 90; preferably, wherein the CDRLs may be determined according to any scheme for determining CDRs, e.g., according to Kabat, AbM, Chothia, Contact, or IMGT schemes, or a combination thereof.

In some embodiments, in the second antigen-binding region that specifically binds to CLDN18.2, CDRH1 comprises or consists of the amino acid sequence of SEQ ID NO: 87; CDRH2 comprises or consists of the amino acid sequence of SEQ ID NO: 88; CDRH3 comprises or consists of the amino acid sequence of SEQ ID NO: 89; CDRL1 comprises or consists of the amino acid sequence of SEQ ID NO: 91; CDRL2 comprises or consists of the amino acid sequence of SEQ ID NO: 92; and/or CDRL3 comprises or consists of the amino acid sequence of SEQ ID NO: 93.

In some embodiments, the second antigen-binding region specifically binding to CLDN18.2 comprises a VH, wherein the VH
comprises three complementarity-determining regions from a heavy chain variable region (CDRHs), CDRH1, CDRH2, and CDRH3, wherein the CDRH1 comprises or consists of the amino acid sequence of SEQ ID NO: 87, the CDRH2 comprises or consists of the amino acid sequence of SEQ ID NO: 88, and the CDRH3 comprises or consists of the amino acid sequence of SEQ ID NO: 89; or
comprises or consists of an amino acid sequence set forth in SEQ ID NO: 86.

In some embodiments, the second antigen-binding region specifically binding to CLDN18.2 comprises a VL, wherein the VL
comprises three complementarity-determining regions from a light chain variable region (CDRHs), CDRH1, CDRH2 and CDRH3, wherein the CDRL1 comprises or consists of the amino acid sequence of SEQ ID NO: 91, the CDRL2 comprises or consists of the amino acid sequence of SEQ ID NO: 92, and the CDRL3 comprises or consists of the amino acid sequence of SEQ ID NO: 93; or
comprises or consists of an amino acid sequence set forth in SEQ ID NO: 90.

In some embodiments, the second antigen-binding region specifically binding to CLDN18.2 comprises three complementarity-determining regions from a heavy chain variable region (CDRHs): CDRH1, CDRH2, and CDRH3, and three complementarity-determining regions from a light chain variable region (CDRLs): CDRL1, CDRL2, and CDRL3, wherein the CDRH1 comprises or consists of the amino acid sequence of SEQ ID NO: 87; the CDRH2 comprises or consists of the amino acid sequence of SEQ ID NO: 88; the CDRH3 comprises or consists of the amino acid sequence of SEQ ID NO: 89; the CDRL1 comprises or consists of the amino acid sequence of SEQ ID NO: 91; the CDRL2 comprises or consists of the amino acid sequence of SEQ ID NO: 92; and the CDRL3 comprises or consists of the amino acid sequence of SEQ ID NO: 93.

In some embodiments, the second antigen-binding region specifically binding to CLDN18.2 comprises a VH and a VL, wherein the VH comprises or consists of a sequence set forth in SEQ ID NO: 86; and/or the VL comprises or consists of a sequence set forth in SEQ ID NO: 90.

In some embodiments, the second antigen-binding region specifically binding to CLDN18.2 is a Fab fragment.

In some embodiments, the second antigen-binding region is an antigen-binding region specifically binding to PSMA. In some embodiments, the second antigen-binding region specifically binding to PSMA is derived from an antibody specifically binding to PSMA, e.g., the antibody that specifically binds to PSMA disclosed in EP1851250B1, e.g., 1C3 disclosed therein.

In some embodiments, the second antigen-binding region that specifically binds to PSMA comprises 1, 2, 3, 4, 5, or 6 CDRs of an antibody known to specifically bind to PSMA (e.g., the PSMA antibody disclosed in EP1851250B1, which is incorporated herein by reference in its entirety, such as 1C3 disclosed therein). In some embodiments, the second antigen-binding region comprises 1, 2, or 3 heavy chain variable region CDRs (i.e., CDRH1, CDRH2, and CDRH3) of an antibody known to specifically bind to PSMA (e.g., the PSMA antibody disclosed in EP1851250B1, such as 1C3 disclosed therein). In some embodiments, the second antigen-binding region comprises 1, 2, or 3 light chain variable region CDRs (i.e., CDRL1, CDRL2, and CDRL3) of an antibody known to specifically bind to PSMA (e.g., the PSMA antibody disclosed in EP1851250B1, such as 1C3 disclosed therein). In some embodiments, the second antigen-binding region comprises 3 heavy chain variable region CDRs and 3 light chain variable region CDRs of an antibody known to specifically bind to PSMA (e.g., the PSMA antibody disclosed in EP1851250B1, such as 1C3 disclosed therein). In some embodiments, the second antigen-binding region comprises the heavy chain variable region of an antibody known to specifically bind to PSMA (e.g., the PSMA antibody disclosed in EP1851250B1, such as 1C3 disclosed therein). In some embodiments, the second antigen-binding region comprises the light chain variable region of an antibody known to specifically bind to PSMA (e.g., the PSMA antibody disclosed in EP1851250B1, such as 1C3 disclosed therein). In some embodiments, the second antigen-binding region comprises the heavy chain variable region and the light chain variable region of an antibody known to specifically bind to PSMA (e.g., the PSMA antibody disclosed in EP1851250B1, such as 1C3 disclosed therein). In some embodiments, the second antigen-binding region comprises a Fab of an antibody known to specifically bind to PSMA (e.g., the PSMA antibody disclosed in EP1851250B1, such as 1C3 disclosed therein).

In some embodiments, the second antigen-binding region specifically binding to PSMA comprises three complementarity-determining regions from a heavy chain variable region (CDRHs): CDRH1, CDRH2, and CDRH3, wherein the CDRH1, the CDRH2, and the CDRH3 are CDRH1, CDRH2, and CDRH3 of a heavy chain variable region set forth in SEQ ID NO: 47; preferably, the CDRH can be determined according to any scheme for determining CDRs, e.g., according to the Kabat, AbM, Chothia, Contact, or IMGT scheme, or a combination thereof. In some embodiments, the second antigen-binding region specifically binding to PSMA comprises three complementarity-determining regions from a light chain variable region (CDRLs): CDRL1, CDRL2, and CDRL3, wherein the CDRL1, CDRL2, and CDRL3 are the CDRL1, CDRL2, and CDRL3 of a light chain variable region set forth in SEQ ID NO: 49; preferably, the CDRLs can be determined according to any scheme for determining CDRs, e.g., according to the Kabat, AbM, Chothia, Contact, or IMGT scheme, or a combination thereof. In some embodiments, in the second antigen-binding region specifically binding to PSMA, CDRH1 comprises or consists of the amino acid sequence of SEQ ID NO: 96; CDRH2 comprises or consists of the amino acid sequence of SEQ ID NO: 97, CDRH3 comprises or consists of the amino acid sequence of SEQ ID NO: 98; CDRL1 comprises or consists of the amino acid sequence of SEQ ID NO: 99; CDRL2 comprises or consists of the amino acid sequence of SEQ ID NO: 100, and/or CDRL3 comprises or consists of the amino acid sequence of SEQ ID NO: 101.

In some embodiments, the second antigen-binding region specifically binding to PSMA comprises a VH, wherein the VH
comprises three complementarity-determining regions from a heavy chain variable region (CDRHs), CDRH1, CDRH2, and CDRH3, wherein the CDRH1 comprises or consists of the amino acid sequence of SEQ ID NO: 96, the CDRH2 comprises or consists of the amino acid sequence of SEQ ID NO: 97, and the CDRH3 comprises or consists of the amino acid sequence of SEQ ID NO: 98; or
comprises or consists of an amino acid sequence set forth in SEQ ID NO: 47.

In some embodiments, the second antigen-binding region specifically binding to PSMA comprises a VL, wherein the VL
comprises three complementarity-determining regions from a light chain variable region (CDRHs), CDRH1, CDRH2 and CDRH3, wherein the CDRL1 comprises or consists of the amino acid sequence of SEQ ID NO: 99, the CDRL2 comprises or consists of the amino acid sequence of SEQ ID NO: 100, and the CDRL3 comprises or consists of the amino acid sequence of SEQ ID NO: 101; or
comprises or consists of an amino acid sequence set forth in SEQ ID NO: 49.

In some embodiments, the second antigen-binding region specifically binding to PSMA comprises three complementarity-determining regions from a heavy chain variable region (CDRHs): CDRH1, CDRH2, and CDRH3, and three complementarity-determining regions from a light chain variable region (CDRLs): CDRL1, CDRL2, and CDRL3, wherein the CDRH1 comprises or consists of the amino acid sequence of SEQ ID NO: 96; the CDRH2 comprises or consists of the amino acid sequence of SEQ ID NO: 97; the CDRH3 comprises or consists of the amino acid sequence of SEQ ID NO: 98; the CDRL1 comprises or consists of the amino acid sequence of SEQ ID NO: 99; the CDRL2 comprises or consists of the amino acid sequence of SEQ ID NO: 100; and the CDRL3 comprises or consists of the amino acid sequence of SEQ ID NO: 101.

In some embodiments, the second antigen-binding region specifically binding to PSMA comprises a VH and a VL, wherein the VH comprises or consists of a sequence set forth in SEQ ID NO: 47; and the VL comprises or consists of a sequence set forth in SEQ ID NO: 49.

In some embodiments, the second antigen-binding region specifically binding to PSMA is a Fab fragment.

### III-2 Fab fragment

In some embodiments, the first antigen-binding region and/or the second antigen-binding region of the present invention are Fab fragments. The Fab fragments suitable for use as the antigen-binding regions of the multispecific binding molecule of the present invention, e.g., a bispecific antibody, consist of two polypeptide chains comprising VH, CH1, VL, and CL domains of the antibody, wherein the VH is paired with the VL, and the CH1 is paired with the CL to form the antigen-binding region. In some embodiments, in the Fab, one chain comprises or consists of, from N-terminus to C-terminus, a VH and a CH1 (i.e., VH-CH1), and the other chain comprises or consists of, from N-terminus to C-terminus, a VL and a CL (i.e., VL-CL). In some embodiments, in the multispecific antibody of the present invention, the Fab may be linked to the N-terminus of the Fc domain of the antibody via the C-terminus of the chain comprising the VH. Preferably, the Fab comprises a VH-CH1 chain and a VL-CL chain and may be linked to the Fc domain of the antibody via the C-terminus of the CH1 of the VH-CH1 chain. In some embodiments, the linkage is direct or via a linker. Herein, a Fab chain comprising or consisting of VH-CH1 is also referred to as a Fab heavy chain, and a Fab chain comprising or consisting of VL-CL is also referred to as a Fab light chain.

In some embodiments, the CH1 is a CH1 from IgG1, IgG2, IgG3, or IgG4, preferably a CH1 from IgG1. In some embodiments, the CH1
(i) comprises or consists of an amino acid sequence having at least 85%, 90%, 91%, 92%, 93%, 94%, 95%, 96%, 97%, 98%, or 99% identity to the amino acid sequence of SEQ ID NO: 74;
(ii) comprises or consists of the amino acid sequence of SEQ ID NO: 74; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 74.

In some embodiments, the CL is a Kappa light chain constant region or a Lambda light chain constant region. In some embodiments, the CL is a Kappa light chain constant region. In some embodiments, the CL
(i) comprises or consists of an amino acid sequence having at least 85%, 90%, 91%, 92%, 93%, 94%, 95%, 96%, 97%, 98%, or 99% identity to the amino acid sequence of SEQ ID NO: 30;
(ii) comprises or consists of the amino acid sequence of SEQ ID NO: 30; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 30.

In some embodiments, the first antigen-binding region is a Fab specifically binding to CD28, wherein the Fab fragment is from the anti-CD28 antibody of the present invention and comprises the heavy chain variable region VH and the light chain variable region VL of the anti-CD28 antibody of the present invention. In some embodiments, the Fab heavy chain of the Fab specifically binding to CD28 as the first antigen-binding region comprises or consists of a VH and a CH1, wherein the VH is the VH of the anti-CD28 antibody of the present invention. In some embodiments, the Fab light chain of the Fab specifically binding to CD28 as the first antigen-binding region comprises or consists of a VL and a CL, wherein the VL is the VL of the anti-CD28 antibody of the present invention.

In some embodiments, the Fab heavy chain specifically binding to CD28
(i) comprises or consists of an amino acid sequence having at least 85%, 90%, 91%, 92%, 93%, 94%, 95%, 96%, 97%, 98%, or 99% identity to the amino acid sequence of SEQ ID NO: 103, 104, or 105;
(ii) comprises or consists of the amino acid sequence of SEQ ID NO: 103, 104, or 105; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 103, 104, or 105.

In some embodiments, the Fab light chain specifically binding to CD28
(i) comprises or consists of an amino acid sequence having at least 85%, 90%, 91%, 92%, 93%, 94%, 95%, 96%, 97%, 98%, or 99% identity to the amino acid sequence of SEQ ID NO: 36, 38, or 40;
(ii) comprises or consists of the amino acid sequence of SEQ ID NO: 36, 38, or 40; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 36, 38, or 40.

In some embodiments, the Fab heavy chain specifically binding to CD28 comprises or consists of an amino acid sequence set forth in SEQ ID NO: 103, and the Fab light chain comprises or consists of an amino acid sequence set forth in SEQ ID NO: 36;

In some embodiments, the Fab heavy chain specifically binding to CD28 comprises or consists of an amino acid sequence set forth in SEQ ID NO: 104, and the Fab light chain comprises or consists of an amino acid sequence set forth in SEQ ID NO: 38; or

In some embodiments, the Fab heavy chain specifically binding to CD28 comprises or consists of an amino acid sequence set forth in SEQ ID NO: 105, and the Fab light chain comprises or consists of an amino acid sequence set forth in SEQ ID NO: 40.

In some embodiments, the second antigen-binding region is a Fab specifically binding to TAA, wherein the Fab fragment is from an anti-TAA antibody and comprises the heavy chain variable region VH and the light chain variable region VL of the anti-TAA antibody (e.g., an anti-CLDN18.2 antibody or an anti-PSMA antibody). In some embodiments, the second antigen-binding region is a Fab specifically binding to CLDN18.2, wherein the Fab fragment is from an anti-CLDN18.2 antibody and comprises the heavy chain variable region VH and the light chain variable region VL of the anti-CLDN18.2 antibody. In some embodiments, the second antigen-binding region is a Fab specifically binding to PSMA, wherein the Fab fragment is from an anti-PSMA antibody and comprises the heavy chain variable region VH and the light chain variable region VL of the anti-PSMA antibody.

In some embodiments, the Fab as the second antigen-binding region comprises a VH or a VL of the second antigen-binding region described herein, or comprises a VH and a VL of the second antigen-binding region described herein.

In some embodiments, the Fab heavy chain specifically binding to CLDN18.2
(i) comprises or consists of an amino acid sequence having at least 85%, 90%, 91%, 92%, 93%, 94%, 95%, 96%, 97%, 98%, or 99% identity to the amino acid sequence of SEQ ID NO: 102;
(ii) comprises or consists of the amino acid sequence of SEQ ID NO: 102; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 102.

In some embodiments, the Fab light chain specifically binding to CLDN18.2
(i) comprises or consists of an amino acid sequence having at least 85%, 90%, 91%, 92%, 93%, 94%, 95%, 96%, 97%, 98%, or 99% identity to the amino acid sequence of SEQ ID NO: 34;
(ii) comprises or consists of the amino acid sequence of SEQ ID NO: 34; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 34.

In some embodiments, the Fab heavy chain specifically binding to CLDN18.2 comprises or consists of an amino acid sequence set forth in SEQ ID NO: 102, and the Fab light chain comprises or consists of an amino acid sequence set forth in SEQ ID NO: 34.

In some embodiments, the Fab heavy chain specifically binding to PSMA
(i) comprises or consists of an amino acid sequence having at least 85%, 90%, 91%, 92%, 93%, 94%, 95%, 96%, 97%, 98%, or 99% identity to the amino acid sequence of SEQ ID NO: 106;
(ii) comprises or consists of the amino acid sequence of SEQ ID NO: 106; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 106.

In some embodiments, the Fab light chain specifically binding to PSMA
(i) comprises or consists of an amino acid sequence having at least 85%, 90%, 91%, 92%, 93%, 94%, 95%, 96%, 97%, 98%, or 99% identity to the amino acid sequence of SEQ ID NO: 50;
(ii) comprises or consists of the amino acid sequence of SEQ ID NO: 50; or
(iii) comprises or consists of an amino acid sequence having one or more (preferably no more than 10 or 10, and more preferably no more than 5, 4, 3, 2, or 1) amino acid alterations (preferably amino acid substitutions, and more preferably conservative amino acid substitutions) compared with the amino acid sequence of SEQ ID NO: 50.

In some embodiments, the Fab heavy chain specifically binding to PSMA comprises or consists of an amino acid sequence set forth in SEQ ID NO: 106, and the Fab light chain comprises or consists of an amino acid sequence set forth in SEQ ID NO: 50.

### III-3 Fc region

In some embodiments, one or more target-binding regions (antigen-binding regions) of the multispecific binding molecule of the present invention, such as a multispecific antibody (e.g., a bispecific antibody), further comprise Fc regions, wherein the Fc regions contained may be the same or different.

In some embodiments, the first Fc region and the second Fc region are different and are capable of dimerizing to form a heterodimeric Fc scaffold.

Herein, the Fc region refers to a C-terminal region of an immunoglobulin heavy chain that contains at least a portion of the constant region, and may include Fc regions of native sequences and variant Fc regions. The F regions of native sequences encompass naturally occurring Fc sequences of various immunoglobulins, such as Fc regions of various Ig subclasses or allotypes thereof (Gestur Vidarsson et al., IgG subclasses and allotypes: from structure to effector functions, 20 October 2014, doi: 10.3389/fimmu.2014.00520). In some embodiments, the Fc region of the present invention comprises a CH2 and a CH3 of the antibody. In some embodiments, the antibody Fc region may also bear an IgG hinge region or a part of the IgG hinge region at the N-terminus, e.g., an IgG1 hinge region or a part of the IgG1 hinge region, e.g., according to EU numbering, a sequence of D221 to P230. The hinge region may comprise a mutation.

Unless otherwise indicated herein, amino acid residues in the Fc region are numbered according to the EU numbering system, also known as the EU index, as described in Kabat, E.A. et al., Sequences of Proteins of Immunological Interest, 5th Ed., Public Health Service, National Institutes of Health, Bethesda, MD (1991), NIH Publication 91-3242.

In some embodiments, the Fc region is human IgG Fc, e.g., human IgG1 Fc, human IgG2 Fc, human IgG3 Fc, or human IgG4 Fc. In one embodiment, the Fc region comprises or consists of the amino acid sequence of SEQ ID NO: 94 or 95 or an amino acid sequence having at least 90% identity, e.g., 95%, 96%, 97%, 98%, 99% or higher identity thereto.

As understood by those skilled in the art, to facilitate the formation of the multispecific antibody of the present invention as a heterodimer, the Fc region contained in the multispecific antibody of the present invention may comprise mutations that favor the heterodimerization of the first Fc region and the second Fc region. In one embodiment, mutations are introduced into the CH3 regions of the two Fc regions.

Methods for promoting the heterodimerization of the Fc regions are known in the art. For example, the CH3 region of the first Fc region and the CH3 region of the second Fc region are engineered in a complementary manner, such that each CH3 region (or the heavy chain comprising the same) can no longer homodimerize with itself but is forced to heterodimerize with other CH3 regions that are complementarily engineered (such that heterodimerization occurs between the CH3 regions of the first and second Fc regions and no homodimers are formed between the two first CH3 regions or the two second CH3 regions). For example, corresponding Knob and Hole mutations are introduced into the first Fc region and the second Fc region based on the Knob-into-Hole technique. See, for example, US 5,731,168; US 7,695,936; Ridgway et al., Prot Eng 9, 617-621 (1996) and Carter, J Immunol Meth 248, 7-15 (2001).

Corresponding mutations can also be introduced into the first Fc region and the second Fc region based on the Innobody technology. See, for example, PCT/CN2021/143141(said patent incorporated herein by reference in its entirety).

In one specific embodiment, the first CH3 region comprises an S364R/K mutation (preferably S364R), and optionally one or more additional mutations. In some embodiments, the second CH3 region comprises a K370S/T/A/V mutation (preferably K370S), and optionally one or more additional mutations. In some embodiments, the first CH3 region comprises an S364R/K mutation, and the second CH3 region comprises a K370S/T/A/V mutation. In some embodiments, the first CH3 region comprises an S364R mutation, and the second CH3 region comprises a K370S mutation.

In some embodiments, the first CH3 region comprises an S364R/K (preferably S364R) mutation and a D399K/R (preferably D399K) mutation. In some embodiments, the second CH3 region comprises a K370S/T/A/V (preferably K370S) mutation and a K409D/E (preferably K409D) mutation. In some embodiments, the first CH3 region comprises S364R/K+D399K/R, and the second CH3 region comprises K370S/T/A/V+Y349T/S/A/V. In some embodiments, the first CH3 region comprises S364R+D399K, and the second CH3 region comprises K370S+Y349T. In some embodiments, the first CH3 region further comprises E375N/Q (preferably E375N) and/or T350V/A (preferably T350V). In some embodiments, the second CH3 region further comprises K409D/E (preferably K409D), Q347D/E (preferably Q347D), and/or T350V/A (preferably T350V).

In some embodiments, the first CH3 region comprises S364R+D399K, and the second CH3 region comprises K370S+Y349T+K409D. In some embodiments, the first CH3 region further comprises E357N. In some embodiments, the second CH3 region further comprises Q347D. In some embodiments, the first CH3 region further comprises E357N, and the second CH3 region further comprises Q347D. In some embodiments, the first CH3 region and the second CH3 region each further comprise T350V, or both comprise T350V.

Thus, in some embodiments, the first CH3 region comprises S364R+D399K, and the second CH3 region comprises K370S+Y349T+K409D+Q347D. In some embodiments, the first CH3 region comprises S364R+D399K+E357N, and the second CH3 region comprises K370S+Y349T+K409D+Q347D. In some embodiments, the first CH3 region comprises S364R+D399K+E357N+T350V, and the second CH3 region comprises K370S+Y349T+K409D+Q347D+T350V.

In some embodiments, the first CH3 region comprises K409E/D (preferably K409E). In some embodiments, the second CH3 region comprises D399K/R (preferably D399K) or K370T/S/A/V (preferably K370T). In some embodiments, the first CH3 region comprises K409E/D (preferably K409E), and the second CH3 region comprises D399K/R (preferably D399K). In some embodiments, the first CH3 region further comprises T411R/K (preferably T411R). In some embodiments, the second CH3 region further comprises K370T/S/A/V (preferably K370T). In some embodiments, the CH3 region comprises K409E/D+T411R/K, and the second CH3 region comprises D399K/R+K370T/S/A/V. In some embodiments, the CH3 region comprises K409E+T411R, and the second CH3 region comprises D399K+K370T.

In some particular embodiments, the first CH3 region and the second CH3 region have the following 5 mutation combinations:

| First CH3 region | Second CH3 region |
|---|---|
| S364R, D399K, E357N, T350V | K370S, Y349T, Q347D, K409D, T350V |
| K409E, T411R | D399K, K370T |
| S364R, D399K, E357N | K370S, Y349T, Q347D, K409D |
| S364R, D399K | K370S, Y349T, Q347D, K409D |
| S364R, D399K | K370S, Y349T, K409D |

In one embodiment, the CH3 of one Fc region comprises S364R and D399K mutations, and the CH3 mutation of the other Fc region comprises Y349T, K370S, and K409D mutations.

In some embodiments, the Fc region further comprises other mutations that facilitate heterodimer purification.

The Fc region in the binding molecule (such as an antibody molecule) of the present invention can also be mutated to obtain desired properties. Mutations to the Fc region are known in the art.

In one embodiment, the Fc region is modified in properties of effector functions of the Fc region (e.g., complement activation functions of the Fc region). In one embodiment, the effector functions have been reduced or eliminated relative to a wild-type isotype Fc region. In one embodiment, the effector function is reduced or eliminated by using a method selected from: using an Fc isotype that naturally has a reduced or an eliminated effector function, and modifying the Fc region.

In one preferred embodiment, the Fc region has reduced effector functions mediated by the Fc region, such as reduced or eliminated ADCC or ADCP or CDC effector functions, for example, comprising mutations for achieving the above functions.

As understood by those skilled in the art, according to the intended use of the binding molecule (such as an antibody molecule) of the present invention, the binding molecule (such as an antibody molecule) of the present invention may also comprise modifications to the Fc domain that alter the binding affinity for one or more Fc receptors. In one embodiment, the Fc receptor is an Fcγ receptor, particularly a human Fcγ receptor. In some embodiments, the Fc region comprises a mutation that reduces the binding to Fcγ receptors. For example, in some embodiments, the Fc region used in the present invention has a mutation that reduces the binding to Fcγ receptors, e.g., mutations L234A/L235A or a deletion of proline at position P329 (P329delP). In yet another preferred embodiment, the Fc fragment may have a mutation that results in an increased serum half-life, e.g., a mutation that improves the binding of the Fc fragment to FcRn.

Thus, in one specific embodiment, the two Fc regions comprised in the antibody of the present invention, such as a multispecific antibody, are heterodimerized, wherein

one Fc-region polypeptide comprises mutations S364R and D399K, and the other Fc-region polypeptide comprises mutations Y349T, K370S, and K409D.

In some embodiments, the Fc region further comprises mutations L234A/L235A, and optionally a deletion of proline at position P329 (P329delP).

Thus, in one specific embodiment, the two Fc regions comprised in the antibody of the present invention, such as a multispecific antibody, are heterodimerized, wherein
a) one Fc region comprises or consists of an amino acid sequence set forth in SEQ ID NO: 80, and the other Fc region polypeptide comprises or consists of an amino acid sequence set forth in SEQ ID NO: 81; or
b) one Fc region comprises or consists of an amino acid sequence set forth in SEQ ID NO: 82, and the other Fc region polypeptide comprises or consists of an amino acid sequence set forth in SEQ ID NO: 83.

In one specific embodiment, the two Fc regions comprised in the antibody of the present invention, such as a multispecific antibody, are heterodimerized, wherein
a) one Fc region polypeptide comprises an amino acid sequence having at least 85%, 90%, 95%, 96%, 97%, 98%, or 99% identity to the amino acid sequence set forth in SEQ ID NO: 80, and; or
b) one Fc region polypeptide comprises an amino acid sequence having at least 85%, 90%, 95%, 96%, 97%, 98%, or 99% identity to the amino acid sequence set forth in SEQ ID NO: 82, and the other Fc region polypeptide comprises an amino acid sequence having at least 85%, 90%, 95%, 96%, 97%, 98%, or 99% identity to the amino acid sequence set forth in SEQ ID NO: 83.

Thus, in one specific embodiment, the two Fc regions comprised in the binding molecule of the present invention, e.g., in an antibody such as a multispecific antibody, are heterodimerized, wherein
a) one Fc region comprises an amino acid sequence having at least 85%, 90%, 95%, 96%, 97%, 98%, or 99% identity to the amino acid sequence set forth in SEQ ID NO: 80 and comprises mutations S364R and D399K, and the other Fc region polypeptide comprising an amino acid sequence having at least 85%, 90%, 95%, 96%, 97%, 98%, or 99% identity to the amino acid sequence set forth in SEQ ID NO: 81 and comprises mutations Y349T, K370S, and K409D;
b) one Fc region comprises an amino acid sequence having at least 85%, 90%, 95%, 96%, 97%, 98%, or 99% identity to the amino acid sequence set forth in SEQ ID NO: 80 and comprises mutations S364R and D399K as well as mutations L234A/L235A and P329delP, and the other Fc region comprises an amino acid sequence having at least 85%, 90%, 95%, 96%, 97%, 98%, or 99% identity to the amino acid sequence set forth in SEQ ID NO: 81 and comprises mutations Y349T, K370S and K409D as well as mutations L234A/L235A and P329delP;
c) one Fc region comprises an amino acid sequence having at least 85%, 90%, 95%, 96%, 97%, 98%, or 99% identity to the amino acid sequence set forth in SEQ ID NO: 82 and comprises mutations S364R and D399K, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, and K409D; or
d) one Fc region comprises an amino acid sequence having at least 85%, 90%, 95%, 96%, 97%, 98%, or 99% identity to the amino acid sequence set forth in SEQ ID NO: 82 and comprises mutations S364R, D399K, and L234A/L235A, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, K409D, and L234A/L235A.

### III-4. Exemplary bispecific antibody

In some embodiments, the bispecific antibody of the present invention comprises the first antigen-binding region specifically binding to the first antigen described herein and the second antigen-binding region specifically binding to the second antigen described herein, wherein the first antigen-binding region comprises a first Fab and the second antigen-binding region comprises a second Fab, wherein the first Fab is linked at the C-terminus of its CH1 to the N-terminus of the first Fc region (directly or via a linker such as a hinge region), and the second Fab is linked at the C-terminus of its CH1 to the N-terminus of the second Fc region (directly or via a linker such as a hinge region). In one specific embodiment, the first Fab and the first Fc region constitute a first half antibody binding to a first antigen, the second Fab and the second Fc region constitute a second half antibody binding to a second antigen, and the first half antibody and the second half antibody constitute the bispecific antibody of the present invention.

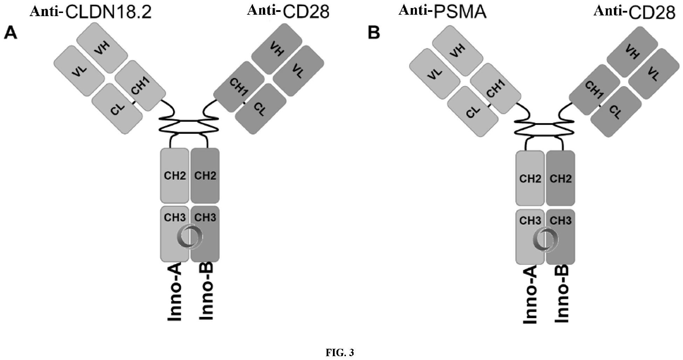

In one specific embodiment, the bispecific antibody is an IgG-like antibody having a configuration shown in FIG. 3. In one specific embodiment, the first Fc region and the second Fc region comprise Innobody mutations, and optionally a mutation that reduces effector function mediated by the Fc region or a mutation that reduces binding to Fcγ receptors. In one embodiment, the bispecific antibody comprises or consists of:
heavy chain 1: comprising or consisting of, from N-terminus to C-terminus, a first Fab heavy chain variable region-a heavy chain constant region CH1-a first Fc region, wherein the heavy chain constant region CH1 is linked at its C-terminus to the N-terminus of the first Fc region directly or via a linker (e.g., a hinge region);
light chain 1: comprising or consisting of, from N-terminus to C-terminus, a first Fab light chain variable region-a light chain constant region;
heavy chain 2: comprising or consisting of, from N-terminus to C-terminus, a second Fab heavy chain variable region-a heavy chain constant region CH1-a second Fc region, wherein the heavy chain constant region CH1 is linked at its C-terminus to the N-terminus of the second Fc region directly or via a linker (e.g., a hinge region);
light chain 2: comprising or consisting of, from N-terminus to C-terminus: a second Fab light chain variable region-a light chain constant region;
preferably, the domains are directly linked to each other.

In some embodiments, the Fab heavy chain variable region and the heavy chain constant region CH1 constitute a Fab heavy chain, i.e., the C-terminus of the Fab heavy chain variable region is linked (directly linked) to the N-terminus of the heavy chain constant region CH1. In some embodiments, the Fab light chain variable region and the light chain constant region constitute the Fab light chain, i.e., the C-terminus of the Fab light chain variable region is linked (directly linked) to the N-terminus of the light chain constant region.

In one specific embodiment, the first Fab is the Fab of the anti-CD28 antibody of the present invention, and the second Fab is the Fab of the anti-TAA antibody, or vice versa.

In one specific embodiment, the first Fab is the Fab of the anti-CD28 antibody of the present invention, and the second Fab is the Fab of the anti-CLDN18.2 antibody, or vice versa.

In one specific embodiment, the bispecific antibody of the present invention specifically binds to CD28 and CLDN18.2 and comprises or consists of:
heavy chain 1: comprising or consisting of, from N-terminus to C-terminus: a Fab heavy chain variable region specifically binding to CD28-a heavy chain constant region CH1-a first Fc region, wherein the heavy chain constant region CH1 is linked at its C-terminus to the N-terminus of the first Fc region directly or via a linker (e.g., a hinge region);
light chain 1: comprising or consisting of, from N-terminus to C-terminus: a Fab light chain variable region specifically binding to CD28-a light chain constant region;
heavy chain 2: comprising or consisting of, from N-terminus to C-terminus: a Fab heavy chain variable region specifically binding to CLDN18.2-a heavy chain constant region CH1-a second Fc region, wherein the heavy chain constant region CH1 is linked at its C-terminus to the N-terminus of the second Fc region directly or via a linker (e.g., a hinge region); and/or
light chain 2: comprising or consisting of, from N-terminus to C-terminus, : a Fab light chain variable regionspecifically binding to CLDN18.2-a light chain constant region,
optionally, the first Fc region comprises mutations Y349T, K370S, and K409D, and the second Fc region comprises S364R and D399K, or vice versa; optionally, the first Fc region and the second Fc region each further comprise mutations L234A/L235A, and optionally a P329delP mutation;
preferably, the domains are directly linked to each other.

In some particular embodiments, the bispecific antibody that specifically binds to CD28 and CLDN18.2 comprises or consists of a heavy chain 1, a heavy chain 2, a light chain 1, and a light chain 2, wherein
the heavy chain 1 comprises or consists of an amino acid sequence set forth in SEQ ID NO: 35, 37, or 39 or an amino acid sequence having at least 85%, 90%, 95%, 96%, 97%, 98%, or 99% identity thereto;
the light chain 1 comprises or consists of an amino acid sequence set forth in SEQ ID NO: 36, 38, or 40 or an amino acid sequence having at least 85%, 90%, 95%, 96%, 97%, 98%, or 99% identity thereto;
the heavy chain 2 comprises or consists of an amino acid sequence set forth in SEQ ID NO: 33 or an amino acid sequence having at least 85%, 90%, 95%, 96%, 97%, 98%, or 99% identity thereto; and/or
the light chain 2 comprises or consists of an amino acid sequence set forth in SEQ ID NO: 34 or an amino acid sequence having at least 85%, 90%, 95%, 96%, 97%, 98%, or 99% identity thereto.

In some particular embodiments, the bispecific antibody that specifically binds to CD28 and CLDN18.2 comprises or consists of a heavy chain 1, a heavy chain 2, a light chain 1, and a light chain 2, wherein
the heavy chain 1 comprises or consists of an amino acid sequence set forth in SEQ ID NO: 35 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto and the light chain 2 comprises or consists of an amino acid sequence set forth in SEQ ID NO: 34 or an amino acid sequence having at least 85%, 90%, 95%, 96%, 97%, 98%, or 99% identity thereto;
the heavy chain 1 comprises or consists of an amino acid sequence set forth in SEQ ID NO: 37 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto; or
the heavy chain 1 comprises or consists of an amino acid sequence set forth in SEQ ID NO: 39 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto.

In some particular embodiments, the bispecific antibody that specifically binds to CD28 and CLDN18.2 comprises or consists of a heavy chain 1, a heavy chain 2, a light chain 1, and a light chain 2, wherein
the heavy chain 1 comprises or consists of an amino acid sequence set forth in SEQ ID NO: 35; the light chain 1 comprises or consists of an amino acid sequence set forth in SEQ ID NO: 36; the heavy chain 2 comprises or consists of an amino acid sequence set forth in SEQ ID NO: 33; and the light chain 2 comprises or consists of an amino acid sequence set forth in SEQ ID NO: 34;
the heavy chain 1 comprises or consists of an amino acid sequence set forth in SEQ ID NO: 37; the light chain 1 comprises or consists of an amino acid sequence set forth in SEQ ID NO: 38; the heavy chain 2 comprises or consists of an amino acid sequence set forth in SEQ ID NO: 33; and the light chain 2 comprises or consists of an amino acid sequence set forth in SEQ ID NO: 34; or
the heavy chain 1 comprises or consists of an amino acid sequence set forth in SEQ ID NO: 39; the light chain 1 comprises or consists of an amino acid sequence set forth in SEQ ID NO: 40; the heavy chain 2 comprises or consists of an amino acid sequence set forth in SEQ ID NO: 33; and the light chain 2 comprises or consists of an amino acid sequence set forth in SEQ ID NO: 34.

In one specific embodiment, the first Fab is the anti-CD28 Fab of the present invention, and the second Fab is the anti-PSMA Fab, or vice versa.

In one specific embodiment, the bispecific antibody of the present invention specifically binds to CD28 and PSMA and comprises or consists of the following:
heavy chain 1: comprising or consisting of, from N-terminus to C-terminus: a Fab heavy chain variable region specifically binding to CD28-a heavy chain constant region CH1-a first Fc region, wherein the heavy chain constant region CH1 is linked at its C-terminus to the N-terminus of the first Fc region directly or via a linker (e.g., a hinge region);
light chain 1: comprising or consisting of, from N-terminus to C-terminus: a Fab light chain variable region specifically binding to CD28-a light chain constant region;
heavy chain 2: comprising or consisting of, from N-terminus to C-terminus: a Fab heavy chain variable region specifically binding to PSMA-a heavy chain constant region CH1-a second Fc region, wherein the heavy chain constant region CH1 is linked at its C-terminus to the N-terminus of the second Fc region directly or via a linker (e.g., a hinge region); and/or
light chain 2: comprising or consisting of, from N-terminus to C-terminus: a Fab light chain variable region specifically binding to PSMA-a light chain constant region,
optionally, the first Fc region comprises mutations Y349T, K370S, and K409D, and the second Fc region comprises S364R and D399K, or vice versa; optionally, the first Fc region and the second Fc region each further comprise mutations L234A/L235A, and optionally a P329delP mutation;
preferably, the domains are directly linked to each other.

In some particular embodiments, the bispecific antibody that specifically binds to CD28 and PSMA comprises or consists of a heavy chain 1, a heavy chain 2, a light chain 1, and a light chain 2, wherein
the heavy chain 1 comprises or consists of an amino acid sequence set forth in SEQ ID NO: 51, 52, or 53 or an amino acid sequence having at least 85%, 90%, 95%, 96%, 97%, 98%, or 99% identity thereto;
the light chain 1 comprises or consists of an amino acid sequence set forth in SEQ ID NO: 36, 38, or 40 or an amino acid sequence having at least 85%, 90%, 95%, 96%, 97%, 98%, or 99% identity thereto;
the heavy chain 2 comprises or consists of an amino acid sequence set forth in SEQ ID NO: 48 or an amino acid sequence having at least 85%, 90%, 95%, 96%, 97%, 98%, or 99% identity thereto; and/or
the light chain 2 comprises or consists of an amino acid sequence set forth in SEQ ID NO: 50 or an amino acid sequence having at least 85%, 90%, 95%, 96%, 97%, 98%, or 99% identity thereto.

In some particular embodiments, the bispecific antibody that specifically binds to CD28 and PSMA comprises or consists of a heavy chain 1, a heavy chain 2, a light chain 1, and a light chain 2, wherein
the heavy chain 1 comprises or consists of an amino acid sequence set forth in SEQ ID NO: 51 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto;
the heavy chain 1 comprises or consists of an amino acid sequence set forth in SEQ ID NO: 52 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists ofan amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto; or
the heavy chain 1 comprises or consists of an amino acid sequence set forth in SEQ ID NO: 53 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto.

In some particular embodiments, the bispecific antibody that specifically binds to CD28 and PSMA comprises or consists of a heavy chain 1, a heavy chain 2, a light chain 1, and a light chain 2, wherein
the heavy chain 1 comprises or consists of an amino acid sequence set forth in SEQ ID NO: 51; the light chain 1 comprises or consists of an amino acid sequence set forth in SEQ ID NO: 36; the heavy chain 2 comprises or consists of an amino acid sequence set forth in SEQ ID NO: 48; and the light chain 2 comprises or consists of an amino acid sequence set forth in SEQ ID NO: 50;
the heavy chain 1 comprises or consists of an amino acid sequence set forth in SEQ ID NO: 52; the light chain 1 comprises or consists of an amino acid sequence set forth in SEQ ID NO: 38; the heavy chain 2 comprises or consists of an amino acid sequence set forth in SEQ ID NO: 48; and the light chain 2 comprises or consists of an amino acid sequence set forth in SEQ ID NO: 50; or
the heavy chain 1 comprises or consists of an amino acid sequence set forth in SEQ ID NO: 53; the light chain 1 comprises or consists of an amino acid sequence set forth in SEQ ID NO: 40; the heavy chain 2 comprises or consists of anor consists of an amino acid sequence set forth in SEQ ID NO: 48; and the light chain 2 comprises or consists of an amino acid sequence set forth in SEQ ID NO: 50.

The various components/domains described herein for the multispecific binding molecule such as a bispecific antibody are as described herein. It will be appreciated by those skilled in the art that any combination of any of the technical features described herein for the various components of the multispecific binding molecule, e.g., bispecific antibody, is within the scope of the present invention, unless otherwise expressly stated to the contrary. Furthermore, it will be understood by those skilled in the art that the antibody of the present invention (including any form of antibody) may comprise any such combination, unless otherwise expressly stated to the contrary.

### IV. Polynucleotide, Vector and Host Cell

The present invention provides a nucleic acid encoding any of the above antibody molecules of the present invention (e.g., an anti-CD28 antibody or an antigen-binding fragment thereof, or a multispecific antibody such as a bispecific antibody of the present invention).

In one aspect, the present invention provides a nucleic acid encoding any of the above anti-CD28 antibodies or antigen-binding fragments thereof, or multispecific antibodies such as bispecific antibodies.

For ease of production and purification, the anti-CD28 antibody or the antigen-binding fragment thereof or the multispecific antibody such as a bispecific antibody may be fused at the N-terminus or C-terminus (e.g., C-terminus) to a secretory signal peptide, and/or a tag peptide that facilitates purification, such as a hexahistidine tag or a biotin tag.

As will be appreciated by those skilled in the art, each antibody or polypeptide amino acid sequence can be encoded by a variety of nucleic acid sequences because of codon degeneracy.

In some embodiments, the nucleic acid of the present invention comprises a nucleic acid encoding an amino acid sequence selected from any one of SEQ ID NOs: 4, 5, 9, 13, 17, 19, 23, 24, 28, 33, 35, 36-40, 48, 51-53, 69, 70, 72, 73, 75, 78, and 102-106, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 4, 5, 9, 13, 17, 19, 23, 24, 28, 33, 35, 36-40, 48, 51-53, 69, 70, 72, 73, 75, 78, and 102-106.

Nucleic acid sequences encoding the molecules of the present invention may be produced using methods well known in the art, for example by de novo solid phase DNA synthesis, or by PCR amplification.

In one aspect, the present invention also provides a vector comprising a nucleic acid of the present invention. In one embodiment, the vector is an expression vector, e.g., a prokaryotic expression vector or a eukaryotic expression vector. The vector includes, but is not limited to, a virus, a plasmid, a cosmid, a lambda phage, or a yeast artificial chromosome (YAC). In a preferred embodiment, the expression vector is pCDNA, such as pCDNA3.1.

In one aspect, the present invention also provides a host cell comprising the nucleic acid or the vector. In one embodiment, the host cell is eukaryotic. In another embodiment, the host cell is selected from a yeast cell and a mammalian cell (e.g., a CHO cell or a 293 cell, such as a HEK 293 or 293F cell, or a 293FT cell, or an Expi293 cell). In another embodiment, the host cell is prokaryotic.

Suitable host cells include prokaryotic microorganisms such as E. coli, eukaryotic microorganisms such as filamentous fungi or yeast, or various eukaryotic cells such as Chinese hamster ovary cells (CHO) and insect cells. Mammalian cell lines suitable for suspension culture can be used. Examples of useful mammalian host cell lines include monkey kidney CV1 line (COS-7) transformed by SV40; human embryonic kidney line (HEK 293 or 293F cells or 293FT cells or Expi293 cells), baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), CHO cells, NSO cells, and myeloma cell lines such as YO, NS0, P3X63, and Sp2/0. Mammalian host cell lines suitable for antibody production are known in the art. In a preferred embodiment, the host cell is a CHO or HEK293 cell, or a 293FT cell, or an Expi293 cell.

### V.Production and Purification of Molecules of the Present Invention

In yet another aspect, the present invention provides a method for producing the antibody molecule (e.g., an anti-CD28 antibody or an antigen-binding fragment thereof, or a multispecific antibody, such as a bispecific antibody) of the present invention, the method comprising: culturing a host cell comprising a polynucleotide encoding the polypeptide chains of the molecule under conditions suitable for the expression of the polypeptide chains; and optionally further comprising assembling the polypeptide chains to produce the molecule under conditions suitable for the assembly of the polypeptide chains into the molecule.

For recombinant production, the polynucleotides encoding the polypeptide chains of the molecule of the present invention may be inserted into one or more vectors for further cloning and/or expression in host cells. Methods which are well known to those skilled in the art can be used to construct expression vectors. The expression vector includes, but is not limited to, a virus, a plasmid, a cosmid, a lambda phage, or a yeast artificial chromosome (YAC). Once an expression vector comprising one or more polynucleotides of the present invention has been prepared for expression, the expression vector can be transfected or introduced into a suitable host cell. Various techniques can be used for this purpose, for example, protoplast fusion, calcium phosphate precipitation, electroporation, retroviral transduction, viral transfection, biolistics, liposome-based transfection, or other conventional techniques.

The molecule prepared as described herein can be purified by known prior art such as high-performance liquid chromatography, ion exchange chromatography, gel electrophoresis, affinity chromatography (e.g., Protein A affinity chromatography), and size exclusion chromatography. The actual conditions used to purify a particular protein also depend on factors such as net charge, hydrophobicity, and hydrophilicity, and these will be apparent to those skilled in the art.

The purity of the molecules of the present invention can be determined by any of a variety of well-known analytical methods including size exclusion chromatography, gel electrophoresis, high performance liquid chromatography, and the like. The physical/chemical properties and/or biological activities of the antibody molecule provided herein can be identified, screened, or characterized through a variety of assays known in the art.

### VI. Assay

The molecules provided herein (e.g., an anti-CD28 antibody or an antigen-binding fragment thereof, or a multispecific antibody such as a bispecific antibody) can be identified, screened, or characterized for their physical/chemical properties and/or biological activities using various assays known in the art. Assays for determining the properties of the anti-CD28 antibody or the antigen-binding fragment thereof or the bispecific antibody are exemplified in the Examples, including bio-layer interferometry (BLI), a T cell reporter gene assay, a T cell activation assay, a T cell-mediated killing assay, an anti-tumor activity assay, and the like.

### VII. Immunoconjugate

In one aspect, the present invention provides an immunoconjugate comprising the molecule of the present invention (e.g., an anti-CD28 antibody or an antigen-binding fragment thereof, or a multispecific antibody such as a bispecific antibody) and one or more additional active ingredients (e.g., an active ingredient from a drug or therapeutic agent for treating a disease of the present invention, such as a small molecule that enhances the therapeutic effect of the molecule of the present invention).

In some embodiments, the immunoconjugate is an antibody-drug-conjugate (ADC).

### VIII. Medicament and Use

In some embodiments, the present invention further relates to a composition (e.g., a pharmaceutical composition or a pharmaceutical formulation) comprising the molecule of the present invention (e.g., an anti-CD28 antibody or an antigen-binding fragment thereof, a multispecific antibody such as a bispecific antibody, or an immunoconjugate).

In one embodiment, the composition further comprises a pharmaceutically acceptable auxiliary material, such as a pharmaceutically acceptable carrier, a pharmaceutically acceptable excipient, including a buffer, as known in the art. In one embodiment, the composition, e.g., the pharmaceutical composition, comprises the molecule of the present invention, and a combination of one or more additional therapeutic agents.

The composition of the present invention may further comprise a suitable pharmaceutical auxiliary material, such as a pharmaceutical carrier and a pharmaceutical excipient, including a buffer, as known in the art. As used herein, a "pharmaceutical carrier" includes any and all solvents, dispersion media, isotonic or absorption delaying agents, and the like that are physiologically compatible. For use and application of the pharmaceutical excipients, see Handbook of Pharmaceutical Excipients, 8th Ed. R. C. Rowe, P. J. Seskey and S. C. Owen, Pharmaceutical Press, London, Chicago. The composition of the present invention may be in a variety of forms. These forms include, for example, liquid, semi-solid, and solid dosage forms, such as liquid solutions (e.g., injectable solutions and infusible solutions), powders or suspensions, liposomes, and suppositories. The preferred form depends on the intended mode of administration and therapeutic use.

The medicament comprising the antibody molecule or the immunoconjugate described herein can be prepared by mixing the antibody molecule (e.g., an anti-CD28 antibody or an antigen-binding fragment thereof, or a multispecific antibody such as a bispecific antibody) or the immunoconjugate of the present invention having a desired purity with one or more optional pharmaceutical supplementary materials, e.g., in the form of a lyophilized formulation or an aqueous solution.

In one aspect, the present invention provides a method for preventing or treating a disease in a subject, comprising administering to the subject an effective amount of the molecule (e.g., an anti-CD28 antibody or an antigen-binding fragment thereof, or a multispecific antibody such as a bispecific antibody or the immunoconjugate thereof), the pharmaceutical composition, the pharmaceutical combination, or the kit of the present invention.In some embodiments, the disease is, for example, a tumor such as cancer.

In some embodiments, the present invention relates to the molecule of the present invention (e.g., an anti-CD28 antibody or an antigen-binding fragment thereof, or a multispecific antibody such as a bispecific antibody or an immunoconjugate thereof of the present invention) for use in therapy, e.g., in the treatment of a tumor such as cancer.

In some embodiments, the present invention relates to a method for treating a disease, e.g., a tumor such as a cancer, with the molecule of the present invention (e.g., an anti-CD28 antibody or an antigen-binding fragment thereof, or a multispecific antibody such as a bispecific antibody, or an immunoconjugate thereof of the present invention), or a use thereoffor the treatment, or a use thereof in the preparation of a medicament for the treatment.

In some embodiments, the tumor is a solid tumor or a hematological tumor and a metastatic lesion. In one embodiment, examples of the solid tumor include a malignant tumor. The cancer may be at an early, intermediate or advanced stage or be a metastatic cancer. In some embodiments, the tumor is tumor immune escape.

In some embodiments, the tumor is a TAA-positive tumor or cancer. In some embodiments, the tumor is a CLDN18.2-positive tumor or cancer. In some embodiments, the tumor is a PSMA-positive tumor or cancer. In some embodiments, the tumor is associated with abnormal expression or abnormal activity of TAA. In some embodiments, the tumor is associated with abnormal expression or abnormal activity of CLDN18.2. In some embodiments, the tumor is associated with abnormal expression or abnormal activity of PSMA.

In some embodiments, a TAA-positive tumor or cancer refers to such a tumor or cancer in which a subject having the tumor or cancer has TAA-expressing tumor cells. In some embodiments, tumor cells in the subject express the TAA, e.g., at a moderate or high level. In some embodiments, the subject has TAA (e.g., an elevated level of, e.g., at a nucleic acid or protein level or activity) relative to a healthy subject. In some embodiments, a biological sample (e.g., a tumor cell or a tumor tissue) of the subject has TAA, for example at an elevated level (e.g., at a nucleic acid or protein level or activity), relative to a biological sample of a healthy subject (e.g., a corresponding tissue or cell in the healthy subject), or relative to a TAA in an adjacent healthy tissue or cell of the subject. In some embodiments, the TAA is CLDN18.2 or PSMA.

In some embodiments, the tumor is a gastrointestinal tumor, e.g., a gastrointestinal cancer, such as colon cancer, rectal cancer, or colorectal cancer, e.g., a CLDN18.2-positive gastrointestinal tumor, e.g., a gastrointestinal cancer, such as gastric cancer, breast cancer, colon cancer, rectal cancer, or colorectal cancer. In some embodiments, the tumor is a PSMA-positive tumor, e.g., a renal cell carcinoma, such as breast cancer, lung cancer, rectal cancer, or colorectal cancer.

Depending on its therapeutic use, the antibody or the antigen-binding fragment thereof, or the immunoconjugate, or the pharmaceutical composition of the present invention may also be administered in combination with one or more other therapies, e.g., therapeutic modalities and/or other therapeutic agents, for the use described herein, e.g., for the prevention and/or treatment of a related disease or disorder mentioned herein.

In some embodiments, when the molecule of the present invention (e.g., an anti-CD28 antibody or an antigen-binding fragment thereof, a bispecific antibody, or an immunoconjugate thereof of the present invention) is used for treating a tumor, the therapeutic agent is, e.g., various therapeutic agents for treating the tumor, such as a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist). In some embodiments, the therapeutic agent is a PD-1 antibody or an antigen-binding fragment thereof. In some embodiments, when the molecule of the present invention (e.g., an anti-CD28 antibody or an antigen-binding fragment thereof, a bispecific antibody, or an immunoconjugate thereof of the present invention) is used for treating a tumor, the therapeutic modality includes surgery, radiotherapy, partial irradiation, focused irradiation, or the like.

In some embodiments, the present invention further provides a pharmaceutical combination or a pharmaceutical combination product comprising the antibody or the antigen-binding fragment thereof or the immunoconjugate of the present invention, and one or more additional therapeutic agents, etc.

Another object of the present invention is to provide a kit of parts comprising the pharmaceutical combination of the present invention, preferably, the kit is in the form of a pharmaceutical dose unit. Dose units may thus be provided according to the dosing regimen or the interval between drug administrations.

In one embodiment, the kit of parts of the present invention comprises in the same package:
- a first container containing a pharmaceutical composition comprising the antibody or the antigen-binding fragment thereof or the immunoconjugate disclosed herein; and
- a second container containing a pharmaceutical composition comprising an additional therapeutic agent.

In some embodiments, when the molecule of the present invention (e.g., an anti-CD28 antibody or an antigen-binding fragment thereof, a bispecific antibody, or an immunoconjugate thereof of the present invention) is used for treating a tumor, the therapeutic agent is, e.g., various therapeutic agents for treating the tumor, such as a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist). In some embodiments, the therapeutic agent is a PD-1 antibody or an antigen-binding fragment thereof.

In other aspects, the present invention provides use of the molecule of the present invention (e.g., an anti-CD28 antibody or an antigen-binding fragment thereof, a bispecific antibody, or an immunoconjugate thereof), or a composition or combination thereof comprising the same, in the manufacture or preparation of a medicament for the uses described herein, e.g., for the prevention or treatment of a related disease or disorder mentioned herein.

In other aspects, the present invention also provides the molecule of the present invention (e.g., an anti-CD28 antibody or an antigen-binding fragment thereof, a bispecific antibody, or an immunoconjugate thereof), or the composition or the medicament or the formulation or the combination product comprising the same for use in therapy, e.g., in the treatment of a related disease or disorder mentioned herein.

### IX. Diagnostics and Detection

In certain embodiments, the molecule provided herein (e.g., an anti-CD28 antibody or an antigen-binding fragment thereof, a bispecific antibody, or an immunoconjugate thereof) can be used to detect the presence of CD28 or TAA in a biological sample.

The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a bodily fluid.

In certain embodiments, the method comprises contacting the biological sample with the molecule (e.g., a bispecific antibody or an immunoconjugate thereof) described herein under conditions that allows the molecule to bind to CD28 and/or TAA, and detecting whether a complex is formed between the molecule and CD28 or TAA, wherein the formation of the complex indicates the presence of CD28 and/or TAA. The method may be an in vitro or an in vivo method. In one embodiment, the molecule of the present invention is used to select a subject suitable for treatment with the anti-CD28 antibody or the antigen-binding fragment thereof or the bispecific antibody or the immunoconjugate thereof of the present invention, e.g., wherein CD28 and/or TAA is a biomarker for selecting the subject.

In certain embodiments, provided is a labeled molecule of the present invention (e.g., an anti-CD28 antibody or an antigen-binding fragment thereof, a bispecific antibody, or an immunoconjugate thereof of the present invention). Labels include, but are not limited to, labels or moieties that are detected directly (such as fluorescent labels, chromophoric labels, electron-dense labels, chemiluminescent labels, and radioactive labels), as well as moieties that are detected indirectly, such as an enzyme or a ligand, e.g., through an enzymatic reaction or molecular interaction.

In some embodiments provided herein, the sample is obtained prior to treatment with the molecule, or the composition, the medicament, the formulation, or the combination product comprising same of the present invention. In some embodiments, the sample is obtained prior to treatment with other therapies. In some embodiments, the sample is obtained during or after treatment with other therapies.

In some embodiments, the TAA is detected prior to treatment, e.g., prior to an initial treatment or prior to a certain treatment following a treatment interval.

In some embodiments, provided is a method for treating a disease of the present invention, which comprises: detecting the presence of a TAA in a subject (e.g., a sample, such as a subject sample), thereby determining a TAA value; comparing the TAA value to a control value (e.g., a value in a normal individual); and if the TAA value is greater than the control value, administering to the subject a therapeutically effective amount of the bispecific antibody of the present invention or the composition, medicament or formulation comprising the same, optionally in combination with one or more additional therapies, thereby treating the disease.

### X. Particular Embodiment

1. An anti-CD28 antibody or an antigen-binding fragment thereof, comprising 3 CDRs of a heavy chain variable region VH: CDRH1, CDRH2 and CDRH3, and 3 CDRs of a light chain variable region VL: CDRL1, CDRL2 and CDRL3, wherein
   (i). the CDRH1, the CDRH2 and the CDRH3 are selected from three complementarity-determining regions CDRH1, CDRH2 and CDRH3 contained in a VH set forth in SEQ ID NO: 24, 23 or 75; and the CDRL1, the CDRL2 and the CDRL3 are three complementarity-determining regions CDRL1, CDRL2 and CDRL3 contained in a VL set forth in SEQ ID NO: 28 or 78;
   (ii). the CDRH1, the CDRH2 and the CDRH3 are selected from three complementarity-determining regions CDRH1, CDRH2 and CDRH3 contained in a VH set forth in any one of SEQ ID NOs: 4, 5 and 72; and the CDRL1, the CDRL2 and the CDRL3 are three complementarity-determining regions CDRL1, CDRL2 and CDRL3 contained in a VL set forth in SEQ ID NO: 9 or 73; or
   (iii). the CDRH1, the CDRH2, and the CDRH3 are selected from three complementarity-determining regions CDRH1, CDRH2, and CDRH3 contained in a VH set forth in SEQ ID NO: 13 or 69; and the CDRL1, the CDRL2, and the CDRL3 are three complementarity-determining regions CDRL1, CDRL2, and CDRL3 contained in a VL set forth in SEQ ID NO: 17, 19, or 70.
2. An anti-CD28 antibody or an antigen-binding fragment thereof, comprising a CDRH1, a CDRH2, a CDRH3, a CDRL1, a CDRL2 and a CDRL3, wherein
   (i). the CDRH1 comprises or consists of the amino acid sequence of SEQ ID NO: 20; the CDRH2 comprises or consists of the amino acid sequence of SEQ ID NO: 21; the CDRH3 comprises or consists of the amino acid sequence of SEQ ID NO: 22; the CDRL1 comprises or consists of the amino acid sequence of SEQ ID NO: 25; the CDRL2 comprises or consists of the amino acid sequence of SEQ ID NO: 26; and the CDRL3 comprises or consists of the amino acid sequence of SEQ ID NO: 27;
   (ii). the CDRH1 comprises or consists of the amino acid sequence of SEQ ID NO: 20; the CDRH2 comprises or consists of the amino acid sequence of SEQ ID NO: 76; the CDRH3 comprises or consists of the amino acid sequence of SEQ ID NO: 77; the CDRL1 comprises or consists of the amino acid sequence of SEQ ID NO: 25; the CDRL2 comprises or consists of the amino acid sequence of SEQ ID NO: 26; and the CDRL3 comprises or consists of the amino acid sequence of SEQ ID NO: 27;
   (iii). the CDRH1 comprises or consists of the amino acid sequence of SEQ ID NO: 1; the CDRH2 comprises or consists of the amino acid sequence of SEQ ID NO: 2; the CDRH3 comprises or consists of the amino acid sequence of SEQ ID NO: 3; the CDRL1 comprises or consists of the amino acid sequence of SEQ ID NO: 6 or 74; the CDRL2 comprises or consists of the amino acid sequence of SEQ ID NO: 7; and the CDRL3 comprises or consists of the amino acid sequence of SEQ ID NO: 8; or
   (iv). the CDRH1 comprises or consists of the amino acid sequence of SEQ ID NO: 10; the CDRH2 comprises or consists of the amino acid sequence of SEQ ID NO: 11; the CDRH3 comprises or consists of the amino acid sequence of SEQ ID NO: 12; the CDRL1 comprises or consists of the amino acid sequence of SEQ ID NO: 14, 18, or 71; the CDRL2 comprises or consists of the amino acid sequence of SEQ ID NO: 15; and the CDRL3 consists of the amino acid sequence of SEQ ID NO: 16.
3. The antibody or the antigen-binding fragment thereof according to embodiment 1 or 2, comprising a heavy chain variable region VH, wherein the heavy chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 24, 4, 5, 13, 23, 69, 72 or 75; or
   (ii) comprises or consists of the amino acid sequence of SEQ ID NO: 24, 4, 5, 13, 23, 69, 72 or 75.
4. The antibody or the antigen-binding fragment thereof according to any one of embodiments 1-3, comprising a light chain variable region VL, wherein the light chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 28, 9, 17, 19, 70, 73 or 78; or
   (ii) comprises or consists of the amino acid sequence of SEQ ID NO: 28, 9, 17, 19, 70, 73 or 78.
5. The antibody or the antigen-binding fragment thereof according to embodiment 1 or 2, comprising a heavy chain variable region VH and a light chain variable region VL, wherein
   (i). the VH comprises or consists of an amino acid sequence set forth in SEQ ID NO: 24 or 23 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or an amino acid sequence having at least 90%, 91%, 92%, 93%, 94%, 95%, 96%, 97%, 98%, or 99% identity thereto;
   (ii). the VH comprises or consists of an amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 78 or an amino acid sequence having at least 90%, 91%, 92%, 93%, 94%, 95%, 96%, 97%, 98%, or 99% identity thereto;
   (iii). the VH comprises or consists of an amino acid sequence set forth in SEQ ID NO: 23 or 24 or 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or 78 or an amino acid sequence having at least 90%, 91%, 92%, 93%, 94%, 95%, 96%, 97%, 98%, or 99% identity thereto;
   (iv). the VH comprises or consists of an amino acid sequence set forth in SEQ ID NO: 4 or 5 or 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or 73 or an amino acid sequence having at least 90%, 91%, 92%, 93%, 94%, 95%, 96%, 97%, 98%, or 99% identity thereto;
   (v). the VH comprises or consists of an amino acid sequence set forth in SEQ ID NO: 4 or 5 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or an amino acid sequence having at least 90%, 91%, 92%, 93%, 94%, 95%, 96%, 97%, 98%, or 99% identity thereto;
   (vi). the VH comprises or consists of an amino acid sequence set forth in SEQ ID NO: 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 73 or an amino acid sequence having at least 90%, 91%, 92%, 93%, 94%, 95%, 96%, 97%, 98%, or 99% identity thereto;
   (vii). the VH comprises or consists of an amino acid sequence set forth in SEQ ID NO: 13 or 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or 70 or an amino acid sequence having at least 90%, 91%, 92%, 93%, 94%, 95%, 96%, 97%, 98%, or 99% identity thereto;
   (viii). the VH comprises or consists of an amino acid sequence set forth in SEQ ID NO: 13 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or an amino acid sequence having at least 90%, 91%, 92%, 93%, 94%, 95%, 96%, 97%, 98%, or 99% identity thereto; or
   (ix). the VH comprises or consists of an amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 70 or an amino acid sequence having at least 90%, 91%, 92%, 93%, 94%, 95%, 96%, 97%, 98%, or 99% identity thereto.
6. The antibody or the antigen-binding fragment thereof according to embodiment 1 or 2, comprising a heavy chain variable region VH and a light chain variable region VL, wherein
   (i). the VH comprises or consists of an amino acid sequence set forth in SEQ ID NO: 24, and the VL comprises or consists of an amino acid sequence set forth in SEQ ID NO: 28;
   (ii). the VH comprises or consists of an amino acid sequence set forth in SEQ ID NO: 23, and the VL comprises or consists of an amino acid sequence set forth in SEQ ID NO: 28;
   (iii). the VH comprises or consists of an amino acid sequence set forth in SEQ ID NO: 72, and the VL comprises or consists of an amino acid sequence set forth in SEQ ID NO: 73;
   (iv). the VH comprises or consists of an amino acid sequence set forth in SEQ ID NO: 4, and the VL comprises or consists of an amino acid sequence set forth in SEQ ID NO: 9;
   (v). the VH comprises or consists of an amino acid sequence set forth in SEQ ID NO: 5, and the VL comprises or consists of an amino acid sequence set forth in SEQ ID NO: 9;
   (vi). the VH comprises or consists of an amino acid sequence set forth in SEQ ID NO: 13, and the VL comprises or consists of an amino acid sequence set forth in SEQ ID NO: 17;
   (vii). the VH comprises or consists of an amino acid sequence set forth in SEQ ID NO: 13, and the VL comprises or consists of an amino acid sequence set forth in SEQ ID NO: 19;
   (viii). the VH comprises or consists of an amino acid sequence set forth in SEQ ID NO: 69, and the VL comprises or consists of an amino acid sequence set forth in SEQ ID NO: 70; or
   (ix). the VH comprises or consists of an amino acid sequence set forth in SEQ ID NO: 75, and the VL comprises or consists of an amino acid sequence set forth in SEQ ID NO: 78.
7. An anti-CD28 antibody or an antigen-binding fragment thereof, comprising a heavy chain variable region VH and a light chain variable region VL, wherein
   (i). the VH comprises or consists of an amino acid sequence set forth in SEQ ID NO: 24, and the VL comprises or consists of an amino acid sequence set forth in SEQ ID NO: 28;
   (ii). the VH comprises or consists of an amino acid sequence set forth in SEQ ID NO: 23, and the VL comprises or consists of an amino acid sequence set forth in SEQ ID NO: 28;
   (iii). the VH comprises or consists of an amino acid sequence set forth in SEQ ID NO: 72, and the VL comprises or consists of an amino acid sequence set forth in SEQ ID NO: 73;
   (iv). the VH comprises or consists of an amino acid sequence set forth in SEQ ID NO: 4, and the VL comprises or consists of an amino acid sequence set forth in SEQ ID NO: 9;
   (v). the VH comprises or consists of an amino acid sequence set forth in SEQ ID NO: 5, and the VL comprises or consists of an amino acid sequence set forth in SEQ ID NO: 9;
   (vi). the VH comprises or consists of an amino acid sequence set forth in SEQ ID NO: 13, and the VL comprises or consists of an amino acid sequence set forth in SEQ ID NO: 17;
   (vii). the VH comprises or consists of an amino acid sequence set forth in SEQ ID NO: 13, and the VL comprises or consists of an amino acid sequence set forth in SEQ ID NO: 19;
   (viii). the VH comprises or consists of an amino acid sequence set forth in SEQ ID NO: 69, and the VL comprises or consists of an amino acid sequence set forth in SEQ ID NO: 70; or
   (ix). the VH comprises or consists of an amino acid sequence set forth in SEQ ID NO: 75, and the VL comprises or consists of an amino acid sequence set forth in SEQ ID NO: 78.
8. The antibody or the antigen-binding fragment thereof according to any one of embodiments 1-7, further comprising a heavy chain constant region HC, for example, the antibody heavy chain constant region HC is a heavy chain constant region of IgG1, IgG2, IgG3, or IgG4, preferably a heavy chain constant region of human IgG1, and preferably the heavy chain constant region
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 29 or 79; or
   (ii) comprises or consists of an amino acid sequence selected from SEQ ID NO: 29 or 79.
9. The antibody or the antigen-binding fragment thereof according to embodiment 7 or 8, wherein the heavy chain constant region comprises a mutation that reduces binding to Fcγ receptors, e.g., a LALA mutation and/or P329del.
10. The antibody or the antigen-binding fragment thereof according to any one of embodiments 1-9, comprising a light chain constant region, for example, the light chain constant region is a Lambda or Kappa light chain constant region.
11. The antibody or the antigen-binding fragment thereof according to embodiment 10, wherein the light chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 30; or
   (ii) comprises or consists of the amino acid sequence of SEQ ID NO: 30.
12. The antibody or the antigen-binding fragment thereof according to any one of embodiments 1-11, wherein the antibody is a humanized antibody or a chimeric antibody.
13. The antibody or the antigen-binding fragment thereof according to any one of embodiments 1-12, wherein the antibody is a monoclonal antibody.
14. The antibody or the antigen-binding fragment thereof according to any one of embodiments 1-13, wherein the antigen-binding fragment is an antibody fragment selected from the group consisting of a Fab, a Fab', a Fab'-SH, a Fv, a single-chain antibody (e.g., scFv), and (Fab')₂, a single-domain antibody (e.g., VHH), a domain antibody (dAb), or a linear antibody.
15. The antibody or the antigen-binding fragment thereof according to any one of embodiments 1-11, wherein the antibody is a bispecific antibody or a multispecific antibody comprising a first binding specificity for CD28 and other binding specificities for one or more tumor-associated antigens.
16. The antibody or the antigen-binding fragment thereof according to embodiment 15, wherein the tumor-associated antigen is selected from the group consisting of CLDN18.2 and PSMA.
17. A bispecific antibody, comprising a first antigen-binding region and a second antigen-binding region,
   wherein the first antigen-binding region specifically binds to CD28 and comprises a VH and a VL, wherein
   the VH comprises CDRH1, CDRH2 and CDRH3 as defined in embodiment 1 or 2, and the VL comprises CDRL1, CDRL2 and CDRL3 as defined in embodiment 1 or 2; or
   the VH and VL are the VH and VL as defined in any one of embodiments 3-6;
   the second antigen-binding region specifically binds to a tumor-associated antigen or an immune checkpoint molecule.
18. The bispecific antibody according to embodiment 17, wherein the first antigen-binding region is the Fab of the anti-CD28 antibody as defined in any one of embodiments 1-14.
19. The bispecific antibody according to embodiment 17 or 18, wherein the second antigen-binding region is a Fab specifically binding to a tumor-associated antigen or an immune checkpoint molecule.
20. The bispecific antibody according to Embodiment 18 or 19, wherein the Fab as the first antigen-binding region or the second antigen-binding region comprises a CH1, wherein the CH1 is a CH1 from IgG1, IgG2, IgG3, or IgG4, preferably a CH1 from IgG1.
21. The bispecific antibody according to embodiment 20, wherein the CH1
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 74; or
   (ii) comprises or consists of the amino acid sequence of SEQ ID NO: 74.
22. The bispecific antibody according to any one of Embodiments 18-21, wherein the Fab serving as the first antigen-binding region or the second antigen-binding region comprises a light chain constant region, wherein the light chain constant region is a Kappa light chain constant region or a Lambda light chain constant region.
23. The bispecific antibody according to embodiment 22, wherein the Kappa light chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 30; or
   (ii) comprises or consists of the amino acid sequence of SEQ ID NO: 30.
24. The bispecific antibody according to any one of Embodiments 17-23, wherein the bispecific antibody is an IgG-like bispecific antibody comprising an Fc dimer, wherein two Fc regions constituting the Fc dimer are identical or different; optionally, the Fc regions are human IgG1, IgG2, IgG3, or IgG4 Fc regions; optionally, the Fc regions
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 94 or 95; or
   (ii) comprises or consists of the amino acid sequence of SEQ ID NO: 94 or 95.
25. The bispecific antibody according to embodiment 24, wherein the two Fc regions are different, and based on the Innobody technology, mutations are introduced into the first Fc region and the second Fc region to promote heterodimerization between the first Fc region and the second Fc region.
26. The antibody or the antigen-binding fragment thereof according to embodiment 25, wherein the CH3 of one Fc region comprises S364R and D399K mutations, and the CH3 mutation of the other Fc region comprises Y349T, K370S, and K409D mutations.
27. The bispecific antibody according to any one of embodiments 24-26, wherein the first and/or second Fc region comprisesmutations that reduce binding to Fcγ receptors, e.g., mutations L234A/L235A, and optionally P329delP.
28. The bispecific antibody according to embodiment 26 or 27, wherein
   a) one Fc region comprises or consists of an amino acid sequence set forth in SEQ ID NO: 80, and the other Fc region comprises or consists of an amino acid sequence set forth in SEQ ID NO: 81;
   b) one Fc region comprises an amino acid sequence having at least 85%, 90%, 95%, 96%, 97%, 98%, or 99% identity to the amino acid sequence set forth in SEQ ID NO: 80 and comprises mutations S364R and D399K, and the other Fc region comprises an amino acid sequence having at least 85%, 90%, 95%, 96%, 97%, 98%, or 99% identity to the amino acid sequence set forth in SEQ ID NO: 81 and comprises mutations Y349T, K370S, and K409D;
   c) one Fc region comprises an amino acid sequence having at least 85%, 90%, 95%, 96%, 97%, 98%, or 99% identity to the amino acid sequence set forth in SEQ ID NO: 80, and comprises mutations S364R and D399K and mutations L234A/L235A and P329delP, and the other Fc region comprises an amino acid sequence having at least 85%, 90%, 95%, 96%, 97%, 98%, or 99% identity to the amino acid sequence set forth in SEQ ID NO: 81, and comprises mutations Y349T, K370S, and K409D and mutations L234A/L235A and P329delP;
   d) one Fc region comprises or consists of an amino acid sequence set forth in SEQ ID NO: 82, and the other Fc region comprises or consists of an amino acid sequence set forth in SEQ ID NO: 83;
   e) one Fc region comprises an amino acid sequence having at least 85%, 90%, 95%, 96%, 97%, 98%, or 99% identity to the amino acid sequence set forth in SEQ ID NO: 82 and comprises mutations S364R and D399K, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, and K409D; or
   f) one Fc region comprises an amino acid sequence having at least 85%, 90%, 95%, 96%, 97%, 98%, or 99% identity to the amino acid sequence set forth in SEQ ID NO: 82 and comprises mutations S364R, D399K, and L234A/L235A, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, K409D, and L234A/L235A.
29. The bispecific antibody according to any one of embodiments 17-28, wherein the TAA is CLDN18.2, and preferably, the second antigen-binding region comprises three complementarity-determining regions from a heavy chain variable region (CDRHs): CDRH1, CDRH2 and CDRH3 and three complementarity-determining regions from a light chain variable region (CDRLs): CDRL1, CDRL2 and CDRL3, wherein
   the CDRH1 comprises or consists of the amino acid sequence of SEQ ID NO: 87; the CDRH2 comprises or consists of the amino acid sequence of SEQ ID NO: 88, the CDRH3 comprises or consists of the amino acid sequence of SEQ ID NO: 89, the CDRL1 comprises or consists of the amino acid sequence of SEQ ID NO: 91, the CDRL2 comprises or consists of the amino acid sequence of SEQ ID NO: 92, and the CDRL3 comprises or consists of the amino acid sequence of SEQ ID NO: 93.
30. The bispecific antibody according to embodiment 29, wherein the second antigen-binding region comprises a VH and a VL, wherein the VH comprises or consists of a sequence set forth in SEQ ID NO: 86; and the VL comprises or consists of a sequence set forth in SEQ ID NO: 90.
31. The bispecific antibody according to any one of embodiments 17-28, wherein the TAA is PSMA; preferably, the second antigen-binding region comprises three complementarity-determining regions from a heavy chain variable region (CDRHs): CDRH1, CDRH2, and CDRH3 and three complementarity-determining regions from a light chain variable region (CDRLs): CDRL1, CDRL2, and CDRL3, wherein
   the CDRH1 comprises or consists of the amino acid sequence of SEQ ID NO: 96; the CDRH2 comprises or consists of the amino acid sequence of SEQ ID NO: 97, CDRH3 comprises or consists of the amino acid sequence of SEQ ID NO: 98, the CDRL1 comprises or consists of the amino acid sequence of SEQ ID NO: 99, the CDRL2 comprises or consists of the amino acid sequence of SEQ ID NO: 100, and the CDRL3 comprises or consists of the amino acid sequence of SEQ ID NO: 101.
32. The bispecific antibody according to embodiment 31, wherein the second antigen-binding region comprises a VH and a VL, wherein the VH comprises or consists of a sequence set forth in SEQ ID NO: 47; and the VL comprises or consists of a sequence set forth in SEQ ID NO: 49.
33. The bispecific antibody according to any one of embodiments 17-32, wherein the bispecific antibody comprises a first antigen-binding region specifically binding to CD28 and a second antigen-binding region specifically binding to TAA, wherein the first antigen-binding region comprises or is a first Fab, and the second antigen-binding region comprises or is a second Fab, wherein the first Fab is linked at the C-terminus of its CH1 to the N-terminus of the first Fc region (directly or via a linker such as a hinge region), and the second Fab is linked at the C-terminus of its CH1 to the N-terminus of the second Fc region (directly or via a linker such as a hinge region).
34. The bispecific antibody according to embodiment 33, wherein the bispecific antibody is an IgG-like antibody having a configuration shown in FIG. 3.
35. The bispecific antibody according to Embodiment 33 or 34, comprising a first Fab as a first antigen-binding region specifically binding to CD28 and a second Fab as a second antigen-binding region specifically binding to TAA, wherein the bispecific antibody comprises
   heavy chain 1: comprising or consisting of, from N-terminus to C-terminus, a first Fab heavy chain variable region-a heavy chain constant region CH1-a first Fc region, wherein the heavy chain constant region CH1 is linked at its C-terminus to the N-terminus of the first Fc region directly or via a linker (e.g., a hinge region);
   light chain 1: comprising or consisting of, from N-terminus to C-terminus, a first Fab light chain variable region-a light chain constant region;
   heavy chain 2: comprising or consisting of, from N-terminus to C-terminus, a second Fab heavy chain variable region-a heavy chain constant region CH1-a second Fc region, wherein the heavy chain constant region CH1 is linked at its C-terminus to the N-terminus of the second Fc region directly or via a linker (e.g., a hinge region);
   light chain 2: comprising or consisting of, from N-terminus to C-terminus: a second Fab light chain variable region-a light chain constant region;
   Preferably, each domain is directly linked.
36. The bispecific antibody according to embodiment 35, wherein the TAA is CLDN18.2, and wherein
   the heavy chain 1 comprises or consists of an amino acid sequence set forth in SEQ ID NO: 35 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto;
   the heavy chain 1 comprises or consists of an amino acid sequence set forth in SEQ ID NO: 37 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto; or
   the heavy chain 1 comprises or consists of an amino acid sequence set forth in SEQ ID NO: 39 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto.
37. The bispecific antibody according to embodiment 35, wherein the TAA is PSMA, and wherein
   the heavy chain 1 comprises or consists of an amino acid sequence set forth in SEQ ID NO: 51 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto;
   the heavy chain 1 comprises or consists of an amino acid sequence set forth in SEQ ID NO: 52 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto; or
   the heavy chain 1 comprises or consists of an amino acid sequence set forth in SEQ ID NO: 53 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto.
38. A nucleic acid molecule, encoding any one of the chains of the antibody or the antigen-binding fragment thereof according to any one of embodiments 1-16 or any one of the chains in the bispecific antibody according to any one of embodiments 17-37, or consisting of a nucleic acid sequence.
39. An expression vector, comprising the nucleic acid molecule according to embodiment 38, preferably, the expression vector is pCDNA, such as pCDNA3.1.
40. A host cell, comprising the nucleic acid molecule according to embodiment 38 or the expression vector according to embodiment 39, preferably, the host cell is prokaryotic or eukaryotic, e.g., a 293 cell or a CHO cell, such as an Expi293 cell.
41. A method for preparing the antibody or the antigen-binding fragment thereof according to any one of Embodiments 1-16 or the bispecific antibody according to any one of Embodiments 17-37, the method comprising culturing the host cell comprising the nucleic acid molecule according to embodiment 38 or the expression vector according to embodiment 39 under conditions suitable for expressing the chains of the antibody, and optionally recovering the antibody from the host cell (or a host cell medium).
42. An immunoconjugate, comprising the antibody or the antigen-binding fragment thereof according to any one of Embodiments 1 to 16 or the bispecific antibody according to any one of Embodiments 17 to 37.
43. A pharmaceutical composition or a medicament or a formulation, comprising the antibody or the antigen-binding fragment thereof according to any one of Embodiments 1-16 or the bispecific antibody according to any one of Embodiments 17-37, or the immunoconjugate according to embodiment 42, and optionally a pharmaceutical supplementary material.
44. A pharmaceutical combination product, comprising the antibody or the antigen-binding fragment thereof according to any one of Embodiments 1-16 or the bispecific antibody according to any one of Embodiments 17-37, or the immunoconjugate according to embodiment 42, and one or more additional therapeutic agents, wherein preferably, the therapeutic agents are various therapeutic agents for treating a tumor, such as a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist); preferably, the additional antibody is a PD-1 antibody.
45. A method for preventing or treating a tumor in a subject, comprising administering to the subject an effective amount of the antibody or the antigen-binding fragment thereof according to any one of Embodiments 1-16 or the bispecific antibody according to any one of Embodiments 17-37, or the immunoconjugate according to embodiment 42, or the pharmaceutical composition or formulation according to embodiment 43, or the pharmaceutical combination product according to embodiment 44.
46. The method according to embodiment 45, wherein the tumor is a solid tumor or a hematological tumor, such as a TAA-positive tumor or cancer; optionally, the tumor is a CLDN18.2-positive gastrointestinal tumor, e.g., a gastrointestinal cancer, such as gastric cancer, breast cancer, colon cancer, rectal cancer, or colorectal cancer; or the tumor is a PSMA-positive tumor, e.g., renal cell carcinoma, such as breast cancer, lung cancer, rectal cancer, or colorectal cancer.
47. The method according to any one of embodiments 45-46, further comprising administering in combination with an additional therapy such as a therapeutic modality (e.g., surgical therapy or radiotherapy) and/or an additional therapeutic agent, wherein preferably, the therapeutic agent is any therapeutic agent for treating a tumor, such as a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist); preferably, the additional antibody is a PD-1 antibody.

### Example 1: Discovery and Affinity Assay of Murine Anti-CD28 Antibodies

### Immunization with CD28 Antigen

A human CD28 extracellular domain protein (Acro, Cat CD8-H52Hc) was emulsified with Complete Freund's Adjuvant (sigma, Cat# F5881) and then used to immunize Balb/c mice (purchased from Beijing Vital River Laboratory Animal Technology Co., Ltd.). Two weeks later, the protein was emulsified with Incomplete Freund's Adjuvant (sigma, F5506) and used to immunize the mice three times by subcutaneous injection once every two weeks (50 µg of protein per mouse).

### Cell Fusion And High-Throughput Screening

When the serum titer met the requirement, the spleen of the mouse was collected to prepare a B lymphocyte suspension, which was electrofused with SP2/0 myeloma cells (ATCC). The fused cells were diluted to 1-2×E4 cells/mL with a selective medium (1640 medium containing 20% FBS and 1× HAT) and plated on a 96-well plate, and 100 µL of cell suspension was added to each well. On day 7 after the fusion, the medium was replaced with screening medium (1640 medium containing 10% FBS and 1× HT). On day 10 (or later, depending on the cell growth status) of the culture, the supernatant was collected for detection.

Hybridoma cells specifically expressing the anti-CD28 antibody were screened out by a flow cytometer (FACS). Cells (huCD28/GS-CHO, constructed in-house) to be tested were counted, diluted to 1E6 cells/mL, and added to a U-bottom 96-well plate at 100 µL/well. The mixture was centrifuged at 500 g for 5 min, and the cell medium was removed. The hybridoma culture supernatant in the 96-well plate was added to the U-shaped plate at 100 µL/well, and the cells were resuspended and left to stand on ice for 30 min. The mixture was centrifuged at 500 g for 5 min, and the supernatant was removed. The cells were washed once with PBS.The mixture was centrifuged at 500 g for 5 min, and PBS was removed. 100 µL of APC-labeled anti-mouse Fab secondary antibody (Jackson Immunoresear, Cat# 115-606-071) (diluted at 1:500 in PBS) was added to each well, and 100 µL of PE-labeled anti-human Fc secondary antibody (Biolegend, Cat# 409304) was added as the positive control antibody (TGN1412 (Creative BioLabs TAB-064LC)). The mixture was incubated in the dark on ice for 30 min and then centrifuged at 500 g for 5 min, and the supernatant was removed. The cells were washed once with PBS, resuspended in 50 µL of 1× PBS, and assayed by FACS. The supernatant of positive clones was subjected to a CD28 activation experiment. Clones with strong activating function were subcloned by a limiting dilution method, and monoclonal cells were picked.

### Subcloning of Positive Hybridoma Cell

Subcloning by limiting dilution method: A 96-well plate was prepared, and 200 µL of medium was added to each well. The medium was obtained by replacing HAT with HT (Gibco, Cat# 11067-030) on the basis of the screening medium, and the other components were the same. Cells from the positive wells selected by fusion screening were prepared into a cell suspension, and 100 µL of the cell suspension was added into each well of the first row and mixed well. Then 100 µL of the cell suspension from the first row was added into the second row, and after mixing well, 100 µL of the cell suspension was added into the next row. The above steps were repeated. The 96-well plate was left to stand for 30 min, and the cells were observed and counted under a microscope. A corresponding volume containing 100 cells was added to 20 mL of medium, and the mixture was well mixed and plated at 200 µL/well. After one week, the cells were observed under a microscope, and monoclonal wells were identified and marked.

When the cell confluence in each well reached 50% or more, the cells were detected using high-throughput screening method described above. Target positive wells were picked out, and the cells were cryopreserved after expansion culture.

### Preparation of Chimeric Antibodies

Antibody light and heavy chain gene sequences of the hybridoma candidate clones obtained in the above experiment were retrieved, and constructed into human-mouse chimeric antibodies 61F3G, 5B11D8, and 4F12B5. The corresponding sequences of the antibody light and heavy chains are as follows, and the antibody heavy chain constant region is a human IgG1 heavy chain constant region (SEQ ID NO: 79). The variable regions and CDR sequences are shown in Table 1 below.

About 5E6 cells from each freshly cultured strain were taken for RNA extraction (Macherey-Nagel, Cat# 740984.250). Reverse transcription was performed using PrimeScript II 1st Strand cDNA Synthesis Kit (Takara) to obtain cDNA. An upstream primer was designed from the base sequence located in the FR1 region at the 5' end and a downstream primer was designed from the base located in the antibody constant region or FR4 region. The gene fragments of the light chain and heavy chain variable regions of the antibody were amplified and ligated into a T vector (Mighty TA-cloning Kit, Takara), and single clones were picked for sequencing. The sequencing results were analyzed and aligned using MEGA7 software.

By alignment, the clones with correct and paired antibody light and heavy chain variable region sequences were obtained, and the gene fragments of the light and heavy chain variable regions thereof were separately ligated to a pcDNA3.1 vector (comprising an IgG1 heavy chain constant region (SEQ ID NO: 79) or a Kappa light chain constant region (SEQ ID NO: 30)) by homologous recombinases from Nanjing Vazyme Biotech Co., Ltd. (Exnase^{®}II, Cat. No. C112-01), wherein an IgG1 subtype was selected for the constant region, and expression plasmids of light and heavy chain antibodies were obtained.

**Table 1: Comparison table of CDR light and heavy chain combination of CD28 chimeric antibodies**

| | Sequence of 61F3G3 | Sequence of 5B11D8 | Sequence of 4F12B5 |
|---|---|---|---|
| VH | 69 | 72 | 75 |
| CDRH1 | 10 | 1 | 20 |
| CDRH2 | 11 | 2 | 76 |
| CDRH3 | 12 | 3 | 77 |
| VL: | 70 | 73 | 78 |
| CDRL1 | 71 | 6 | 25 |
| CDRL2 | 15 | 7 | 26 |
| CDRL3 | 16 | 8 | 27 |

Expi293F cells (purchased from Gibco) were cultured in an Expi293F medium (Gibco, REF# A14351-01). The cell density was measured the day before transfection ( viability required to be greater than 95%), and the cell density was adjusted to 3E6 cells/mL with a fresh Expi293F medium and cultured again. On the day of transfection, the cell density was adjusted to 3E6 cells/mL.

1/10 of the final transfection volume of Opti-MEM medium (Gibco, REF# 31985-070) was used as a transfection buffer, and the plasmids of the chimeric antibody to be transfected were added at a ratio of 1 mg/L, wherein the light-to-heavy chain plasmid ratio was 1:1. The mixture was mixed well, and PEIMax (Polysciences Inc. Cat# 24765-1) was then added at a DNA: PEI mass ratio of 1:3, and the mixture was mixed well and incubated at room temperature for 20 min. Then the mixture was gently poured into the Expi293F cell suspension while swirling the resulted mixture. The cells were cultured in a shaker under the culture condition of 8% CO₂, 36.5°C, and 120 rpm.

After culturing for 16-18 h, the cell suspension was supplemented with 2% (v/v) of Feed (100 g/L Phytone Peptone + 100 g/L Difco Select Phytone) at a concentration of 200 g/L, a glucose solution at a final concentration of 5 g/L, and Valproic acid sodium salt (Merk, Cat# P4543-100G) at a final concentration of 2.2 mM, gently mixed well. The cells were continuously cultured at 8 % CO₂, 36.5°C and 120 rpm for 7 days before sample collection. The cell culture broth was mixed well with diatomaceous earth (Sartorius, Cat. No. 1000037025) (1 L of the cell culture broth was mixed with 40 g of diatomaceous earth), and the mixture was filtered using a 0.22 µm disposable vacuum filtration apparatus.

Purification of target protein by affinity chromatography: a HiTrap MabSelect PrismA (GE Healthcare, Cat# 17549853) affinity chromatography column was selected for affinity capture. Before purification, 10-20 column volumes of 0.1 M NaOH were passed through the pipeline and the affinity chromatography column, and then the pipeline and the column were washed with 10-20 column volumes of distilled water. The packing column was equilibrated with 5 column volumes of 1× PBS (Gibco). The filtered cell culture broth was passed through the column, and then the packing column was washed with 10 column volumes of 1× PBS to remove non-specific binding proteins. The packing was washed with 5 column volumes of an elution buffer (100 mM sodium citrate, pH 3.5). The eluent was collected and adjusted to pH6.0 with 2 M Tris, and the eluent was filtered to remove bacteria and used for subsequent functional analysis after the purity was determined to be acceptable.

### Assay for Affinity of Chimeric Anti-CD28 Antibodies

In this study, the binding kinetic parameters (KD) of the antibody-antigen interaction of the present invention were determined by bio-layer interferometry (BLI). A BLI affinity assay was performed according to the existing method (Estep, P et al., High throughput solution Based measurement of antibody-antigen affinity and epitope binning. MAbs, 2013.5(2): 270-8).

Half an hour before the experiment, an appropriate number of AHC sensors (18-5060, Sartorius) were taken according to the number of samples and soaked in an SD buffer (1× PBS, 0.1% BSA, 0.05% Tween-20). The anti-CD28 antibodies mentioned above and the human CD28 recombinant protein (CD8-H52Hc-1mg, ACRO) were each diluted to 100 nM.

The SD buffer, the antibody solution, and the CD28 recombinant protein were separately added to 96-well black polystyrene microplates (Greiner, 655209). Detection was performed using Fortebio Octet Red96e, and the plate layout was configured based on sample positions, and sensor positions were selected accordingly. The instrument settings were as follows: The operation procedures were as follows: baseline equilibration for 120 s, sample loading for antibody immobilization for 100 s, baseline equilibration for 120 s, antigen binding for 100 s, and dissociation for 120 s, at a agitation speed of 1000 rpm and a temperature of 30°C. After the experiment was completed, KD values were analyzed using ForteBio Octet analysis software. The results are shown in the following **Table 2**.

It can be seen from the kinetic parameters of the affinity experiment that these chimeric CD28 have strong affinity for the human CD28 recombinant protein.

**Table 2: Affinity of anti-CD28 antibodies for CD28**

| Sample ID | K_{D} (M) | kₒₙ(1/Ms) | k_{dis}(1/s) |
|---|---|---|---|
| 61F3G3 | 6.39E-10 | 3.13E+05 | 2.00E-04 |
| 5B11D8 | 5.42E-10 | 4.70E+05 | 2.54E-04 |
| 4F12B5 | 1.47E-09 | 2.75E+05 | 4.04E-04 |

### Example 2: Detection of Activation of T Cell Reporter by Murine Anti-CD28 Antibodies

To verify whether the anti-CD28 antibodies can effectively recognize human CD28 and activate CD28 signals, we selected the T cell line Jurkat NFkB (Gimman Biotech, GM-C07855) reporter cells as our research object to determine the activity of the CD28 antibodies. The specific process is as follows:
1. Anti-human IgG (Jackson ImmuneResearch, 109-005-098) antibody (1 µg/mL) was diluted with PBS and added to a 96-well flat-bottom plate (Shanghai Yuhong Scientific Co., Ltd.; WHB-96-03) at 100 µL/well. The plate was incubated overnight in a refrigerator at 4°C to allow the antibodies to bind well to the bottom of the plate.
2. After the incubation was completed, the unbound antibody solution in the plate was discarded, PBS was added at 100 µL/well, and the plate was washed once and the PBS was discarded.
3. The cultured Jurkat NFkB cells were centrifuged and resuspended in Assay buffer (1% FBS (Gibco, SV30208.02), RPMI 1640 (Gibco, 22400-071)), and 50 µL of the cell suspension was added to each well to ensure 50000 cells per well.
4. The chimeric CD28 antibodies to be tested (2 µg/mL) were diluted with the assay buffer, with or without CD3 antibody(Acro, CDE-M120A-1MG, 0.5µg/mL final concentration) and added to the plate at 50 µL/well. The plate was incubated in a 5% CO₂ incubator at 37°C for 6 h.
5. After the experiment was completed, the plate was taken out and equilibrated to room temperature on the benchtop, and 100 µL of Bio-Glo detection reagent (Promega, G7940) was added. The plate was detected on a microplate reader (Molecular Devices, spectra MAX i3x).

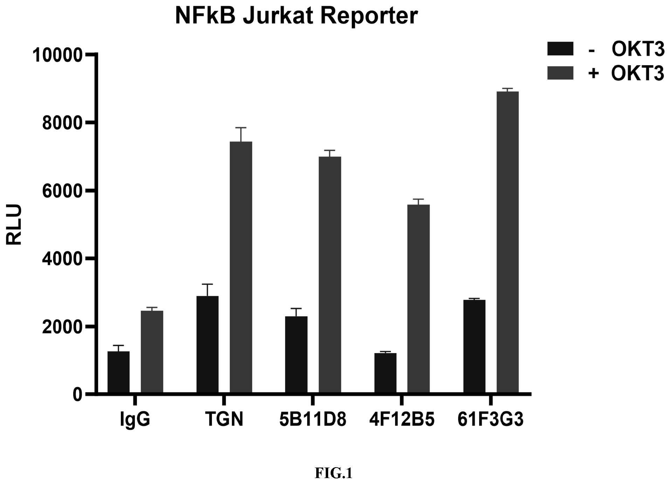

The results showed that (**FIG.1**), in the absence of the first signal provided by the CD3 (OKT3, Acro, CDE-M120A-1MG) antibody, the control antibodies TGN1412 (Tegenero, US20060188493A1, abbreviated as TGN in the present invention), 5B11D8 and 61F3G3 were able to activate the NFkB signal downstream of CD28, whereas 4F12B5 could not. In the presence of CD3 antibodies, the CD28 antibodies in this study were all able to activate CD28 signals.

### Example 3: Humanization of Murine Anti-CD28 Antibodies

The chimeric antibodies obtained in Examples 1-2 were humanized according to a conventional method. Thus, humanized antibodies hz5B11D8-a, hz5B11D8-b, hz61F3G3-a, hz61F3G3-b, hz4F12B5-a, and hz4F12B5-b were obtained. Their CDR sequences, light chain variable region and heavy chain variable region sequences, and amino acid sequences of the light chain and heavy chain are shown in the sequence listing and **Table 3**. The human IgG1 sequence (SEQ ID NO: 29) was selected for the heavy chain constant region, and the CL-Kappa constant region (SEQ ID NO: 30) was selected for the light chain constant region, corresponding to the Kappa type of the variable regions.The heavy chain sequence and the light chain sequence of the antibody were then constructed into an expression vector pcDNA3.1 (Invitrogen, V790-20), and transfected into Expi293 cells (Invitrogen, A14527) to obtain corresponding humanized antibodies.The specific transfection procedure was the same as in Example 1.

**Table 3: Comparison table of light and heavy chain combinations of humanized CD28 antibodies with CDR mutations**

| Protein ID | VH | SEQ ID NO | CDRH 1 SEQ ID NO | CDRH 2 SEQ ID NO | CDRH 3 SEQ ID NO | VL | SEQ ID NO | CDRL 1 SEQ ID NO | CDRL 2 SEQ ID NO | CDRL 3 SEQ ID NO |
|---|---|---|---|---|---|---|---|---|---|---|
| hz5B11D8 -a | hz5B11D8H. g.a | 4 | 1 | 2 | 3 | hz5B11D8K.g 0 | 9 | 6 | 7 | 8 |
| hz5B11D8 -b | hz5B11D8H. g.b | 5 | 1 | 2 | 3 | hz5B11D8K.g 0 | 9 | 6 | 7 | 8 |
| hz61F3G3-a | hz61F3G3H.g0 | 13 | 10 | 11 | 12 | hz61F3G3Kg0.h p.a | 17 | 14 | 15 | 16 |
| hz61F3G3-b | hz61F3G3H.g0 | 13 | 10 | 11 | 12 | hz61F3G3Kg0.h p.b | 19 | 18 | 15 | 16 |
| hz4F12B5-a | hz4F12B5H.g1. hp | 23 | 20 | 21 | 22 | hz4F12B5K.g0 | 28 | 25 | 26 | 27 |
| hz4F12B5-b | hz4F12B5H.g2. hp | 24 | 20 | 21 | 22 | hz4F12B5K.g0 | 28 | 25 | 26 | 27 |

### Example 4: Preparation and Affinity Assay of Humanized Anti-CD28 Antibodies

Antibody preparation: Expi293F cells (purchased from Gibco) were cultured in an Expi293F medium (Gibco, REF# A14351-01). The cell density was measured the day before transfection (viability required to be greater than 95%), and the cell density was adjusted to 3E6 cells/mL with a fresh Expi293F medium for further culture. On the day of transfection, the cell density was adjusted to 3E6 cells/mL.

1/10 of the final transfection volume of Opti-MEM medium (Gibco, REF# 31985-070) was used as a transfection buffer, and the plasmids of the humanized monoclonal antibody to be transfected (the plasmids obtained in Example 3) were added at a ratio of 1 mg/L, wherein the light and heavy chain plasmid ratio was 1:1. The mixture was mixed well, and PEIMax (Polysciences Inc. Cat# 24765-1) was added at a DNA : PEI mass ratio of 1:3, and the mixture was mixed well and incubated at room temperature for 20 min. Then the mixture was gently poured into the Expi293F cell suspension while swirling the resulted mixture. The cells were cultured in a shaker under the culture condition of 8% CO₂, 36.5°C, and 120 rpm.

After culturing for 16-18 h, the cell suspension was supplemented with 2% (v/v) of Feed (100 g/L Phytone Peptone + 100 g/L Difco Select Phytone) at a concentration of 200 g/L, a glucose solution at a final concentration of 5 g/L, and Valproic acid sodium salt (Merk, Cat# P4543-100G) at a final concentration of 2.2 mM, gently mixed well. The cells were continuously cultured at 8 % CO₂, 36.5°C and 120 rpm for 7 days before samples collection. The cell culture broth was mixed well with diatomaceous earth (Sartorius, Cat. No. 1000037025) (1 L of the cell culture broth was mixed with 40 g of diatomaceous earth), and the mixture was filtered using a 0.22 µm disposable vacuum filtration apparatus.

Purification of target protein by affinity chromatography: a HiTrap MabSelect PrismA (GE Healthcare, Cat# 17549853) affinity chromatography column was selected for affinity capture. Before purification, 10-20 column volumes of 0.1 M NaOH were passed through the pipeline and the affinity chromatography column, and then the pipeline and the column were washed with 10-20 column volumes of distilled water. The packing column was equilibrated with 5 column volumes of 1× PBS (Gibco). The filtered cell culture broth was passed through the column, and then the packing column was washed with 10 column volumes of 1× PBS to remove non-specific binding proteins. The packing was washed with 5 column volumes of an elution buffer (100 mM sodium citrate, pH 3.5). The eluent was collected and adjusted to pH6.0 with 2 M Tris, and the eluent was filtered to remove bacteria and used for subsequent functional analysis after the purity was determined to be acceptable.

The affinity of the humanized CD28 antibodies of the present invention was determined using the bio-layer interferometry (BLI) technique mentioned in Example 1. The affinities of the humanized molecules are shown in the table below (**Table 4**).

**Table 4: Affinity of humanized antibodies for CD28**

| **Sample ID** | **K_{D} (M)** | **kₒₙ(1/Ms)** | **k_{dis}(1/s)** |
|---|---|---|---|
| hz5B11D8-a | 7.46E-10 | 2.68E+05 | 2.00E-04 |
| hz5B11D8-b | 5.33E-10 | 3.75E+05 | 2.00E-04 |
| hz61F3G3-a | 8.12E-09 | 5.51E+05 | 4.48E-03 |
| hz61F3G3-b | 9.39E-09 | 6.49E+05 | 6.09E-03 |
| hz4F12B5-a | 3.06E-08 | 3.58E+05 | 1.09E-02 |
| hz4F12B5-b | 1.70E-08 | 3.22E+05 | 5.47E-03 |

### Example 5: Detection of Activation of T Cells by Humanized Anti-CD28 Antibodies

To verify whether the humanized CD28 antibodies still retained activity, we examined whether these antibodies could effectively activate human peripheral T cells in vitro. The specific procedure is as follows:
1. PBMC cells (Milestone) were taken out one day in advance, centrifuged at 25°C, 400 G for 4 min, resuspended in a growth medium (Gibco, 1640), and put into a culture flask. DNase I (SIGMA, D5025-375KU) was added at a final concentration of 2 µg/mL. The cells were incubated overnight at 37°C in a 5% CO₂ incubator.
2. The CD28 humanized antibodies to be tested were diluted with PBS (1 µg/well) (or added simultaneously with CD3 antibody(Acro, CDE-M120A-1MG, 0.1 µg/well)) and added to a 96-well flat-bottom plate, and the CD28 humanized antibodies to be tested were added at 100 µL/well. The plate was incubated overnight in a refrigerator at 4°C to allow the antibodies to bind well to the bottom of the plate.
3. On the day of the experiment, after the antibody incubation was completed, the unbound antibody solution in the plate was discarded. Each well was washed once with100 µL of PBS and the PBS was discarded then.
4. The PBMC cells thawed the day before were taken out, and the suspended cells were transferred into a centrifuge tube. After centrifugation at 25°C for 4 min at 400 G, the cell density was adjusted with Assay buffer (RPMI 1640, 1% FBS). The cell concentration was adjusted in the premise of 100 µL per well (100000 cells/well), and the cells were plated in a 96-well flat-bottom plate coated with antibodiesand incubated in a 5% CO₂ incubator at 37°C.
5. 4 h before the end of cell culture, the 96-well plate was taken out from the incubator, and 50 µL of supernatant was pipetted from each well to a new 96-well round-bottom plate for cytokine assay. The plate was cryopreserved in a refrigerator at -40°C. The protease transport inhibitor (Invitrogen, 00-4980-93) was added to a 96-well flat-bottom plate at 50 µL/well, and incubated in a 5% CO₂ incubator at 37°C for 4 h.
6. After the incubation was completed, flow cytometry antibody staining was prepared. First, a 96-well flat-bottom plate was centrifuged at 25°C and 400 G for 4 min, and the culture supernatant was discarded. The cells were resuspended in PBS, transferred to a new 96-well round-bottom plate, and centrifuged at 25°C and 400 G for 4 min, and the PBS washing buffer was discarded.
7. 50 microliters per well of pre-prepared surface-blocking dye mixture was added: Fc blocking (BD, 564219, 1:200 PBS) live/dead dye (Invitrogen, L34968A 1:100 PBS). The plate was incubated at room temperature in the dark for 10 min. After the incubation, 50 µL of a 1:100 cell surface dyes mixture: PD1 antibody (Biolegend, 329918), CD4 antibody (Biolegend, 329918), or CD8 antibody (Biolegend, 344724) in the FACS buffer, was added directly to each well, and the plate was incubated at 4°C for 30 min in the dark.
8. After the incubation with the surface dye was completed, the mixture was centrifuged at 25°C and 400 G for 4 min, and the dye that did not bind to the cells was discarded. The cells were resuspended in FACS wash buffer (2 mM EDTA, 0.5% BSA, PBS) and washed twice by centrifugation.
9. Fix/Perm buffer (ebioscience 00-5523-00 Invitrogen, 00-8333-56) was added at 100 µL/well. The plate was incubated at room temperature in the dark for 30 min to permeabilize the cell membranes.
10. After the incubation was completed, the plate was centrifuged at 25°C and 400 G for 4 min, and the Fix/Perm buffer was discarded. The cells were resuspended in a PBE (Invitrogen, 00-8333-56) wash buffer and centrifuged and washed twice by centrifugation.
11. An intracellular dye TNF-α antibody (Biolegend, 502946) was added at 50 µL/well, and the plate was incubated at room temperature for 30 min in the dark.
12. After the incubation was completed, the mixture was centrifuged at 25°C and 400 G for 4 min, and the dye that did not bind to the cells was discarded. The cells were resuspended in a PBE wash buffer and washed twice, and the wash buffer was discarded.
13. The cells were resuspended in FACS wash buffer at 150 µL/well and assayed on a flow cytometer (BD FACS SYMPHONY A3).

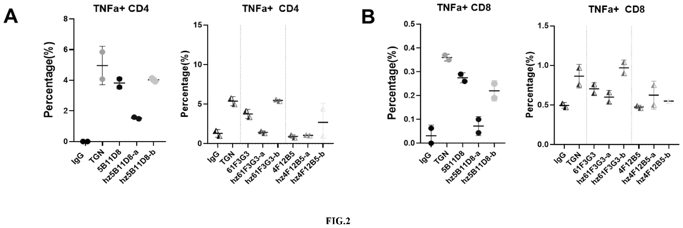

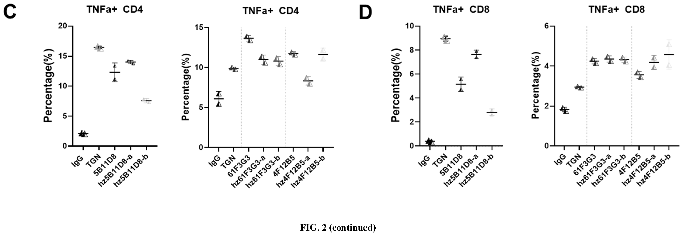

The results showed that (**FIGs.2A-B**), in the absence of a first signal provided by the CD3 antibody, the control antibody TGN1412 (abbreviated as TGN in this study) was able to activate CD4T and CD8T cells to release TNFα independent of the first signal. The chimeric antibodies 5B11D8 and 61F3G3 of the present invention exhibited CD28 superagonist activity, while 4F12B5 was a conventional CD28 agonist antibody. After humanization, hz5B11D8-a and hz61F3G3-a had reduced activity compared to the parent, while hz5B11D8-b, hz61F3G3-b, hz4F12B5-a, and hz4F12B5-b had similar activity to the parent.

In the presence of the first signal provided by the CD3 antibody (**FIGs. 2C-D**), the antibodies of the present invention can all activate CD4 and CD8 T cells to release TNF to different degrees.

### Example 6: Construction and Preparation of CLDN18.2×CD28 Bispecific Antibodies

To detect the monovalent affinity of the CD28 humanized antibodies and the activation ability for tumor-associated antigen-dependent T cells, CLDN18.2×CD28 bispecific antibodies BsAb-1, BsAb-2, BsAb-3 and BsAb-BM-1 targeting Claudin18.2 were constructed in this study (**Table 5**). The bispecific antibody molecules were in "1 + 1" form (**FIG. 3A**), wherein the variable region targeting CLDN18.2 was derived from HB37A6 (CN202010570517.X (WO2021254481A1), the sequence information is shown in the sequence listing), and the variable region sequence of the anti-CD28 antibodies were derived from the variable region sequences of the humanized antibodies hz5B11D8-a, hz61FG3-a, and hz4F12B5-b and TGN1412 (Tegenero, US20060188493A1, the sequences of which are shown in the sequence listing SEQ ID NOs: 41-44) of the present invention (**Table 6**). The Fc fragment of the antibody was in the form of Innobody (bispecific antibody technology from CN116888149A) IgG1 LALA delP, and the schematic diagram of the antibody is shown in **FIG. 3A**.

Therefore, the heavy chain of the CLDN18.2-targeting arm of the bispecific antibody was set forth in SEQ ID NO: 33, and the light chain was set forth in SEQ ID NO: 34. The heavy chains of the CD28-targeting arm of the bispecific antibodies of the present invention was set forth in SEQ ID NOs: 35, 37 and 39, respectively, and the light chains was set forth in SEQ ID NOs: 36, 38 and 40, respectively. The heavy chain and light chain of the control CD28 (TGN1412) used in the bispecific antibodies of the present invention are set forth in SEQ ID NO: 42 and SEQ ID NO: 44, respectively.

**Table 5: CLDN18.2×CD28 bispecific antibody combinations**

| **CLDN18.2×CD28 bispecific antibody** | **CLDN18.2** | **CD28** |
|---|---|---|
| BsAb-1 | HB37A6 | hz5B11D8-a |
| BsAb-2 | HB37A6 | hz61FG3-a |
| BsAb-3 | HB37A6 | hz4F12B5-b |
| BsAb-BM-1 | HB37A6 | TGN1412 |

Sequences corresponding to the heavy chain and light chain of HB37A6: SEQ ID NO: 33 and SEQ ID NO: 34.

Sequences corresponding to the heavy chain and light chain of TGN1412: SEQ ID NO: 42 and SEQ ID NO: 44.

**Table 6: Sequence information of CLDN18.2×CD28 bispecific antibodies**

| **CLDN18.2×CD28 bispecific antibody** | **CLDN18.2-targeting arm** | **CD28-targeting arm** |
|---|---|---|
| BsAb-1 | | Heavy chain variable region: SEQ ID NO: 4 |
| | | Heavy chain: SEQ ID NO: 35 |
| | | Heavy chain constant region (Inno-B): SEQ ID NO: 32 |
| | | Fc region: SEQ ID NO: 81 |
| | | Light chain variable region: SEQ ID NO: 9 |
| | | Light chain: SEQ ID NO: 36 |
| | | Light chain constant region: SEQ ID NO: 30 |
| BsAb-2 | | Heavy chain variable region: SEQ ID NO: 13 |
| | | Heavy chain: SEQ ID NO: 37 |
| | | Heavy chain constant region (Inno-B): SEQ ID NO: 32 |
| | | Fc region: SEQ ID NO: 81 |
| | Heavy chain variable region: SEQ ID NO: 86 | Light chain variable region: SEQ ID NO: 17 |
| | Heavy chain: SEQ ID NO: 33 | Light chain: SEQ ID NO: 38 |
| | Heavy chain constant region (Inno-A): SEQ ID NO: 31 | Light chain constant region: SEQ ID NO: 30 |
| BsAb-3 | Fc region: SEQ ID NO: 80 | Heavy chain variable region: SEQ ID NO: 24 |
| | Light chain variable region: 90 | |
| | Light chain: SEQ ID NO: 34 | Heavy chain: SEQ ID NO: 39 |
| | Light chain constant region: SEQ ID NO: 30 | Heavy chain constant region (Inno-B): SEQ ID NO: 32 |
| | | Fc region: SEQ ID NO: 81 |
| | | Light chain variable region: SEQ ID NO: 28 |
| | | Light chain: SEQ ID NO: 40 |
| | | Light chain constant region: SEQ ID NO: 30 |
| BsAb-BM-1 | | Heavy chain variable region: SEQ ID NO: 41 |
| | | Heavy chain: SEQ ID NO: 42 |
| | | Heavy chain constant region (Inno-B): SEQ ID NO: 32 |
| | | Fc region: SEQ ID NO: 81 |
| | | Light chain variable region: SEQ ID NO: 43 |
| | | Light chain: SEQ ID NO: 44 |
| | | Light chain constant region: SEQ ID NO: 30 |

### Preparation of Bispecific Antibodies

The bispecific antibodies of the present invention are prepared by expressing and purifying the two parent proteins separately, and then recombining the same by in vitro reduction and oxidation. The preparation route of the bispecific antibodies was as follows:
Plasmid construction: The sequences of the diabodies of the present invention were synthesized by Genewiz. The light chain and heavy chain variable regions corresponding to the bispecific antibodies (Table 6) were cloned into pcDNA3.1 vectors (Invitrogen, V790-20) containing the corresponding heavy chain or light chain constant regions, yielding the heavy and light chain plasmids for the CLDN18.2-targeting arm and heavy and light chain plasmids for the CD28-targeting arm.

Expi293F cells (purchased from Gibco) were cultured in an Expi293F medium (Gibco, REF# A14351-01). The cell density was measured the day before transfection (viability required to be greater than 95%), and the cell density was adjusted to 3E6 cells/mL with a fresh Expi293F medium and cultured again. On the day of transfection, the cell density was adjusted to 3E6 cells/mL.

1/10 of the final transfection volume of Opti-MEM medium (Gibco, REF# 31985-070) was used as a transfection buffer, and the DNA to be transfected was added at a ratio of 1 mg/L, wherein the plasmid ratio of light-to-heavy chains was 1:1. The mixture was mixed well, and PEIMax (Polysciences Inc. Cat# 24765-1) was then added at a DNA: PEI mass ratio of 1:3, and the mixture was mixed well and incubated at room temperature for 20 min. Then the mixture was gently poured into the Expi293F cell suspension while swirling the resulted mixture. The cells were cultured in a shaker under the culture condition of 8% CO₂, 36.5°C, and 120 rpm.

After culturing for 16-18 h, the cell suspension was supplemented with 2% (v/v) of Feed (100 g/L Phytone Peptone + 100 g/L Difco Select Phytone) at a concentration of 200 g/L, a glucose solution at a final concentration of 5 g/L, and Valproic acid sodium salt (Merk, Cat# P4543-100G) at a final concentration of 2.2 mM, gently mixed well.The cells were continuously cultured at 8 % CO₂, 36.5°C and 120 rpm for 7 days before sample collection.The cell culture broth was mixed well with diatomaceous earth (Sartorius, Cat. No. 1000037025) (1 L of the cell culture broth was mixed with 40 g of diatomaceous earth), and the mixture was filtered using a 0.22 µm disposable vacuum filtration apparatus.

Purification of target protein by affinity chromatography: A HiTrap MabSelect PrismA (GE Healthcare, Cat# 17549853) affinity chromatography column was selected for affinity capture. Before purification, 10-20 column volumes of 0.1 M NaOH were passed through the pipeline and the affinity chromatography column, and then the pipeline and the column were washed with 10-20 column volumes of distilled water. The packing column was equilibrated with 5 column volumes of 1× PBS (Gibco). The filtered cell culture broth was passed through the column, and then the packing column was washed with 10 column volumes of 1× PBS to remove non-specific binding proteins. The packing was washed with 5 column volumes of an elution buffer (100 mM sodium citrate, pH 3.5), and the eluent was collected and adjusted to pH6.0 with 2 M Tris. The eluent was filtered to remove bacteria and used for subsequent functional analysis after the purity was determined to be acceptable.

In vitro reduction oxidation: The protein collections from the affinity chromatography were mixed in a molar ratio of 1:1. A proper amount of GSH was added, and the reaction was adjusted to pH 8.0 with 2 M Tris. The reaction system was incubated at room temperature overnight. The reactant was buffer-exchanged into PBS and left to stand at 4°C for later use.

Purification of bispecific antibodies by ion exchange chromatography: a Mono S 5/50 GL (obtained from GE Healthcare) ion exchange chromatography column was selected and placed in an AKTApure system (GE healthcare). The AKTApure system equipped with a Mono S 5/50 GL ion exchange chromatography column was treated with 0.5 M NaOH for 2h to remove endotoxins, and then the system and the column were washed with distilled water. The column was equilibrated with 5-10 column volumes of loading buffer (20 mM NaPO4, pH6.6) until the conductivity and pH were stable. The protein obtained by affinity chromatography was diluted 10-fold with the loading buffer before loading. The column was reequilibrated with 5 column volumes of the loading buffer. Linear elution was performed with a gradient of 0-40% elution buffer (20 mM NaPO4, 1 M NaCl, pH 6.6) for a total of 30 column volumes, and samples were collected based on the UV absorption value.

Collected samples in all fraction tubes were analyzed for purity by size exclusion chromatography (SEC). According to the SEC results, the samples in the fraction tubes with a purity greater than 95% were combined.

The purified bispecific antibody solution was centrifuged in a 15 mL ultrafiltration centrifuge tube at 4500 rpm for 30 min. The protein was diluted with PBS and then centrifuged again at 4500 rpm for 30 min, and this operation was repeated several times to exchange the buffer. The antibodies after buffer exchange were combined, and the antibody concentration was measured. Qualitative and quantitative analyses of the bispecific antibody's composition and content were further conducted using a combination of capillary electrophoresis (CE-SDS) and liquid chromatography-mass spectrometry (LC-MS). The bispecific antibody obtained (as listed in **Table** 5) was used for the studies described in the following examples.

### Affinity Assay of CLDN18.2×CD28 Bispecific Antibodies

In order to characterize the affinities of the two moieties of the bispecific antibody CD28, Biolayer Interferometry (BLI) was employed in this study to determine the equilibrium dissociation constants (KD) for the binding of the bispecific antibody of the present invention to human CD28 and Claudin18.2 proteins. For the specific method, please refer to Example 1.

It was found by assay (Table 7) that the CLDN18.2 moieties of the bispecific antibodies had high affinity for Claudin18.2 protein. The CD28 moieties of the diabodies showed certain differences in affinity.

**Table 7: Detection of affinities of two moieties of CLDN18.2×CD28 bispecific antibodies**

| | Affinity of CLDN18.2-targeting arm | | | Affinity for CD28-targeting arm | | |
|---|---|---|---|---|---|---|
| Sample ID | K_{D} (M) | kₒₙ(1/Ms) | k_{dis}(1/s) | K_{D} (M) | kₒₙ(1/Ms) | k_{dis}(1/s) |
| BsAb-1 | 2.25E-10 | 8.90E+05 | 2.00E-04 | 1.71E-08 | 2.17E+05 | 3.70E-03 |
| BsAb-2 | 2.71E-10 | 7.37E+05 | 2.00E-04 | 1.19E-07 | 8.27E+05 | 9.85E-02 |
| BsAb-3 | 2.24E-10 | 8.94E+05 | 2.00E-04 | 2.67E-07 | 1.44E+05 | 3.83E-02 |

### Example 7: In Vitro Activity Assay of CLDN18.2×CD28 Bispecific Antibodies

### 7.1 Detection of NFkB Reporter Activation by CLDN18.2×CD28 Bispecific Antibodies

In the previous studies, we have verified that CD28 monoclonal antibodies can effectively activate the CD28 signaling pathway, thereby activating T cells. Here, we wanted to verify whether CD28, after being assembled into a bispecific antibody, could still have very good agonistic activity for T cells. Firstly, the activation properties of the CLDN18.2×CD28 bispecific antibodies on NFkB Jurkat Reporter cells were verified in vitro. In this research system, we need to apply Claudin18.2-expressing tumor cells to provide a "hand-grip" that enables theCLDN18.2-targeting arm of the CLDN18.2×CD28 bispecific antibody to bind effectively to the tumor cells. In addition, the CD28-targeting arm also needs to be verified in the study. For this reason, a co-culture system of a CD28-expressing T cell line Jurkat and Claudin18.2-expressing tumor cells was introduced in this study. The whole study process was as follows:

### Construction of DANG-18.2 cells:

The full-length gene of human CLDN18.2 (UniProt ID: P56856-2) was constructed into a vector pWPT-GFP (Addgene, 12255) to replace the GFP sequence therein, and the vector was transfected, together with the packaging vectors psPAX2 (Addgene, 12260) and pMD2.G (Addgene, 12259) of the lentivirus, into HEK293T (ATCC, CRL-3216) cells for virus packaging. Culture supernatants were collected after 48h and 72h of culture, and lentiviruses were concentrated using PEG8000. The pancreatic cancer DAN-G cells (DSMZ, Catalog No. ACC 249) were transfected with the concentrated virus, and then the cells expressing CLDN18.2 were sorted out by using a flow cytometric sorter (MoFlo XDP, Beckman Coulter) to obtain the tumor cell line DANG-18.2 stably transfected with CLDN18.2.

### Activation of NFkB Reporter by CLDN18.2×CD28 bispecific antibody

Treatment for target cells and effector cells: The cultured DANG-18.2 cell line was taken from the T75 simple culture flask and centrifuged at 25°C and 400 G for 4 min. The cell density was adjusted with growth medium. The cell concentration was adjusted in the premise of 100 µL per well (40000 cells/well), and the cells were mixed well and plated in a 96-well white culture plate. The 96-well white culture plate coated with the target cells was taken out, and the culture medium was discarded.

CLDN18.2×CD28 bispecific antibodies were serially diluted according to the experimental design (**Table 5**). The antibody was serially 4-fold diluted from a concentration of 100 nM to the 10th concentration gradient with the Assay buffer (1%FBS, 1640). 50 µL of the antibody to be detected (IgG, Jackson ImmuneResearch, 109-005-098) was added to each well of the plate coated with the target cells.

The cultured NFkB Jurkat cell line (Gimman Biotech, GM-C07855) was taken from the T75 simple culture flask and centrifuged at 25°C, 400 G for 4 min. The cell density was adjusted with the Assay buffer, and the cell concentration was adjusted in the premise of 50 µL per well (50000 cells/well). The mixture was mixed well, and OKT3 (at a final concentration of 0.5 µg/mL) was added at the same time. The mixture was mixed well and added to a 96-well white culture plate and incubated in a 5% CO₂ incubator at 37°C for 6 h.

The 96-well white culture plate was taken out from the incubator, and 100 µL of Bio-Glo Luciferase Reagent (Promega, G7940) solution was added to each well. After 2 min of incubation at room temperature, detection was performed using a microplate reader (Molecular Devices, spectra MAX i3x).

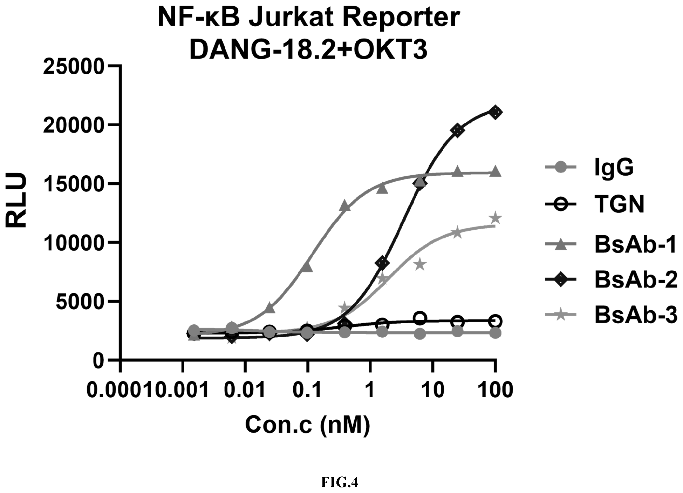

The results were as shown in **FIG.4**, the CD28 monoclonal antibody TGN was not able to effectively activate CD28 signaling. In the presence of CLDN18.2 which provides a "hand-grip", the CD28 bispecific antibodies BsAb-1, BsAb-2, and BsAb-3 were all able to effectively activate NFkB signals downstream of the CD28 signaling pathway, with stronger signals along with the increase in concentration.

### 7.2. T Cell Killing Experiment Mediated by CLDN18.2×CD28 Bispecific Antibodies In Vitro

To further verify the in vitro activity of the CD28 bispecific antibodies, we determined whether the CLDN18.2×CD28 bispecific antibodies were capable of activating human peripheral T cells to kill tumor cells. In this study, the surface of tumor cells provided a first signal by expressing an aAPC. In addition, CLDN18.2 was also transfected on the surface of tumor cells as a specific recognition target for one arm of the antibody. The whole experimental process is as follows:
CLDN18.2-aAPC-CHO K1 was constructed as follows:
The full-length gene of human CLDN18.2 (UniProt ID: P56856-2) was constructed into a vector pWPT-GFP (Addgene, 12255) to replace the GFP sequence therein, and the vector, together with the packaging vectors psPAX2 (Addgene, 12260) and pMD2.G (Addgene, 12259) of the lentivirus, were transfected into aAPC-CHO K1 (Promega, J1250) cells for virus packaging. Culture supernatants were collected after 48h and 72h of culture, and lentiviruses were concentrated using a Lenti-X Concentrator (Takara, Cat# 631232, Lot# 2109261A). Hamster ovary aAPC-CHO K1 cells (Promega, J1250) were transfected with concentrated virus, followed by FACS analysis using a flow cytometric sorter (BD FACSAria^{™} Fusion Cell Sorter) to sort out cells expressing CLDN18.2 to obtain a cell line CLDN18.2-aAPC-CHO K1 stably transfected with CLDN18.2.

The T cell killing process mediated by CLDN18.2xCD28 was as follows:
1. Target cell seeding
   The cultured CLDN18.2-aAPC-CHO K1 was taken out and digested with trypsin, and the cells were resuspended in a growth medium to stop the digestion. The cells were centrifuged, and the cell density was adjusted with Assay buffer. The cells were seeded into a 96-well round-bottom plate at 100 µL per well (10000 cells/well) and cultured for 16-20h, and Assay buffer was added to the edge wells at 200 µL/well.
2. Effector cell treatment
   A vial of cryopreserved PBMC was taken out, thawed in a water bath at 37°C, and centrifuged at 25°C and 400 G for 4 min. CD8+ T cells were isolated according to the instructions of EasySep Human CD8+ T Cell (STEMCELL, 19053). The cells were resuspended in an RPMI 1640 medium and placed in a 5% CO₂ incubator at 37°C overnight. The next day, the cells were taken out and centrifuged at 25°C and 400 G for 4 min. The cell concentration was adjusted with Assay buffer. CD8+ T cells were added to a 96-well round-bottom plate at 50 µL per well (50000 cells/well), with effector cells: target cells = 5:1.
3. Dilution of antibodies
   The antibodies to be tested were serially diluted according to the experimental design. The antibodies were serially 4-fold diluted from a starting concentration of 50 nM to the 10th concentration gradients. The diluent was the Assay buffer. 50 µL of the serially diluted antibody was pipetted into a 96-well round-bottom plate coated with CD8+ T cells and CLDN18.2-aAPC-CHO K1 cells and incubated in a 5% CO₂ incubator at 37°C for 48 h, with the total volume of each well being 200 µL.
4. 20 µL of 10× Lysis Buffer was added to the wells of the cell control group to lyse the target cells. The plate was incubated in a 5% CO₂ incubator at 37°C for 45 min. The detection reagent was prepared according to the instructions of the Radio Cytotoxicity Assay Kit (Promega, G1781). The cells in a 96-well round-bottom plate were centrifuged and pelleted. 50 µL of Mix buffer was added to a 96-well flat-bottom culture plate, and 50 µL of culture supernatant in the experimental well plate was transferred to each well. The plate was incubated at room temperature for 30 min in the dark. The stop buffer was added at 50 µL/well, and the plate were read at a wavelength of 490 nm or 492 nm within 1h on a microplate reader (Molecular Devices, spectra MAX i3x).

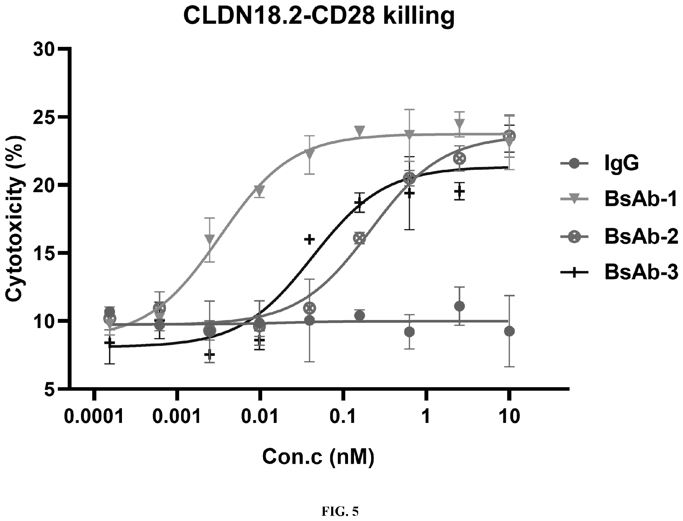

The results (**FIG. 5**) shows that CD28 bispecific antibodies to be tested were capable of effectively activating T cells and killing tumor cells in the presence of the first signal and cell surface Claudin18.2.

### 7.3 Experiment on Activation of T Cells by CLDN18.2×CD28 bispecific antibodies in vitro

To further verify the activity of the CLDN18.2×CD28 antibodies in the present invention, we examined whether these antibodies could effectively activate human peripheral T cells in vitro. The specific procedure is as follows:
1. PBMC cells were taken out one day in advance, centrifuged at 25°C, 400 G for 4 min, and resuspended in a growth medium and transferred to a culture flask, and DNase I (SIGMA, D5025-375KU) was added to a final concentration of 2 µg/mL. The cells were cultured in a 5% CO₂ incubator at 37°C overnight.
2. CLDN18.2×CD28 bispecific antibodies diluted with PBS (1 µg/well) or together with CD3 antibody (Acro, CDE-M120A-1MG, 0.1 µg/well) as required was added to a 96-well flat-bottom plate, wherein the antibody to be tested was added at 100 µL per well. The plate was incubated overnight in a refrigerator at 4°C to allow the antibodies to bind well to the bottom of the plate.
3. On the day of the experiment, after the antibody incubation was completed, the unbound antibody solution in the plate was discarded, 100 µL of PBS was added to each well, and the plate was washed once and the PBS was discarded.
4. The PBMC cells thawed the day before were taken out, and the suspended cells were transferred into a centrifuge tube. After centrifugation at 25°C for 4 min at 400 G, the cell density was adjusted with Assay buffer (RPMI 1640, 1% FBS). The cell concentration was adjusted in the premise of 100 µL per well (100000 cells/well), and the cells were plated in a 96-well flat-bottom plate coated with antibodies and incubated in a 5% CO₂ incubator at 37°C.
5. 4 h before the end of cell culture, the 96-well plate was taken out from the incubator, and 50 µL of supernatant was pipetted from each well to a new 96-well round-bottom plate for cytokine detection. The plate was cryopreserved in a refrigerator at -40°C. The protease transport inhibitor (Invitrogen, 00-4980-93) was added to a 96-well flat-bottom plate at 50 µL/well, and incubated in a 5% CO₂ incubator at 37°C for 4 h.
6. After the incubation was completed, flow cytometry antibody staining was prepared. First, a 96-well flat-bottom plate was centrifuged at 25°C and 400 G for 4 min, and the culture supernatant was discarded. The cells were resuspended in PBS, transferred to a new 96-well round-bottom plate, and centrifuged at 25°C and 400 G for 4 min, and the PBS washing buffer was discarded.
7. 50 microliters per well of pre-prepared surface-blocking dye mixture was added: Fc blocking (BD, 564219, 1:200 PBS) live/dead dye (Invitrogen, L34968A 1:100 PBS). The plate was incubated at room temperature in the dark for 10 min. After the incubation, 50 µL of a 1:100 cell surface dyes mixture: PD1 antibody (Biolegend, 329918), CD4 antibody (Biolegend, 329918), and CD8 antibody (Biolegend, 344724) in the FACS buffer, was added directly to each well, and the plate was incubated at 4°C for 30 min in the dark.
8. After the incubation with the surface dye was completed, the mixture was centrifuged at 25°C and 400 G for 4 min, and the dye that did not bind to the cells was discarded. The cells were resuspended in FACS wash buffer (2 mM EDTA, 0.5% BSA, PBS) and washed twice by centrifugation.
9. Fix/Perm buffer (ebioscience 00-5523-00 Invitrogen, 00-8333-56) was added at 100 µL/well. The plate was incubated at room temperature in the dark for 30 min to permeabilize the cell membranes.
10. After the incubation was completed, the plate was centrifuged at 25°C and 400 G for 4 min, and the Fix/Perm buffer was discarded. The cells were resuspended in a PBE (Invitrogen, 00-8333-56) wash buffer and centrifuged and washed twice by centrifugation.
11. A intracellular dye mixture: a mixed solution of IFN-γ antibody (Biolegend, 506504) and TNF-α antibody (Biolegend, 502946), was added at 50 µL/well, and the plate was incubated at room temperature for 30 min in the dark.
12. After the incubation was completed, the mixture was centrifuged at 25°C and 400 G for 4 min, and the dye that did not bind to the cells was discarded. The cells were resuspended in a PBE wash buffer and washed twice, and the wash buffer was discarded.
13. The cells were resuspended in FACS wash buffer at 150 µL/well and assayed on a flow cytometer (BD FACS SYMPHONY A3).

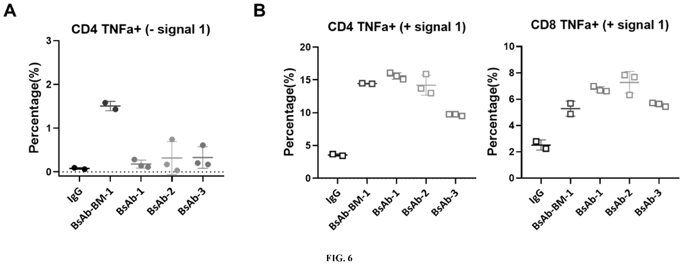

The results showed that (**FIG. 6**), in the absence of the first signal provided by the CD3 antibody, the control antibody BsAb-BM-1 (monovalent TGN) was able to activate CD4T cells to release TNFα independent of the first signal, and it was further confirmed that TGN, as a single-arm CD28 antibody, also exhibited superagonist properties (FIG. 6A). Under the same conditions, three of the CD28 bispecific antibodies of the present invention did not exhibit CD28 superagonist activity. In the presence of the first signal provided by the CD3 antibody, all the CD28 bispecific antibodies can effectively activate T (CD4 and CD8) cells (**FIG.6B**), releasing varying levels of the cytokine TNFα.

### Example 8: Construction and Preparation of PSMA×CD28 Bispecific Antibodies

To further evaluate the function of the CD28 humanized antibodies, PSMA×CD28 bispecific antibodies targeting human PSMA were constructed in this study (**Table 8**). The bispecific antibody molecules were in "1 + 1" form (**FIG. 3B**), wherein the variable region targeting PSMA is derived from 1C3 (EP1851250B1, the sequence information is shown in **Table 9**), and the variable region sequences of anti-CD28 are derived from the variable region sequences of humanized antibodies hz5B11D8-a, hz61FG3-a, and hz4F12B5-b of the present invention (**Table 9**). The Fc fragment of the antibody was in the form of Innobody (bispecific antibody technology from CN116888149A) IgG1 LALA, and the schematic diagram of the antibody is shown in **FIG. 3B**. Thus, the heavy chain of the PSMA -targeting arm 1C3 portion of the bispecific antibody was set forth in SEQ ID NO: 48, and the light chain was set forth in SEQ ID NO: 50. In the bispecific antibody of the present invention, the heavy chains of CD28-targeting arm are set forth in SEQ ID NOs: 51, 52 and 53, respectively, and light chains are set forth in SEQ ID NOs: 36, 38 and 40, respectively.

The control PSMAxCD28 antibody used in the present invention (REGN5678; US20190389951A1) has a heavy chain of SEQ ID NO: 55 and a light chain of SEQ ID NO: 57 for the PSMA-targeting arm, and a heavy chain of SEQ ID NO: 59 and a light chain of SEQ ID NO: 57 for the CD28-targeting arm.

**Table 8: PSMA×CD28 bispecific antibody combination**

| PSMA×CD28 bispecific antibody | PSMA | CD28 |
|---|---|---|
| BsAb-4 | 1C3 | hz5B11D8-a |
| BsAb-5 | 1C3 | hz61FG3-b |
| BsAb-6 | 1C3 | hz4F12B5-b |
| REGN5678 | 11838P2 | 14226P2(Regeneron, US20190389951A1) |

**Table 9: Table of PSMA×CD28 bispecific antibodies**

| PSMA×CD28 bispecific antibody | PSMA-targeting arm | CD28-targeting arm |
|---|---|---|
| BsAb-4 | Heavy chain variable region: SEQ ID NO: 47 | Heavy chain variable region: SEQ ID NO: 4 |
| | Heavy chain: SEQ ID NO: 48 | Heavy chain: SEQ ID NO: 51 |
| | Heavy chain constant region (Inno-A): SEQ ID NO: 45 | Heavy chain constant region (Inno-B): SEQ ID NO: 46 |
| | Fc region: SEQ ID NO: 82 | Fc region: SEQ ID NO: 83 |
| | Light chain variable region: SEQ ID NO: 49 | Light chain variable region: SEQ ID NO: 9 |
| | Light chain: SEQ ID NO: 50 | Light chain: SEQ ID NO: 36 |
| | Light chain constant region: SEQ ID NO: 30 | Light chain constant region: SEQ ID NO: 30 |
| BsAb-5 | | Heavy chain variable region: SEQ ID NO: 13 |
| | | Heavy chain: SEQ ID NO: 52 |
| | | Heavy chain constant region: SEQ ID NO: 46 |
| | | Fc region: SEQ ID NO: 83 |
| | | Light chain variable region: SEQ ID NO: 17 |
| | | Light chain: SEQ ID NO: 38 |
| | | Light chain constant region: SEQ ID NO: 30 |
| BsAb-6 | | Heavy chain variable region: SEQ ID NO: 24 |
| | | Heavy chain: SEQ ID NO: 53 |
| | | Heavy chain constant region: SEQ ID NO: 46 |
| | | Fc region: SEQ ID NO: 83 |
| | | Light chain variable region: SEQ ID NO: 28 |
| | | Light chain: SEQ ID NO: 40 |
| | | Light chain constant region: SEQ ID NO: 30 |
| REGN5678 | Heavy chain variable region: SEQ ID NO: 54 | Heavy chain variable region: SEQ ID NO: 58 |
| | Heavy chain: SEQ ID NO: 55 | Heavy chain: SEQ ID NO: 59 |
| | Light chain variable region: SEQ ID NO: 56 | Light chain variable region: SEQ ID NO: 56 |
| | Light chain: SEQ ID NO: 57 | Light chain: SEQ ID NO: 57 |

For all the PSMA×CD3 bispecific antibodies in this study, the sequences used for PSMA are from the patent (WO2017023761A1), and the antibody variable region sequences of the CD3-targeting arm are from (CN202180066476A; the sequences for the light chain, heavy chain, and their respective variable regions of the molecule are listed in **Table 10**.The heavy and light chain sequences corresponding to the PSMA-targeting arm are set forth in SEQ ID NO: 61 and SEQ ID NO: 63, respectively, and the heavy and light chain sequences corresponding to the CD3-targeting arm are set forth in SEQ ID NO: 65 and SEQ ID NO: 68, respectively.

**Table 10: Sequence information of PSMA×CD3 bispecific antibodies**

| | PSMA | CD3: Hzsp34.24 |
|---|---|---|
| PSMA×CD3 bispecific antibodies | Heavy chain variable region: SEQ ID NO: 60 | Heavy chain variable region: SEQ ID NO: 64 |
| | Heavy chain: SEQ ID NO: 61 | Heavy chain: SEQ ID NO: 65 |
| | Heavy chain constant region: SEQ ID NO: 45 | |
| | | Heavy chain constant region: SEQ ID NO: 46 |
| | Fc region: SEQ ID NO: 82 | |
| | Light chain variable region: SEQ ID NO: 62 | Fc region: SEQ ID NO: 83 |
| | | Light chain variable region: SEQ ID NO: 66 |
| | Light chain: SEQ ID NO: 63 | |
| | Light chain constant region: SEQ ID NO: 30 | Light chain: SEQ ID NO: 68 |
| | | Light chain constant region: SEQ ID NO: 67 |

### Preparation of PSMA×CD28 Bispecific Antibodies

The bispecific antibody BsAb-4, BsAb-5, BsAb-6 and PSMAxCD3 molecules in the present invention were prepared by expressing and purifying the two parent proteins separately, and then recombining the same through in vitro reduction and oxidation. The control bispecific antibody REGN5678 was prepared by intracellular assembly.

Plasmid construction: The sequences of the diabodies of the present invention were synthesized by Genewiz. The light chain and heavy chain variable regions corresponding to the bispecific antibodies (**Table 9-10**) were cloned into pcDNA3.1 vectors (Invitrogen, V790-20) containing the corresponding heavy chain or light chain constant regions, yielding heavy and light chain plasmids for PSMA-targeting arm, heavy and light chain plasmids for CD28-targeting arm, and heavy and light chain plasmids for CD3-targeting arm, respectively.

The preparation routes for the four bispecific antibody molecules BsAb-4, BsAb-5, BsAb-6 and PSMA×CD3 can be found in Example 7. The expression and purification methods for the control bispecific antibody REGN5678 were based on the protocol described by Andrew D. Tustian et al., (2016) ("Development of purification processes for fully human bispecific antibodies based upon upon modification of protein A binding affinity", mAbs, 8:4, 828-838).

The PSMAxCD28 and PSMAxCD3 bispecific antibody molecules listed in **Tables 8-10** were prepared and obtained for use in the study described in the following examples.

### Example 9: In Vitro Activity Assay of PSMA×CD28 Bispecific Antibodies

### 9.1 Cell surface affinity assay of PSMA×CD28 bispecific antibodies

To evaluate the activity of our CD28 bispecific antibodies, we first detected the affinity of several antibodies for the cell surface in vitro.

Firstly, we constructed tumor cells MC38_hPSMA expressing human PSMA, and the specific construction process was as follows:
The full-length gene of human PSMA (UniProt ID: Q04609-1) was constructed into a vector pWPT-GFP (Addgene, 12255) to replace the GFP sequence therein, and the vector was transfected, together with the packaging vectors psPAX2 (Addgene, 12260) and pMD2.G (Addgene, 12259) of the lentivirus, into HEK293T (ATCC, CRL-3216) cells for virus packaging. Culture supernatants after 48h and 72h of culture were collected, and lentiviruses were concentrated using a Lenti-X Concentrator (Takara, Cat# 631232, Lot# 2109261A). The concentrated virus was transfected into colon cancer MC38 cells (Nanjing Cobioer Biosciences), and then the cells expressing PSMA were sorted out using a flow cytometry sorter (BD FACSAria^{™} Fusion Cell Sorter) to obtain the tumor cell line MC38_hPSMA stably transfected with PSMA.

Next, we constructed tumor cells 024-hCD28 CHO GS expressing human CD28 as follows:
The full-length gene of human CD28 (UniProt ID: P10747) was constructed into a vector pXC17.4 (Lonza), and the resulting plasmid was digested with PvuI (NEB, R3150L) into a linear fragment. The linear plasmids were transferred into CHO-GS cells (Lonza) by electroporation using an electroporator (Bio Rad, GENEPULSER XCELL, 300 V, 900 µF, Resistance infinity (∞), 2 mm). following transfection, the cells were transferred to a CD CHO culture medium (Gibco, 10743-029) at 37°C, added with 1% SP4 (Lonza, BESP1076E), and cultured in a shaker at 37°C with 6% CO₂, 120 rpm. At 16-30h post-electroporation, the cells were centrifuged at 1000 rpm for 5 min, and the supernatant was discarded. The cells were resuspended in a fresh medium supplemented with SP4, and MSX (SIGMA, M5379-250MG) was added to a final concentration of 50 µM, the cell density was adjusted to 5E5 cells/mL, and the culture was continued. Starting on day 7, the cell density and viability were measured every 1-2 days. Once the cell viability exceeded 90%, cells expressing human CD28 were sorted out using a flow cytometric sorter (BD FACSAria^{™} Fusion Cell Sorter) to obtain the tumor cell line 024-hCD28 CHO GS stably transfected with CD28.

### Affinity assay

The cultured 024-hCD28 CHO GS and MC38_hPSMA cell lines were taken from the T75 simple culture flask, centrifuged at 25°C and 400 G for 4 min. The cell density was adjusted using a washing buffer (2 mM EDTA, 0.5% BSA, PBS), and the cell concentration was adjusted in the premise of 50 µL per well (100,000 cells/well). The cells were mixed well and plated in a 96-well round-bottom culture plate. The antibodies to be tested were serially diluted according to the experimental design. The antibodies were serially 4-fold diluted from an initial concentration of 100 nM to the 10th concentration gradient with FACS wash buffer. 50 µL of the antibody to be tested was added to each well of a plate coated with cells. The plate was incubated in a refrigerator at 4°C for 30-60 min to allow the antibodies to bind well to the cell surfaces. After the incubation was completed, the plate was centrifuged at 25°C, 400 G for 4 min, and the unbound antibodies in the plate were discarded. 100 µL of FACS wash buffer was added to each well to resuspend the cells. The mixture was centrifuged at 25°C and 400 G for 4 min, and the washing solution was discarded. The diluted PE-hIgG-heavy chain (Biolegend, 410708) was added to a well plate at 100 µL/well to bind to the heavy chain of the test antibody. The plate was incubated at room temperature in the dark for 20-30 min. After the incubation was completed, the plate was centrifuged at 25°C and 400 G for 4 min, and the unbound antibodies in the plate were discarded. 100 µL of FACS wash buffer was added to each well to resuspend the cells. The mixture was centrifuged at 25°C and 400 G for 4 min, and the washing solution was discarded. Finally, the cells were resuspended in 100 µL of FACS wash buffer per well and prepared for analysis on the flow cytometer (BD FACS SYMPHONY A3).

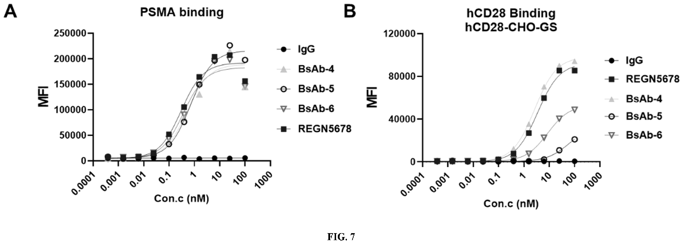

The results showed that (**FIG. 7**), the bispecific antibodies all exhibited very strong affinity for the PSMA target, and overall affinity of BsAb-4, BsAb-5, and BsAb-6 for PSMA on the cell surface was comparable to that of REGN5678 (**FIG. 7A**). In terms of the affinity for the CD28 target, BsAb-4, BsAb-5 and BsAb-6 all demonstrated binding (**FIG. 7B**).

### 9.2 Detection of Activation of NFkB Reporter by PSMA×CD28 Bispecific Antibodies In Vitro

Next, we further verified whether CD28, when assembled into a bispecific antibody, still retains potent agonistic activity on T cells. Firstly, the activation of NFkB Jurkat Reporter cells by PSMA×CD28 bispecific antibodies were verified in vitro. In this research system, tumor cells expressing human PSMA were used to provide a "hand-grip" to allow the PSMA-targeting arm of the bispecific antibody to efficiently bind to the tumor cells. In addition, the CD28-targeting arm also needs to be verified in the study. For this reason, a CD28-expressing T cell line Jurkat was introduced in this study. The whole study process was as follows:

### Treatment for target and effector cells

The cultured MC38_hPSMA cell line was taken from the T75 simple culture flask and centrifuged at 25°C and 400 G for 4 min. The cell density was adjusted with a growth medium, and the cell concentration was adjusted in the premise of 100 µL/well (40000 cells/well). The cells were mixed well and plated in a 96-well white culture plate (WHB). The 96-well white culture plate (WHB) coated with the target cells was taken out, and the culture medium was discarded.

The PSMA×CD28 bispecific antibodies were serially diluted according to the experimental design. The antibodies were serially 4-fold diluted from an initial concentration of 100 nM to the 10th concentration gradientwith Assay buffer (1%FBS, 1640). The antibodies to be tested were added at 50 µL/well to a plate coated with the target cells.

The cultured NFkB Jurkat cell line was taken out from the T75 simple culture flask and centrifuged at 25°C and 400 G for 4 min. The cell density was adjusted with the Assay buffer, and the cell concentration was adjusted in the premise of 50 µL per well (50000 cells/well). The mixture was mixed well, and the CD3 antibody (Acro, CDE-M120A-1MG, final concentration: 0.5 µg/mL) or PSMAxCD3 bispecific antibody (final concentration: 10 pM) was added at the same time. The mixture was mixed well and added to a 96-well white culture plate (WHB) and incubated in a 5% CO₂ incubator at 37°C for 6 h.

The 96-well white culture plate (WHB) was taken out of the incubator, and 100 µL of Bio-Glo Luciferase Reagent (Promega, G7940) solution was added to each well. After 2 min of incubation at room temperature, detection was performed using a microplate reader (Molecular Devices, spectra MAX i3x).

In this study, we employed two first signals to assess the effects of the CD28 bispecific antibodies on the activation of CD28 signals.

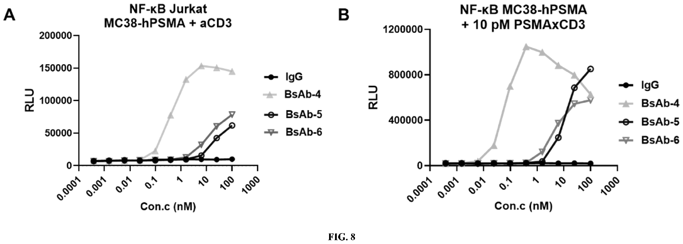

First, we employed an anti-CD3 antibody as the first signal. The CD28 bispecific antibodies as shown in **FIG. 8A** were all able to activate the NF-κB signal downstream of CD28 on Jurkat cells.

Secondly, we selected low concentrations of PSMA×CD3 bispecific antibodies as the first signal to evaluate the activation of T cells by CD28 bispecific antibodies. As shown in **FIG. 8B**, the tested PSMA×CD28 bispecific antibodies were all able to activate NFkB reporter and the trend was similar to that observed in **FIG. 8A**.

### 9.3. In Vitro T-Cell Killing Assay Mediated by PSMA×CD28 Bispecific Antibody

Next, we further tested whether the PSMA×CD28 bispecific antibodies could activate human peripheral T cells to kill tumor cells. In this study, an aAPC was integrated on the surface of the tumor cells to provide the first signal. In addition, we also transfected human PSMA on the surface of tumor cells as a specific recognition target for one targeting arm of the antibody. The whole experimental process is as follows:
PSMA-aAPC-CHO K1 was constructed as follows:
The full-length gene of human PSMA (UniProt ID: Q04609-1) was constructed into a vector pWPT-GFP (Addgene, 12255) to replace the GFP sequence therein, and the vector was transfected, together with the packaging vectors psPAX2 (Addgene, 12260) and pMD2.G (Addgene, 12259) of the lentivirus, into aAPC-CHO K1 (Promega, J1250) cells for virus packaging. Culture supernatants were collected after 48h and 72h of culture, and lentiviruses were concentrated using a Lenti-X Concentrator (Takara, Cat# 631232, Lot# 2109261A). Hamster ovarian aAPC-CHO K1 cells were transfected with the concentrated virus, and then PSMA-expressing cells were sorted out by using a flow cytometric sorter (MoFlo XDP, Beckman Coulter) to obtain the cell line PSMA-aAPC-CHO K1 stably transfected with PSMA.

PSMA×CD28 mediates the killing of tumor cells by T cells, and the specific process is as follows:
1. Target cell seeding
   The cultured PSMA-aAPC-CHO K1 was taken out and digested with trypsin, and the cells were resuspended in a growth medium to stop the digestion. The cells were centrifuged, and the cell density was adjusted with Assay buffer. The cells were seeded into a 96-well round-bottom plate at 100 µL per well (10000 cells/well) and cultured for 16-20 h, and Assay buffer was added to the edge wells at 200 µL/well.
2. Effector cell treatment
   A vial of cryopreserved PBMC was taken out, thawed in a water bath at 37°C, and centrifuged at 25°C and 400 G for 4 min. CD8+ T cells were isolated according to the instructions of EasySep Human CD8+ T Cell (STEMCELL, 19053). The cells were resuspended in an RPMI 1640 medium and placed in a 5% CO₂ incubator at 37°C overnight. The next day, the cellswere taken out and centrifuged at 25°C and 400 G for 4 min. The cell concentration was adjusted with Assay buffer. CD8+ T cells were added to a 96-well round-bottom plate at 50 µL per well (50000 cells/well), with effector cells: target cells = 5:1.
3. Dilution of antibodies
   The antibody to be tested was serially diluted according to the experimental design. The antibody was serially 4-fold diluted from an initial concentration of 50 nM to to the 10th concentration gradient. The diluent was the Assay buffer. 50 µL of the serially diluted antibody was pipetted into a 96-well round-bottom plate coated with CD8+ T cells and PSMA-aAPC-CHO K1 cells and incubated in a 5% CO₂ incubator at 37°C for 48 h, with the total volume of each well being 200 µL.
4. 20 µL of 10× Lysis Buffer was added to the wells of the cell control group to lyse the target cells. The plate was incubated in a 5% CO₂ incubator at 37°C for 45 min. The detection reagent was prepared according to the instructions of the Radio Cytotoxicity Assay Kit (Promega, G1781). The cells in a 96-well round-bottom plate were centrifuged and pelleted. 50 µL of Mix buffer was added to a 96-well flat-bottom culture plate, and 50 µL of culture supernatant in the experimental well plate was transferred to each well. The plate was incubated at room temperature for 30 min in the dark. The stop buffer was added at 50 µL/well, and the plate were read at a wavelength of 490 nm or 492 nm within 1h on a microplate reader (Molecular Devices, spectra MAX i3x).

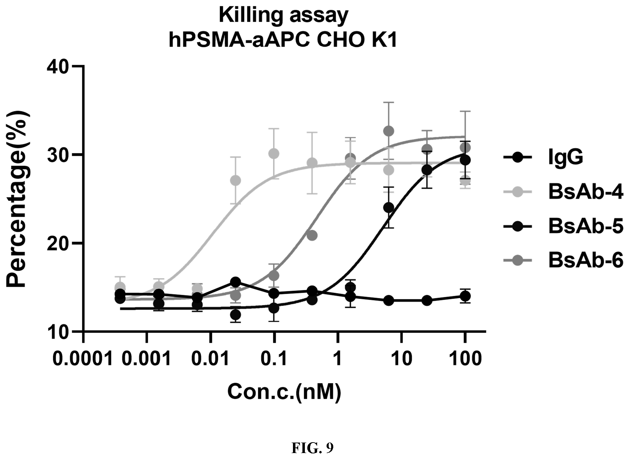

The results (**FIG. 9**) shows that the three PSMA×CD28 bispecific antibodies were all able to activate T cells and produce killing effects on PSMA-expressing tumor cells. In terms of the degree of the target cells death, BsAb-4 with strong affinity for CD28 has the strongest CD28 activation activity and is capable of mediating T cells to kill tumor cells at low concentrations. BsAb-5 has the weakest killing activity as the CD28-targeting arm thereof has the lowest affinity. The overall killing level mediated by the BsAb-6 bispecific antibodies was also weakened due to the decrease in the affinity for CD28.

### Example 10: T-Cell Activation Assay for PSMA×CD28 Bispecific Antibody

To further evaluate the activity of PSMAxCD28 antibodies, we tested whether these antibodies can effectively activate human peripheral T cells in vitro, releasing cytokines IL2. The specific process is as follows:
1. PBMC cells were taken out one day in advance, centrifuged at 25°C, 400 G for 4 min, and resuspended in a growth medium, and put into a culture flask. DNase I (SIGMA, D5025-375KU) was added at a final concentration of 2 µg/mL. The cells were incubated overnight at 37°C in a 5% CO₂ incubator.
2. The PSMA×CD28 antibody was diluted with PBS (1 µg/well) (or added with CD3 antibody (Acro, CDE-M120A-1MG, 0.1 µg/well) as required) and added to a 96-well flat-bottom plate, and the antibodies to be tested were added at 100 µL/well. The plate was incubated overnight in a refrigerator at 4°C to allow the antibodies to bind well to the bottom of the plate.
3. On the day of the experiment, after the antibody incubation was completed, the unbound antibody solution in the plate was discarded. Each well was washed once with100 µL of PBS and the PBS was discarded then.
4. The PBMC cells thawed the day before were taken out, and the suspended cells were transferred into a centrifuge tube. After centrifugation at 25°C for 4 min at 400 G, the cell density was adjusted with Assay buffer (RPMI 1640, 1% FBS). The cell concentration was adjusted in the premise of 100 µL/well (100000 cells/well), and the cells were plated in a 96-well flat-bottom plate coated with antibodies and incubated in a 5% CO₂ incubator at 37°C.
5. 4 h before the end of cell culture, the 96-well plate was taken out from the incubator, and 50 µL of supernatant was pipetted from each well to a new 96-well round-bottom plate for cytokine detection.
6. According to the instructions of the IL2 Human ELISA KIT (Mabtech, 3445-1H-20), the Human IL-2 antibody was diluted with PBS and added to a 96-well flat-bottom plate at 50 µL/well, and the plate was covered with a sealing film and incubated overnight in a refrigerator at 4°C.
7. The unbound purified antibody in the 96-well flat-bottom plate was removed, and PBST buffer (0.05% Tween 20) was added at 100 µL/well for washing for 6 times. Then Assay buffer (1000 mL of PBS, 500 µL of Tween 20, and 5 g of BSA) was added at 100 µL/well, and the mixture was incubated at room temperature for 1h.
8. After the incubation, the 96-well flat-bottom plate was taken out, the liquid in the plate was discarded, 50 µL of the prepared sample supernatant and standards were added, and the plate was incubated at room temperature for 2h.
9. The 96-well flat-bottom plate was taken out, and the liquid in the plate was discarded followed by 6 times washing for the plate. 50 µL of diluted biotin antibody (Mabtech, 3445-1H-20) was added to each well, and the plate was incubated on a plate shaker at room temperature for 1h.
10. The 96-well flat-bottom plate was taken out, and the liquid in the plate was discarded followed by 6 times washing for the plate. 50 µL of diluted HRP was added to each well, and the plate was incubated at room temperature for 1h.
11. The 96-well flat-bottom plate was taken out, and the liquid in the plate was discarded followed by 6 times washing for the plate. 50 µL of TMB was added to each well, and after the color development reaction was completed, 50 µL of stop solution was added to each well. The values were read at a wavelength of 450 nm in a microplate reader (Molecular Devices, spectra MAX i3x) within 1h.

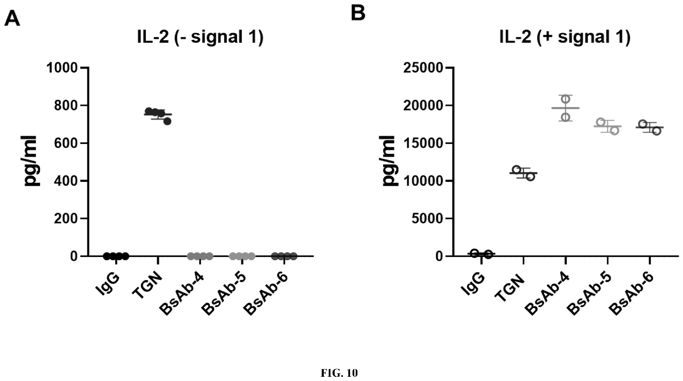

The results showed that in the absence of the first signal CD3 antibody (-signal 1) (**FIG. 10A**), the control antibody TGN1412 (abbreviated as TGN in this study) can activate T cells to release IL2 independent of the first signal, confirming that TGN is a CD28 superagonist antibody. Under the same conditions, the three CD28 bispecific antibodies BsAb-4, BsAb-5 and BsAb-6 in the present invention were unable to activate T cells, which was manifested as conventional CD28 agonist antibodies. In the presence of the first signal (+ signal 1) provided by the CD3 antibody, all CD28 antibodies were able to effectively activate T cells to release IL2 (**FIG. 10B**).

### Example 11: In Vivo Anti-Tumor Activity Assay of PSMA×CD28 Bispecific Antibodies

To further evaluate the activity of the PSMA×CD28 bispecific antibodies, we tested the anti-tumor activity of the PSMA×CD28 bispecific antibodies in vivo in an mouse tumor model.

Mice: hCD28 humanized mice (Shanghai Model Organisms Center, Inc.) aged 6-8 weeks were placed in an SPF environment for 3-5 days before the experiment to better adapt to the current living environment.

In all experiments, the long and short diameters of the tumor were measured twice a week using a vernier caliper, and the tumor volume (mm3) was calculated from the measurement data. The calculation formula is as follows:$Tumor volume \left(mm3\right) = 0.5 \times long diameter \left(mm\right) \times short diameter \left(mm\right) \times short diameter \left(mm\right)$

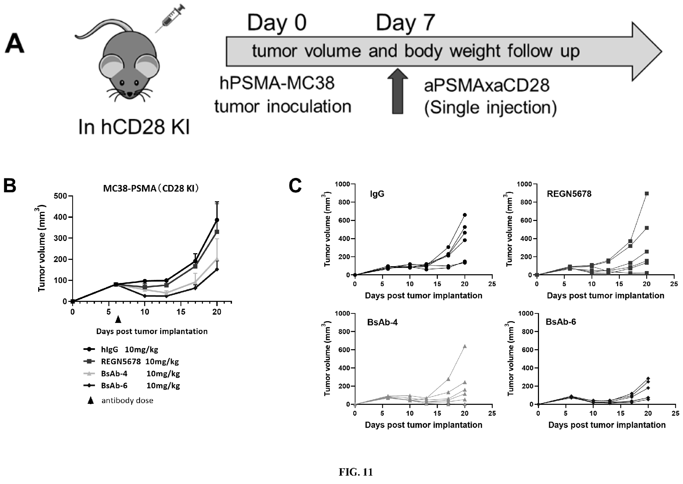

hPSMA-MC38 cells expressing human PSMA were resuspended in 1× PBS (Gibco, 10010049) at an appropriate concentration, and tumor cells were subcutaneously grafted in the right abdomen of the mice at an inoculation amount of 1E6/mouse (**FIG.11A**). About 7 days after inoculation, when the tumor volume reached 80-100 mm3, the mice were grouped, to allow the average initial administration volume of each group was basically the same. After grouping, 10 mg/kg of PSMAxCD28 bispecific antibody was injected into the mice intraperitoneally, and the effect of the drug on tumor growth was observed.

The results show that in the MC38 tumor model overexpressing human PSMA, PSMAxCD28 at a single dose of 10 mg/kg was able to inhibit the growth of tumor cells to some extent (**FIG. 11B**). REGN5678 has a relatively weak tumor inhibitory effect, with TGI of only 42.5%. BsAb-4 and BsAb-6 (**FIG. 11C****, each line represents a mouse**) can produce a stronger tumor inhibitory effect than REGN5678 under the same conditions.

### Example 12: In-Vivo Anti-Tumor Activity Assay of PSMA×CD28 Bispecific Antibodies in Combination with PD1 Antibody

To further evaluate the activity of the PSMA×CD28 bispecific antibodies, the anti-tumor activity of the CD28 bispecific antibodies in combination with a PD1 antibody in vivo in an mouse tumor model was detected.

The anti-mouse PD1 antibody was derived from a patent (WO2018184964A1), and its sequence is shown below. The preparation process was the same as in Example 1.

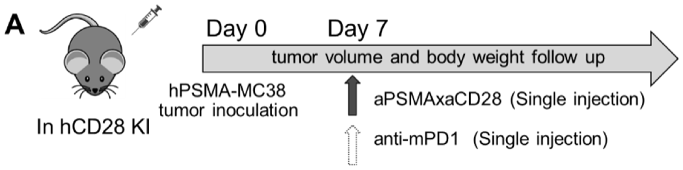

The whole operation was performed as in Example 12. hPSMA-MC38 cells expressing human PSMA were resuspended in 1× PBS (Gibco, 10010049) at an appropriate concentration, and tumor cells were subcutaneously grafted at the right abdomen of the mice in an inoculation amount of 1E6/mouse (**FIG. 12A**). About 7 days after inoculation, when the tumor volume reached 80-100 mm3, the mice were grouped, to allow the average initial administration volume of each group was basically the same. After grouping, the BsAb-6 bispecific antibody or the anti-PD1 antibody or the combination thereof was administered to the mice by intraperitoneal injection, and the effect of the drug on tumor growth was observed.

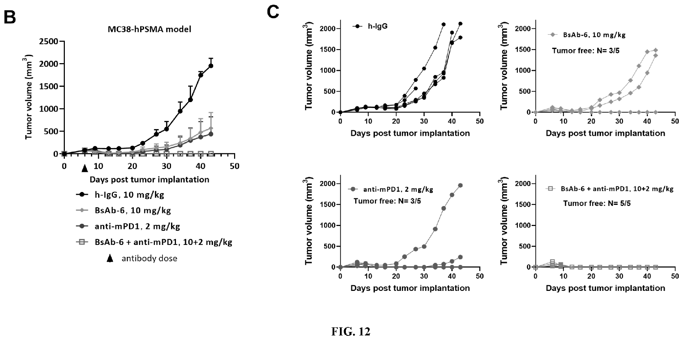

The results show that in the MC38 tumor model overexpressing human PSMA, the combined administration of 10 mg/kg BsAb-6 and 2 mg/kg of murine PD1 antibody produced a strong synergistic effect, significantly inhibiting the growth of tumor cells (**FIG. 12B**). After a single administration, the proportion of complete tumor regression reached 100% during the observation process lasting for more than 40 days, which was higher than the proportion of complete tumor regression in the monotherapy group (60%) (**FIG. 12C****, each line represents a mouse**).

### Example 13: Mouse PK Experiment of PSMA×CD28 Bispecific Antibodies

In this study, 10 mg/kg REGN5678 and BsAb-6 were injected into female C57BL/6 hCD28KI mice (Shanghai Model Organisms Center, Inc.) via the tail vein for administration, with 6 mice in each group, 12 mice in total, to study their pharmacokinetic properties in the mice.

Blood was collected from the eyeballs of the mice at time points of 0.086 h, 0.5 h, 2 h, 6 h, 24 h, 48 h, 4 days, 7 days, 14 days, and 21 days after administration and centrifuged at 3000 rpm at 4°C for 10 min, and serum was collected. The antibody content in the serum was determined by ELISA, and the half-lives of REGN5678 and BsAb-6 in the mice were calculated.

The experimental results were as shown in **Table 11**, BsAb-6 has good stability, long half-life (t1/2) and relatively good blood exposure in hCD28 transgenic mice. It can be seen from the half-life that BsAb-6 achieved stability data similar to that of the normal monoclonal antibody in both detection methods.

**Table 11: In Vivo PK Assay of PSMA×CD28 Bispecific Antibodies in Mice**

| Samples | t1/2 (h) | Cmax (ug/mL) | AUC 0-t (ug*h/mL) | AUC 0-inf_obs (ug*h/mL) | Cl_obs (ml/kg/h) | Vss_obs (ml/kg) |
|---|---|---|---|---|---|---|
| REGN5678 Anti-Fc arm) | 87 | 189 | 11675 | 11813 | 0.85 | 80 |
| REGN5678 Anti-CD28 arm) | 57 | 262 | 10431 | 10569 | 0.95 | 67 |
| BsAb-6 (anti-Fc arm) | 151 | 210 | 13818 | 15100 | 0.66 | 136 |
| BsAb-6 (anti-CD28 arm) | 160 | 238 | 11693 | 12931 | 0.77 | 164 |

### Sequence Listing

| SEQ ID NO | Name of variable region sequence | Specific sequence |
|---|---|---|
| | hz5B11D8H.g.a | |
| 1 | CDRH1 | GYTFTFYWMH |
| 2 | CDRH2 | EIDPSDSYTNYNQEFKD |
| 3 | CDRH3 | DPWYFDV |
| 4 | VH | QVQLVQSGAEVKKPGASVKVSCKASGYTFTFYWMHWVRQAPGQGLEWMGE IDPSDSYTNYNQEFKDRVTMTVDTSTSTVYMELSSLRSEDTAVYYCARDPWYF DVWGQGTLVTVSS |
| 29 | HC(heavy chain constant region)(IgG1 , WT-DEL IgG1) | |
| | hz5B11D8H.g.b | |
| 1 | CDRH1 | SEQ ID NO:1 |
| 2 | CDRH2 | SEQ ID NO:2 |
| 3 | CDRH3 | SEQ ID NO:3 |
| 5 | VH | QVQLVQSGAEVKKPGASVKVSCKASGYTFTFYWMHWVRQAPGQGLEWMGE IDPSDSYTNYNQEFKDRVTMTVDTSTSTAYMELSSLRSEDTAVYYCARDPWYF DVWGQGTLVTVSS |
| 29 | HC(heavy chain constant region) | SEQ ID NO:29 |
| | hz5B11D8K.g0 | |
| 6 | CDRL1 | SASSSITYMY |
| 7 | CDRL2 | DTSKLAS |
| 8 | CDRL3 | HQRSSYPWT |
| 9 | VL | DIQLTQSPSFLSASVGDRVTITCSASSSITYMYWYQQKPGKAPKLLIYDTSKLAS GVPSRFSGSGSGTEFTLTISSLQPEDFATYYCHQRSSYPWTFGQGTRLEIK |
| 30 | LC(Light chain constant region) | |
| | hz61F3G3H.g0 | |
| 10 | CDRH1 | GYTFTSYWMH |
| 11 | CDRH2 | NIYPGSGSTNYDEKFKS |
| 12 | CDRH3 | EHYGSIYEAMDY |
| 13 | VH | QVQLVQSGAEVKKPGASVKVSCKASGYTFTSYWMHWVRQAPGQGLEWMG NIYPGSGSTNYDEKFKSRVTMTRDTSTSTVYMELSSLRSEDTAVYYCAREHYG SIYEAMDYWGQGTLVTVSS |
| 29 | HC(heavy chain constant region) | SEQ ID NO:29 |
| | hz61F3G3Kg0.h p.a | |
| 14 | CDRL1 | RASQEIQGYLS |
| 15 | CDRL2 | AASTLDS |
| 16 | CDRL3 | LQYVSYPYT |
| 17 | VL | DIQMTQSPSSLSASVGDRVTITCRASQEIQGYLSWFQQKPGKAPKSLIYAASTL DSGVPSRFSGSGSGTDFTLTISSLQPEDFATYYCLQYVSYPYTFGQGTKLEIK |
| 30 | LC(Light chain constant region) | SEQ ID NO:30 |
| | hz61F3G3Kg0.h p.b | |
| 18 | CDRL1 | RASQEINVYLS |
| 15 | CDRL2 | SEQ ID NO:15 |
| 16 | CDRL3 | SEQ ID NO:16 |
| 19 | VL | DIQMTQSPSSLSASVGDRVTITCRASQEINVYLSWFQQKPGKAPKSLIYAASTL DSGVPSRFSGSGSGTDFTLTISSLQPEDFATYYCLQYVSYPYTFGQGTKLEIK |
| 30 | LC(Light chain constant region) | SEQ ID NO:30 |
| | hz4F12B5H.g1. hp | |
| 20 | CDRH1 | GFSLTGYGVT |
| 21 | CDRH2 | MIWGDASTDYNSVLKS |
| 22 | CDRH3 | DAYRYDVPLAY |
| 23 | VH | QVQLQESGPGLVKPSETLSLTCTVSGFSLTGYGVTWIRQPPGKGLEWIGMIWG DASTDYNSVLKSRVTISVDTSKNQVSLKLSSVTAADTAVYYCARDAYRYDVPL AYWGQGTLVTVSS |
| | HC(heavy chain constant region) | SEQ ID NO:29 |
| | hz4F12B5H.g2. hp | |
| 20 | CDRH1 | SEQ ID NO:20 |
| 21 | CDRH2 | SEQ ID NO:21 |
| 22 | CDRH3 | SEQ ID NO:22 |
| 24 | VH | QVQLQESGPGLVKPSETLSLTCTVSGFSLTGYGVTWIRQPPGKGLEWIGMIWG DASTDYNSVLKSRVTISKDTSKNQVSLKLSSVTAADTAVYYCARDAYRYDVPL AYWGQGTLVTVSS |
| 29 | HC(heavy chain constant region) | SEQ ID NO:29 |
| | hz4F12B5K.g0 | |
| 25 | CDRL1 | RASKSVSTSGYSNMH |
| 26 | CDRL2 | LASNLES |
| 27 | CDRL3 | LHTRELPRT |
| 28 | VL | DIVMTQSPDSLAVSLGERATINCRASKSVSTSGYSNMHWYQQKPGQPPKLLIY LASNLESGVPDRFSGSGSGTDFTLTISSLQAEDVAVYYCLHTRELPRTFGGGTK VEIK |
| 30 | LC(Light chain constant region) | SEQ ID NO:30 |
| 31 | Inno-A (CH1+CH2+CH 3, with mutations L234A, L235A, P329delP, S364R and D399K | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALA PIEKTISKAKGQPREPQVYTLPPSRDELTKNQVRLTCLVKGFYPSDIAVEWESNG QPENNYKTTPPVLKSDGSFFLYSKLTVDKSRWQQGNVFSCSVMHEALHNHYT QKSLSLSPGK |
| 32 | Inno-B(CH1+C H2+CH3,with mutations L234A, L235A, P329delP, K370S, Y349T and K409D) | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALA PIEKTISKAKGQPREPQVTTLPPSRDELTKNQVSLTCLVSGFYPSDIAVEWESNG QPENNYKTTPPVLDSDGSFFLYSDLTVDKSRWQQGNVFSCSVMHEALHNHYT QKSLSLSPGK |
| | CLDN18.2-targ eting arm HB37A6 in bispecific antibody | |
| 33 | Heavy Chain | EVQLLDSGGGLVQPGGSLRLSCAASGFTFSSYVMSWVRQAPGKGLNWVSTIS HSGGSTYYADSVKGRFTISRDNSKNTLYLQMNSLRAEDTAVYYCAIDAPYYDI LTGYRYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKVEPKSCDKTHTCPPCPAPEAAGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALAPIEKTISKAKGQPREPQVYTLPPSRDELTKNQVRLT CLVKGFYPSDIAVEWESNGQPENNYKTTPPVLKSDGSFFLYSKLTVDKSRWQQ GNVFSCSVMHEALHNHYTQKSLSLSPGK |
| 102 | Fab heavy chain | EVQLLDSGGGLVQPGGSLRLSCAASGFTFSSYVMSWVRQAPGKGLNWVSTIS HSGGSTYYADSVKGRFTISRDNSKNTLYLQMNSLRAEDTAVYYCAIDAPYYDI LTGYRYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKVEPKSC |
| 34 | Light chain/Fab light chain | DIQMTQSPSTLSASVGDRVTITCRASQSISSWLAWYQQKPGKAPKLLIYKASSL ESGVPSRFSGSGSGTEFTLTISSLQPDDFATYYCQQYNSYSYTFGQGTKLEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| | CD28-targeting arm hz5B11D8-a in bispecific antibody | |
| 35 | Heavy Chain | QVQLVQSGAEVKKPGASVKVSCKASGYTFTFYWMHWVRQAPGQGLEWMGE IDPSDSYTNYNQEFKDRVTMTVDTSTSTVYMELSSLRSEDTAVYYCARDPWYF DVWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALAPIEKTISKAKGQPREPQVTTLPPSRDELTKNQVSLTCLVSG FYPSDIAVEWESNGQPENNYKTTPPVLDSDGSFFLYSDLTVDKSRWQQGNVFS CSVMHEALHNHYTQKSLSLSPGK |
| 103 | Fab heavy chain | QVQLVQSGAEVKKPGASVKVSCKASGYTFTFYWMHWVRQAPGQGLEWMGE IDPSDSYTNYNQEFKDRVTMTVDTSTSTVYMELSSLRSEDTAVYYCARDPWYF DVWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSC |
| 36 | Light chain/Fab light chain | DIQLTQSPSFLSASVGDRVTITCSASSSITYMYWYQQKPGKAPKLLIYDTSKLAS GVPSRFSGSGSGTEFTLTISSLQPEDFATYYCHQRSSYPWTFGQGTRLEIKRTVA APSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| | CD28-targeting arm hz61FG3-a in bispecific antibody | |
| 37 | Heavy Chain | QVQLVQSGAEVKKPGASVKVSCKASGYTFTSYWMHWVRQAPGQGLEWMG NIYPGSGSTNYDEKFKSRVTMTRDTSTSTVYMELSSLRSEDTAVYYCAREHYG SIYEAMDYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNHK PSNTKVDKKVEPKSCDKTHTCPPCPAPEAAGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALAPIEKTISKAKGQPREPQVTTLPPSRDELTKNQVS LTCLVSGFYPSDIAVEWESNGQPENNYKTTPPVLDSDGSFFLYSDLTVDKSRWQ QGNVFSCSVMHEALHNHYTQKSLSLSPGK |
| 104 | Fab heavy chain | QVQLVQSGAEVKKPGASVKVSCKASGYTFTSYWMHWVRQAPGQGLEWMG NIYPGSGSTNYDEKFKSRVTMTRDTSTSTVYMELSSLRSEDTAVYYCAREHYG SIYEAMDYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNHK PSNTKVDKKVEPKSC |
| 38 | Light chain/Fab light chain | DIQMTQSPSSLSASVGDRVTITCRASQEIQGYLSWFQQKPGKAPKSLIYAASTL DSGVPSRFSGSGSGTDFTLTISSLQPEDFATYYCLQYVSYPYTFGQGTKLEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| SEQ ID NO | CD28-targeting arm hz4F12B5-b in bispecific antibody | |
| 39 | Heavy Chain | QVQLQESGPGLVKPSETLSLTCTVSGFSLTGYGVTWIRQPPGKGLEWIGMIWG DASTDYNSVLKSRVTISKDTSKNQVSLKLSSVTAADTAVYYCARDAYRYDVPL AYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALAPIEKTISKAKGQPREPQVTTLPPSRDELTKNQVSLTCLVSG FYPSDIAVEWESNGQPENNYKTTPPVLDSDGSFFLYSDLTVDKSRWQQGNVFS CSVMHEALHNHYTQKSLSLSPGK |
| 105 | Fab heavy chain | QVQLQESGPGLVKPSETLSLTCTVSGFSLTGYGVTWIRQPPGKGLEWIGMIWG DASTDYNSVLKSRVTISKDTSKNQVSLKLSSVTAADTAVYYCARDAYRYDVPL AYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSC |
| 40 | Light chain/Fab light chain | DIVMTQSPDSLAVSLGERATINCRASKSVSTSGYSNMHWYQQKPGQPPKLLIY LASNLESGVPDRFSGSGSGTDFTLTISSLQAEDVAVYYCLHTRELPRTFGGGTK VEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSF |
| SEQ ID NO | CD28-targeting arm TGN1412 in bispecific antibody | NRGEC |
| 41 | VH | QVQLVQSGAEVKKPGASVKVSCKASGYTFTSYYIHWVRQAPGQGLEWIGCIY PGNVNTNYNEKFKDRATLTVDTSISTAYMELSRLRSDDTAVYFCTRSHYGLDW NFDVWGQGTTVTVSS |
| 42 | Heavy Chain | QVQLVQSGAEVKKPGASVKVSCKASGYTFTSYYIHWVRQAPGQGLEWIGCIY PGNVNTNYNEKFKDRATLTVDTSISTAYMELSRLRSDDTAVYFCTRSHYGLDW NFDVWGQGTTVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPEAAGGPSVFLFPPKPKDTLMISRTPEVTCVV VDVSHEDPEVKFNWYVDGVEVHNAKTKPREEQYNSTYRVVSVLTVLHQDWL NGKEYKCKVSNKALAPIEKTISKAKGQPREPQVTTLPPSRDELTKNQVSLTCLV SGFYPSDIAVEWESNGQPENNYKTTPPVLDSDGSFFLYSDLTVDKSRWQQGNV FSCSVMHEALHNHYTQKSLSLSPGK |
| 43 | VL | DIQMTQSPSSLSASVGDRVTITCHASQNIYVWLNWYQQKPGKAPKLLIYKASN LHTGVPSRFSGSGSGTDFTLTISSLQPEDFATYYCQQGQTYPYTFGGGTKVEIK |
| 44 | Light Chain | DIQMTQSPSSLSASVGDRVTITCHASQNIYVWLNWYQQKPGKAPKLLIYKASN LHTGVPSRFSGSGSGTDFTLTISSLQPEDFATYYCQQGQTYPYTFGGGTKVEIKR TVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |
| 45 | Inno-A (CH1+CH2+CH 3)(IgG1 with L234A L235A S364R D399K) | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALP APIEKTISKAKGQPREPQVYTLPPSRDELTKNQVRLTCLVKGFYPSDIAVEWESN GQPENNYKTTPPVLKSDGSFFLYSKLTVDKSRWQQGNVFSCSVMHEALHNHY TQKSLSLSPGK |
| 46 | Inno-B(CH1+ CH2+CH3)(Ig G1 with L234A L235A, K370S, Y349T, K409D) | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALP APIEKTISKAKGQPREPQVTTLPPSRDELTKNQVSLTCLVSGFYPSDIAVEWESN GQPENNYKTTPPVLDSDGSFFLYSDLTVDKSRWQQGNVFSCSVMHEALHNHY TQKSLSLSPGK |
| SEQ ID NO | PSMA-targeting arm 1C3 in bispecific antibody | |
| 47 | VH | QVQLVESGGGVVQPGRSLRLSCAASGFTFSSYAMHWVRQAPGKGLEWVAVIS YDGNNKYYADSVKGRFTISRDNSKNTLYLQMNSLRAEDTAVYYCARAVPWGS RYYYYGMDVWGQGTTVTVSS |
| 48 | Heavy Chain | QVQLVESGGGVVQPGRSLRLSCAASGFTFSSYAMHWVRQAPGKGLEWVAVIS YDGNNKYYADSVKGRFTISRDNSKNTLYLQMNSLRAEDTAVYYCARAVPWGS RYYYYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYICNVN HKPSNTKVDKKVEPKSCDKTHTCPPCPAPEAAGGPSVFLFPPKPKDTLMISRTP EVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKN QVRLTCLVKGFYPSDIAVEWESNGQPENNYKTTPPVLKSDGSFFLYSKLTVDKS RWQQGNVFSCSVMHEALHNHYTQKSLSLSPGK |
| 106 | Fab heavy chain | QVQLVESGGGVVQPGRSLRLSCAASGFTFSSYAMHWVRQAPGKGLEWVAVIS YDGNNKYYADSVKGRFTISRDNSKNTLYLQMNSLRAEDTAVYYCARAVPWGS RYYYYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYICNVN HKPSNTKVDKKVEPKSC |
| 49 | VL | AIQLTQSPSSLSASVGDRVTITCRASQGISSALAWYQQKSGKAPKLLIFDASSLE SGVPSRFSGSGSGTDFTLTISSLQPEDFATYYCQQFNSYPLTFGGGTKVEIK |
| 50 | Light chain/Fab light chain | AIQLTQSPSSLSASVGDRVTITCRASQGISSALAWYQQKSGKAPKLLIFDASSLE SGVPSRFSGSGSGTDFTLTISSLQPEDFATYYCQQFNSYPLTFGGGTKVEIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| SEQ ID NO | CD28-targeting arm hz5B11D8-a in bispecific antibody | |
| 51 | Heavy Chain | QVQLVQSGAEVKKPGASVKVSCKASGYTFTFYWMHWVRQAPGQGLEWMGE IDPSDSYTNYNQEFKDRVTMTVDTSTSTVYMELSSLRSEDTAVYYCARDPWYF DVWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALPAPIEKTISKAKGQPREPQVTTLPPSRDELTKNQVSLTCLVS GFYPSDIAVEWESNGQPENNYKTTPPVLDSDGSFFLYSDLTVDKSRWQQGNVF SCSVMHEALHNHYTQKSLSLSPGK |
| 103 | Fab heavy chain | SEQ ID NO:103 |
| / | Light chain/Fab light chain | SEQ ID NO:36 |
| SEQ ID NO | CD28-targeting arm hz61FG3-a in bispecific antibody | |
| 52 | Heavy Chain | QVQLVQSGAEVKKPGASVKVSCKASGYTFTSYWMHWVRQAPGQGLEWMG |
| 104 | Fab heavy chain | SEQ ID NO:104 |
| / | Light chain/Fab light chain | SEQ ID NO:38 |
| SEQ ID NO | CD28-targeting arm hz4F12B5-b in bispecific antibody | |
| 53 | Heavy Chain | QVQLQESGPGLVKPSETLSLTCTVSGFSLTGYGVTWIRQPPGKGLEWIGMIWG DASTDYNSVLKSRVTISKDTSKNQVSLKLSSVTAADTAVYYCARDAYRYDVPL AYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALPAPIEKTISKAKGQPREPQVTTLPPSRDELTKNQVSLTCLVS GFYPSDIAVEWESNGQPENNYKTTPPVLDSDGSFFLYSDLTVDKSRWQQGNVF SCSVMHEALHNHYTQKSLSLSPGK |
| 105 | Fab heavy chain | SEQ ID NO:105 |
| / | Light chain/Fab light chain | SEQ ID NO:40 |
| SEQ ID NO | 11838P2 in bispecific antibody REGN5678 | |
| 54 | VH | QVQLVESGGGVVQPGRSLRLSCAASGFTFSSYGMHWVRQAPGKGLEWVAFM SYDGSNKFYSDSVKGRFTISRDNSRKMLFLQMNNLRAEDTAVYYCARDQYYD FLTDHGVFDYWGQGTLVTVSS |
| 55 | Heavy chain | QVQLVESGGGVVQPGRSLRLSCAASGFTFSSYGMHWVRQAPGKGLEWVAFM SYDGSNKFYSDSVKGRFTISRDNSRKMLFLQMNNLRAEDTAVYYCARDQYYD FLTDHGVFDYWGQGTLVTVSSASTKGPSVFPLAPCSRSTSESTAALGCLVKDYF PEPVTVSWNSGALTSGVHTFPAVLQSSGLYSLSSVVTVPSSSLGTKTYTCNVDH KPSNTKVDKRVESKYGPPCPPCPAPPVAGPSVFLFPPKPKDTLMISRTPEVTCVV VDVSQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRWQEGN VFSCSVMHEALHNHYTQKSLSLSLGK |
| 56 | VL | EIVLTQSPGTLSLSPGERATLSCRASQSVSSSYLAWYQQKPGQAPRLLIYGASSR ATGIPDRFSGSGSGTDFTLTISRLEPEDFAVYYCQQYGSSPWTFGQGTKVEIK |
| 57 | Light chain | EIVLTQSPGTLSLSPGERATLSCRASQSVSSSYLAWYQQKPGQAPRLLIYGASSR ATGIPDRFSGSGSGTDFTLTISRLEPEDFAVYYCQQYGSSPWTFGQGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| SEQ ID NO | CD28-targeting arm 14226P2 in bispecific antibody | |
| 58 | VH | QVQLQESGPGLVKPSETLSLTCTVSGGSISSYYWSWIRQPPGKGLEWIGYIYYS GITHYNPSLKSRVTISVDTSKIQFSLKLSSVTAADTAVYYCARWGVRRDYYYY GMDVWGQGTTVTVSS |
| 59 | Heavy chain | QVQLQESGPGLVKPSETLSLTCTVSGGSISSYYWSWIRQPPGKGLEWIGYIYYS GITHYNPSLKSRVTISVDTSKIQFSLKLSSVTAADTAVYYCARWGVRRDYYYY GMDVWGQGTTVTVSSASTKGPSVFPLAPCSRSTSESTAALGCLVKDYFPEPVT VSWNSGALTSGVHTFPAVLQSSGLYSLSSVVTVPSSSLGTKTYTCNVDHKPSNT KVDKRVESKYGPPCPPCPAPPVAGPSVFLFPPKPKDTLMISRTPEVTCVVVDVS QEDPEVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLVKGF YPSDIAVEWESNGQPENNYKTTPPVLDSDGSFFLYSRLTVDKSRWQEGNVFSC SVMHEALHNRFTQKSLSLSLGK |
| / | VL | SEQ ID NO:56 |
| / | Light chain | SEQ ID NO:57 |
| SEQ ID NO | | PSMA in bispecific antibody: 3465P |
| 60 | VH | QVQLVESGGGVVQPGRSLRLSCAASGFTFITYGMHWVRQAPGKGLEWVAIIY YDESNKYYADSVKGRFTISRDISKNTLYLQMNGLRAEDTAVYYCARAPRVAVE EDYSYYYGMDVWGQGTTVTVSS |
| 61 | Heavy chain | QVQLVESGGGVVQPGRSLRLSCAASGFTFITYGMHWVRQAPGKGLEWVAIIY YDESNKYYADSVKGRFTISRDISKNTLYLQMNGLRAEDTAVYYCARAPRVAVE EDYSYYYGMDVWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKKVEPKSCDKTHTCPPCPAPEAAGGPSVFLFPPKPKDTLMISR TPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELT KNQVRLTCLVKGFYPSDIAVEWESNGQPENNYKTTPPVLKSDGSFFLYSKLTV DKSRWQQGNVFSCSVMHEALHNHYTQKSLSLSPGK |
| 107 | Fab heavy chain | QVQLVESGGGVVQPGRSLRLSCAASGFTFITYGMHWVRQAPGKGLEWVAIIY YDESNKYYADSVKGRFTISRDISKNTLYLQMNGLRAEDTAVYYCARAPRVAVE EDYSYYYGMDVWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKKVEPKSC |
| 62 | VL/Fab light chain | DIQMTQSPSSLSASVGDRVTITCRASQGIRNDLGWYQQKPGRAPKRLIYGASN LQSGVPSRFSGSGSGTEFTLTISSLQPEDFATYYCLQHNSHPYTFGQGTKLEIK |
| / | CL | SEQ ID NO:30 |
| 63 | Light chain | DIQMTQSPSSLSASVGDRVTITCRASQGIRNDLGWYQQKPGRAPKRLIYGASN LQSGVPSRFSGSGSGTEFTLTISSLQPEDFATYYCLQHNSHPYTFGQGTKLEIKR TVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |
| SEQ ID NO | | Anti-CD3 in bispecific antibody PSMCxCD3: Hzsp34.24 |
| 64 | VH | EVQLVESGGGLVQPGGSLKLSCAASGFTFNTYAMNWVRQASGKGLEWVGRIR SKYNNYATYYADSVKDRFTISRDDSKSTLYLQMNSLKTEDTAVYYCARHGNFG QSYVSWFAYWGQGTTVTVSS |
| 65 | Heavy chain | EVQLVESGGGLVQPGGSLKLSCAASGFTFNTYAMNWVRQASGKGLEWVGRIR SKYNNYATYYADSVKDRFTISRDDSKSTLYLQMNSLKTEDTAVYYCARHGNFG QSYVSWFAYWGQGTTVTVSSASTKGPSVFPLAPSSKSTSGGTAALGCLVKDYF PEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVTTLPPSRDELTKNQ VSLTCLVSGFYPSDIAVEWESNGQPENNYKTTPPVLDSDGSFFLYSDLTVDKSR WQQGNVFSCSVMHEALHNHYTQKSLSLSPGK |
| 66 | VL | QAVVTQEPSLTVSPGGTVTLTCRSSTGAVTTSNYANWVQQKPGQAPRGLIGGT NKRAPGVPARFSGSLLGDKAALTLLGAQPEDEAEYYCALWYSNLWVFGQGTK LTVL |
| 67 | CL | GQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 68 | Light chain | QAVVTQEPSLTVSPGGTVTLTCRSSTGAVTTSNYANWVQQKPGQAPRGLIGGT NKRAPGVPARFSGSLLGDKAALTLLGAQPEDEAEYYCALWYSNLWVFGQGTK LTVLGQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKTVAPT ECS |
| | 61F3G3 sequence | |
| 69 | VH | QVQLQQSGSELVRPGASVKLSCKASGYTFTSYWMHWVKQRHGQGLEWIGNI YPGSGSTNYDEKFKSKGTLTVDTSSSTAYIHLSSLTSEDSAVYYCTREHYGSIYE AMDYWGQGTSVTVSS |
| 10 | CDRH1 | SEQ ID NO:10 |
| 11 | CDRH2 | SEQ ID NO:11 |
| 12 | CDRH3 | SEQ ID NO:12 |
| 70 | VL: | DIVMTQSPSSLSASLGERVSLTCRASQEINGYLSWLQQKPDGTIKRLIYAASTLD SGVPKRFSGSRSGSDYSLTISSLESEDFADYYCLQYVSYPYTFGGGTKLEIK |
| 71 | CDRL1 | RASQEINGYLS |
| 15 | CDRL2 | SEQ ID NO:15 |
| 16 | CDRL3 | SEQ ID NO:16 |
| | 5B11D8 sequence | |
| 72 | VH | EVQLQQSGAELVKPGASVKLSCKASGYTFTFYWMHWVKQRPGQGLEWIGEI DPSDSYTNYNQEFKDKATLTVDKSSSTAYMHLISLTSEDSAVYYCAKDPWYFD VWGAGTSVTVSS |
| 1 | CDRH1 | SEQ ID NO:1 |
| 2 | CDRH2 | SEQ ID NO:2 |
| 3 | CDRH3 | SEQ ID NO:3 |
| 73 | VL | DIVLTQSPAIMSASPGEKVTMTCSASSSITYMYWYQQKPGTSPKRWIYDTSKLA SGVPARFSGSGSGPSYSLTISSMEAEDAATYYCHQRSSYPWTFGGGTRLEIK |
| 6 | CDRL1 | SEQ ID NO:6 |
| 7 | CDRL2 | SEQ ID NO:7 |
| 8 | CDRL3 | SEQ ID NO:8 |
| | 4F12B5 sequence | |
| 75 | VH | EVQLQQSGPGLVAPSQSLSITCTVSGFSLTGYGVTWVRQPPGKGLEWLGMIWG DGSTDYNSVLKSRLSISKDNSKSQVFLKMNSLQTDDTARYYCARDGYRYDVP LAYWGQGTLVTVSS |
| 20 | CDRH1 | SEQ ID NO:20 |
| 76 | CDRH2 | MIWGDGSTDYNSVLKS |
| 77 | CDRH3 | DGYRYDVPLAY |
| 78 | VL: | DIKMTQSPASLAVSLGQRATISCRASKSVSTSGYSNMHWYQQKLGQPPKLLIY LASNLESGVPARFSGSGSGTDFTLNIHPVEEEDAATYYCLHTRELPRTFGGGTK LEIK |
| 25 | CDRL1 | SEQ ID NO:25 |
| 26 | CDRL2 | SEQ ID NO:26 |
| 27 | CDRL3 | SEQ ID NO:27 |
| 79 | IgG1 heavy chain constant region (CH1+CH2+CH 3, EEM) | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALHNH YTQKSLSLSPGK |
| 80 | Inno-A-Fc(IgG1 with mutations L234A, L235A, P329delP, S364R and D399K) | DKTHTCPPCPAPEAAGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALAPIEKTISKAKGQPREPQVYTLPPSRDELTKNQVRLTCLVKGFYPSDIAVE WESNGQPENNYKTTPPVLKSDGSFFLYSKLTVDKSRWQQGNVFSCSVMHEAL HNHYTQKSLSLSPGK |
| 81 | Inno-B-Fc(IgG1, with mutations L234A, L235A, P329delP, K370S, Y349T and K409D) | DKTHTCPPCPAPEAAGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALAPIEKTISKAKGQPREPQVTTLPPSRDELTKNQVSLTCLVSGFYPSDIAVE WESNGQPENNYKTTPPVLDSDGSFFLYSDLTVDKSRWQQGNVFSCSVMHEAL HNHYTQKSLSLSPGK |
| 82 | Inno-A-Fc(IgG1 with L234A, L235A, S364R and D399K) | DKTHTCPPCPAPEAAGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALPAPIEKTISKAKGQPREPQVYTLPPSRDELTKNQVRLTCLVKGFYPSDIAV EWESNGQPENNYKTTPPVLKSDGSFFLYSKLTVDKSRWQQGNVFSCSVMHEA LHNHYTQKSLSLSPGK |
| 83 | Inno-B-Fc(IgG1, with L234A, L235A, K370S, Y349T and K409D) | DKTHTCPPCPAPEAAGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALPAPIEKTISKAKGQPREPQVTTLPPSRDELTKNQVSLTCLVSGFYPSDIAV EWESNGQPENNYKTTPPVLDSDGSFFLYSDLTVDKSRWQQGNVFSCSVMHEA LHNHYTQKSLSLSPGK |
| 84 | Mouse PD1 antibody heavy chain | EVQLQESGPGLVKPSQSLSLTCSVTGYSITSSYRWNWIRKFPGNRLEWMGYINS AGISNYNPSLKRRISITRDTSKNQFFLQVNSVTTEDAATYYCARSDNMGTTPFT YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVD KKVEPKSCDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTCLVKG FYPSDIAVEWESNGQPENNYKTTPPVLDSDGSFFLYSKLTVDKSRWQQGNVFS CSVMHEALHNHYTQKSLSLSPGK |
| 85 | Mouse PD1 antibody light chain | DIVMTQGTLPNPVPSGESVSITCRSSKSLLYSDGKTYLNWYLQRPGQSPQLLIY WMSTRASGVSDRFSGSGSGTDFTLKISGVEAEDVGIYYCQQGLEFPTFGGGTK LELKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSF NRGEC |
| 86 | HB37A6 heavy chain variable region | EVQLLDSGGGLVQPGGSLRLSCAASGFTFSSYVMSWVRQAPGKGLNWVSTIS HSGGSTYYADSVKGRFTISRDNSKNTLYLQMNSLRAEDTAVYYCAIDAPYYDI LTGYRYWGOGTLVTVSS |
| 87 | HB37A6 heavy chain variable region CDRH1 | GFTFSSYVMS |
| 88 | HB37A6 heavy chain variable region CDRH2 | TISHSGGSTYYADSVKG |
| 89 | HB37A6 heavy chain variable region CDRH3 | DAPYYDILTGYRY |
| 90 | HB37A6 light chain variable region | DIQMTQSPSTLSASVGDRVTITCRASQSISSWLAWYQQKPGKAPKLLIYKASSL ESGVPSRFSGSGSGTEFTLTISSLQPDDFATYYCQQYNSYSYTFGQGTKLEIK |
| 91 | HB37A6 light chain variable region CDRL1 | RASQSISSWLA |
| 92 | HB37A6 light chain variable region CDRL2 | KASSLES |
| 93 | HB37A6 light chain variable region CDRL3 | QQYNSYSYT |
| 74 | CH1 | |
| 94 | IgG1 Fc (WT-DEL ) | |
| 95 | IgG1 Fc (WT-EEM ) | DKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEA LHNHYTQKSLSLSPGK |
| 96 | PSMA 1C3 heavy chain variable region CDRH1 | GFTFSSYAMH |
| 97 | PSMA 1C3 heavy chain variable region CDRH2 | VISYDGNNKYYADSVKG |
| 98 | PSMA 1C3 heavy chain variable region CDRH3 | AVPWGSRYYYYGMDV |
| 99 | PSMA 1C3 heavy chain variable region CDRL1 | RASQGISSALA |
| 100 | PSMA 1C3 heavy chain variable region CDRL2 | DASSLES |
| 101 | PSMA 1C3 heavy chain variable region CDRL3 | QQFNSYPLT |

## Claims

1. An anti-CD28 antibody or an antigen-binding fragment thereof, comprising 3 CDRs of a heavy chain variable region VH: CDRH1, CDRH2 and CDRH3, and 3 CDRs of a light chain variable region VL: CDRL1, CDRL2 and CDRL3, wherein
(i). the CDRH1, the CDRH2 and the CDRH3 are selected from three complementarity-determining regions CDRH1, CDRH2 and CDRH3 contained in a VH set forth in SEQ ID NO: 24, 23 or 75 ; and the CDRL1, the CDRL2 and the CDRL3 are three complementarity-determining regions CDRL1, CDRL2 and CDRL3 contained in a VL set forth in SEQ ID NO: 28 or 78;
(ii). the CDRH1, the CDRH2 and the CDRH3 are selected from three complementarity-determining regions CDRH1, CDRH2 and CDRH3 contained in a VH set forth in any one of SEQ ID NOs: 4, 5 and 72; and the CDRL1, the CDRL2 and the CDRL3 are three complementarity-determining regions CDRL1, CDRL2 and CDRL3 contained in a VL set forth in SEQ ID NO: 9 or 73; or
(iii). the CDRH1, the CDRH2, and the CDRH3 are selected from three complementarity-determining regions CDRH1, CDRH2, and CDRH3 contained in a VH set forth in SEQ ID NO: 13 or 69; and the CDRL1, the CDRL2, and the CDRL3 are three complementarity-determining regions CDRL1, CDRL2, and CDRL3 contained in a VL set forth in SEQ ID NO: 17, 19, or 70.

2. An anti-CD28 antibody or an antigen-binding fragment thereof, comprising a CDRH1, a CDRH2, a CDRH3, a CDRL1, a CDRL2 and a CDRL3, wherein
(i). the CDRH1 comprises or consists of the amino acid sequence of SEQ ID NO: 20; the CDRH2 comprises or consists of the amino acid sequence of SEQ ID NO: 21; the CDRH3 comprises or consists of the amino acid sequence of SEQ ID NO: 22; the CDRL1 comprises or consists of the amino acid sequence of SEQ ID NO: 25; the CDRL2 comprises or consists of the amino acid sequence of SEQ ID NO: 26; and the CDRL3 comprises or consists of the amino acid sequence of SEQ ID NO: 27;
(ii). the CDRH1 comprises or consists of the amino acid sequence of SEQ ID NO: 20; the CDRH2 comprises or consists of the amino acid sequence of SEQ ID NO: 76; the CDRH3 comprises or consists of the amino acid sequence of SEQ ID NO: 77; the CDRL1 comprises or consists of the amino acid sequence of SEQ ID NO: 25; the CDRL2 comprises or consists of the amino acid sequence of SEQ ID NO: 26; and the CDRL3 comprises or consists of the amino acid sequence of SEQ ID NO: 27;
(iii). the CDRH1 comprises or consists of the amino acid sequence of SEQ ID NO: 1; the CDRH2 comprises or consists of the amino acid sequence of SEQ ID NO: 2; the CDRH3 comprises or consists of the amino acid sequence of SEQ ID NO: 3; the CDRL1 comprises or consists of the amino acid sequence of SEQ ID NO: 6 or 74; the CDRL2 comprises or consists of the amino acid sequence of SEQ ID NO: 7; and the CDRL3 comprises or consists of the amino acid sequence of SEQ ID NO: 8; or
(iv). the CDRH1 comprises or consists of the amino acid sequence of SEQ ID NO: 10; the CDRH2 comprises or consists of the amino acid sequence of SEQ ID NO: 11; the CDRH3 comprises or consists of the amino acid sequence of SEQ ID NO: 12; the CDRL1 comprises or consists of the amino acid sequence of SEQ ID NO: 14, 18, or 71; the CDRL2 comprises or consists of the amino acid sequence of SEQ ID NO: 15; and the CDRL3 comprises or consists of the amino acid sequence of SEQ ID NO: 16.

3. The antibody or the antigen-binding fragment thereof according to claim 1 or 2, comprising a heavy chain variable region VH, wherein the heavy chain variable region
(i) comprises or consists of an amino acid sequence having at least 90%, 91%, 92%, 93%, 94%, 95%, 96%, 97%, 98%, or 99% identity to the amino acid sequence of SEQ ID NO: 24, 4, 5, 13, 23, 69, 72 or 75; or
(ii) comprises or consists of the amino acid sequence of SEQ ID NO: 24, 4, 5, 13, 23, 69, 72 or 75.

4. The antibody or the antigen-binding fragment thereof according to any one of claims 1-3, comprising a light chain variable region VL, wherein the light chain variable region
(i) comprises or consists of an amino acid sequence having at least 90%, 91%, 92%, 93%, 94%, 95%, 96%, 97%, 98%, or 99% identity to the amino acid sequence of SEQ ID NO: 28, 9, 17, 19, 70, 73 or 78; or
(ii) comprises or consists of the amino acid sequence of SEQ ID NO: 28, 9, 17, 19, 70, 73 or 78.

5. The antibody or the antigen-binding fragment thereof according to claim 1 or 2, comprising a heavy chain variable region VH and a light chain variable region VL, wherein
(i). the VH comprises or consists of an amino acid sequence set forth in SEQ ID NO: 24 or 23 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or an amino acid sequence having at least 90%, 91%, 92%, 93%, 94%, 95%, 96%, 97%, 98%, or 99% identity thereto;
(ii). the VH comprises or consists of an amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 78 or an amino acid sequence having at least 90%, 91%, 92%, 93%, 94%, 95%, 96%, 97%, 98%, or 99% identity thereto;
(iii). the VH comprises or consists of an amino acid sequence set forth in SEQ ID NO: 23 or 24 or 75 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 28 or 78 or an amino acid sequence having at least 90%, 91%, 92%, 93%, 94%, 95%, 96%, 97%, 98%, or 99% identity thereto;
(iv). the VH comprises or consists of an amino acid sequence set forth in SEQ ID NO: 4 or 5 or 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or 73 or an amino acid sequence having at least 90%, 91%, 92%, 93%, 94%, 95%, 96%, 97%, 98%, or 99% identity thereto;
(v). the VH comprises or consists of an amino acid sequence set forth in SEQ ID NO: 4 or 5 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 9 or an amino acid sequence having at least 90%, 91%, 92%, 93%, 94%, 95%, 96%, 97%, 98%, or 99% identity thereto;
(vi). the VH comprises or consists of an amino acid sequence set forth in SEQ ID NO: 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 73 or an amino acid sequence having at least 90%, 91%, 92%, 93%, 94%, 95%, 96%, 97%, 98%, or 99% identity thereto;
(vii). the VH comprises or consists of an amino acid sequence set forth in SEQ ID NO: 13 or 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or 70 or an amino acid sequence having at least 90%, 91%, 92%, 93%, 94%, 95%, 96%, 97%, 98%, or 99% identity thereto;
(viii). the VH comprises or consists of an amino acid sequence set forth in SEQ ID NO: 13 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17 or 19 or an amino acid sequence having at least 90%, 91%, 92%, 93%, 94%, 95%, 96%, 97%, 98%, or 99% identity thereto; or
(ix). the VH comprises or consists of an amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 70 or an amino acid sequence having at least 90%, 91%, 92%, 93%, 94%, 95%, 96%, 97%, 98%, or 99% identity thereto.

6. The antibody or the antigen-binding fragment thereof according to claim 1 or 2, comprising a heavy chain variable region VH and a light chain variable region VL, wherein
(i). the VH comprises or consists of an amino acid sequence set forth in SEQ ID NO: 24, and the VL comprises or consists of an amino acid sequence set forth in SEQ ID NO: 28;
(ii). the VH comprises or consists of an amino acid sequence set forth in SEQ ID NO: 23, and the VL comprises or consists of an amino acid sequence set forth in SEQ ID NO: 28;
(iii). the VH comprises or consists of an amino acid sequence set forth in SEQ ID NO: 72, and the VL comprises or consists of an amino acid sequence set forth in SEQ ID NO: 73;
(iv). the VH comprises or consists of an amino acid sequence set forth in SEQ ID NO: 4, and the VL comprises or consists of an amino acid sequence set forth in SEQ ID NO: 9;
(v). the VH comprises or consists of an amino acid sequence set forth in SEQ ID NO: 5, and the VL comprises or consists of an amino acid sequence set forth in SEQ ID NO: 9;
(vi). the VH comprises or consists of an amino acid sequence set forth in SEQ ID NO: 13, and the VL comprises or consists of an amino acid sequence set forth in SEQ ID NO: 17;
(vii). the VH comprises or consists of an amino acid sequence set forth in SEQ ID NO: 13, and the VL comprises or consists of an amino acid sequence set forth in SEQ ID NO: 19;
(viii). the VH comprises or consists of an amino acid sequence set forth in SEQ ID NO: 69, and the VL comprises or consists of an amino acid sequence set forth in SEQ ID NO: 70; or
(ix). the VH comprises or consists of an amino acid sequence set forth in SEQ ID NO: 75, and the VL comprises or consists of an amino acid sequence set forth in SEQ ID NO: 78.

7. An anti-CD28 antibody or an antigen-binding fragment thereof, comprising a heavy chain variable region VH and a light chain variable region VL, wherein
(i). the VH comprises or consists of an amino acid sequence set forth in SEQ ID NO: 24, and the VL comprises or consists of an amino acid sequence set forth in SEQ ID NO: 28;
(ii). the VH comprises or consists of an amino acid sequence set forth in SEQ ID NO: 23, and the VL comprises or consists of an amino acid sequence set forth in SEQ ID NO: 28;
(iii). the VH comprises or consists of an amino acid sequence set forth in SEQ ID NO: 72, and the VL comprises or consists of an amino acid sequence set forth in SEQ ID NO: 73;
(iv). the VH comprises or consists of an amino acid sequence set forth in SEQ ID NO: 4, and the VL comprises or consists of an amino acid sequence set forth in SEQ ID NO: 9;
(v). the VH comprises or consists of an amino acid sequence set forth in SEQ ID NO: 5, and the VL comprises or consists of an amino acid sequence set forth in SEQ ID NO: 9;
(vi). the VH comprises or consists of an amino acid sequence set forth in SEQ ID NO: 13, and the VL comprises or consists of an amino acid sequence set forth in SEQ ID NO: 17;
(vii). the VH comprises or consists of an amino acid sequence set forth in SEQ ID NO: 13, and the VL comprises or consists of an amino acid sequence set forth in SEQ ID NO: 19;
(viii). the VH comprises or consists of an amino acid sequence set forth in SEQ ID NO: 69, and the VL comprises or consists of an amino acid sequence set forth in SEQ ID NO: 70; or
(ix). the VH comprises or consists of an amino acid sequence set forth in SEQ ID NO: 75, and the VL comprises or consists of an amino acid sequence set forth in SEQ ID NO: 78.

8. The antibody or the antigen-binding fragment thereof according to any one of claims 1-7, further comprising a heavy chain constant region HC, for example, the antibody heavy chain constant region HC is a heavy chain constant region of IgG1, IgG2, IgG3, or IgG4, preferably a heavy chain constant region of human IgG1, and preferably the heavy chain constant region
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 29 or 79; or
(ii) comprises or consists of an amino acid sequence selected from SEQ ID NO: 29 or 79.

9. The antibody or the antigen-binding fragment thereof according to claim 7 or 8, wherein the heavy chain constant region comprises a mutation that reduces binding to Fcγ receptors, e.g., a LALA mutation and/or P329delP.

10. The antibody or the antigen-binding fragment thereof according to any one of claims 1-9, comprising a light chain constant region, for example, the light chain constant region is a Lambda or Kappa light chain constant region.

11. The antibody or the antigen-binding fragment thereof according to claim 10, wherein the light chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 30; or
(ii) comprises or consists of the amino acid sequence of SEQ ID NO: 30.

12. The antibody or the antigen-binding fragment thereof according to any one of claims 1-11, wherein the antibody is a humanized antibody or a chimeric antibody.

13. The antibody or the antigen-binding fragment thereof according to any one of claims 1-12, wherein the antibody is a monoclonal antibody.

14. The antibody or the antigen-binding fragment thereof according to any one of claims 1-13, wherein the antigen-binding fragment is an antibody fragment selected from the group consisting of a Fab, a Fab', a Fab'-SH, a Fv, a single-chain antibody (e.g., scFv), and (Fab')₂, a single-domain antibody (e.g., VHH), a domain antibody (dAb), or a linear antibody.

15. The antibody or the antigen-binding fragment thereof according to any one of claims 1-11, wherein the antibody is a bispecific antibody or a multispecific antibody comprising a first binding specificity for CD28 and additional binding specificities for one or more tumor-associated antigens.

16. The antibody or the antigen-binding fragment thereof according to claim 15, wherein the tumor-associated antigen is selected from the group consisting of CLDN18.2 and PSMA.

17. A bispecific antibody, comprising a first antigen-binding region and a second antigen-binding region,
wherein the first antigen-binding region specifically binds to CD28 and comprises a VH and a VL, wherein
the VH comprises CDRH1, CDRH2 and CDRH3 as defined in claim 1 or 2, and the VL comprises CDRL1, CDRL2 and CDRL3 as defined in claim 1 or 2; or
the VH and VL are the VH and VL as defined in any one of claims 3-7;
the second antigen-binding region specifically binds to a tumor-associated antigen TAA or an immune checkpoint molecule.

18. The bispecific antibody according to claim 17, wherein the first antigen-binding region is the Fab of the anti-CD28 antibody as defined in any one of claims 1-14.

19. The bispecific antibody according to claim 17 or 18, wherein the second antigen-binding region is a Fab specifically binding to a tumor-associated antigen or an immune checkpoint molecule.

20. The bispecific antibody according to claim 18 or 19, wherein the Fab as the first antigen-binding region or the second antigen-binding region comprises a CH1, wherein the CH1 is a CH1 from IgG1, IgG2, IgG3, or IgG4, preferably a CH1 from IgG1.

21. The bispecific antibody according to claim 20, wherein the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 74; or
(ii) comprises or consists of the amino acid sequence of SEQ ID NO: 74.

22. The bispecific antibody according to any one of claims 18-21, wherein the Fab serving as the first antigen-binding region or the second antigen-binding region comprises a light chain constant region, wherein the light chain constant region is a Kappa light chain constant region or a Lambda light chain constant region.

23. The bispecific antibody according to claim 22, wherein the Kappa light chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 30; or
(ii) comprises or consists of the amino acid sequence of SEQ ID NO: 30.

24. The bispecific antibody according to any one of claims 17-23, wherein the bispecific antibody is an IgG-like bispecific antibody comprising an Fc dimer, wherein two Fc regions constituting the Fc dimer are identical or different; optionally, the Fc regions are human IgG1, IgG2, IgG3, or IgG4 Fc regions; optionally, the Fc regions
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 94 or 95; or
(ii) comprises or consists of the amino acid sequence of SEQ ID NO: 94 or 95.

25. The bispecific antibody according to claim 24, wherein the two Fc regions are different, wherein based on the Innobody technology, mutations are introduced into the first Fc region and the second Fc region to promote heterodimerization between the first Fc region and the second Fc region.

26. The antibody or the antigen-binding fragment thereof according to claim 25, wherein the CH3 of one Fc region comprises S364R and D399K mutations, and the CH3 of the other Fc region comprises Y349T, K370S, and K409D mutations.

27. The bispecific antibody according to any one of claims 24-26, wherein the first and/or second Fc region comprises mutations that reduce binding to Fcγ receptors, e.g., mutations L234A/L235A, and optionally P329delP.

28. The bispecific antibody according to claim 26 or 27, wherein
a) one Fc region comprises or consists of an amino acid sequence set forth in SEQ ID NO: 80, and the other Fc region comprises or consists of an amino acid sequence set forth in SEQ ID NO: 81;
b) one Fc region comprises an amino acid sequence having at least 85%, 90%, 95%, 96%, 97%, 98%, or 99% identity to the amino acid sequence set forth in SEQ ID NO: 80 and comprises mutations S364R and D399K, and the other Fc region comprises an amino acid sequence having at least 85%, 90%, 95%, 96%, 97%, 98%, or 99% identity to the amino acid sequence set forth in SEQ ID NO: 81 and comprises mutations Y349T, K370S, and K409D;
c) one Fc region comprises an amino acid sequence having at least 85%, 90%, 95%, 96%, 97%, 98%, or 99% identity to the amino acid sequence set forth in SEQ ID NO: 80, and comprises mutations S364R and D399K and mutations L234A/L235A and P329delP, and the other Fc region comprises an amino acid sequence having at least 85%, 90%, 95%, 96%, 97%, 98%, or 99% identity to the amino acid sequence set forth in SEQ ID NO: 81, and comprises mutations Y349T, K370S and K409D and mutations L234A/L235A and P329delP;
d) one Fc region comprises or consists of an amino acid sequence set forth in SEQ ID NO: 82, and the other Fc region comprises or consists of an amino acid sequence set forth in SEQ ID NO: 83;
e) one Fc region comprises an amino acid sequence having at least 85%, 90%, 95%, 96%, 97%, 98%, or 99% identity to the amino acid sequence set forth in SEQ ID NO: 82 and comprises mutations S364R and D399K, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, and K409D; or
f) one Fc region comprises an amino acid sequence having at least 85%, 90%, 95%, 96%, 97%, 98%, or 99% identity to the amino acid sequence set forth in SEQ ID NO: 82 and comprises mutations S364R, D399K, and L234A/L235A, and the other Fc region polypeptide comprises an amino acid sequence having at least 85%, 90%, 95%, 96%, 97%, 98%, or 99% identity to the amino acid sequence set forth in SEQ ID NO: 83 and comprises mutations Y349T, K370S, K409D, and L234A/L235A.

29. The bispecific antibody according to any one of claims 17-28, wherein the TAA is CLDN18.2, and preferably, the second antigen-binding region comprises three complementarity-determining regions from a heavy chain variable region (CDRHs): CDRH1, CDRH2 and CDRH3; and three complementarity-determining regions from a light chain variable region (CDRLs): CDRL1, CDRL2 and CDRL3, wherein
the CDRH1 comprises or consists of the amino acid sequence of SEQ ID NO: 87; the CDRH2 comprises or consists of the amino acid sequence of SEQ ID NO: 88, the CDRH3 comprises or consists of the amino acid sequence of SEQ ID NO: 89, the CDRL1 comprises or consists of the amino acid sequence of SEQ ID NO: 91, the CDRL2 comprises or consists of the amino acid sequence of SEQ ID NO: 92, and the CDRL3 comprises or consists of the amino acid sequence of SEQ ID NO: 93.

30. The bispecific antibody according to claim 29, wherein the second antigen-binding region comprises a VH and a VL, wherein the VH comprises or consists of a sequence set forth in SEQ ID NO: 86; and the VL comprises or consists of a sequence set forth in SEQ ID NO: 90.

31. The bispecific antibody according to any one of claims 17-28, wherein the TAA is PSMA;
preferably, the second antigen-binding region comprises three complementarity-determining regions from a heavy chain variable region (CDRHs): CDRH1, CDRH2, and CDRH3; and three complementarity-determining regions from a light chain variable region (CDRLs): CDRL1, CDRL2, and CDRL3, wherein
the CDRH1 comprises or consists of the amino acid sequence of SEQ ID NO: 96; the CDRH2 comprises or consists of the amino acid sequence of SEQ ID NO: 97; the CDRH3 comprises or consists of the amino acid sequence of SEQ ID NO: 98;; the CDRL1 comprises or consists of the amino acid sequence of SEQ ID NO: 99; the CDRL2 comprises or consists of the amino acid sequence of SEQ ID NO: 100; and the CDRL3 comprises or consists of the amino acid sequence of SEQ ID NO: 101.

32. The bispecific antibody according to claim 31, wherein the second antigen-binding region comprises a VH and a VL, wherein the VH comprises or consists of a sequence set forth in SEQ ID NO: 47; and the VL comprises or consists of a sequence set forth in SEQ ID NO: 49.

33. The bispecific antibody according to any one of claims 17-32, wherein the bispecific antibody comprises a first antigen-binding region specifically binding to CD28 and a second antigen-binding region specifically binding to TAA, wherein the first antigen-binding region comprises or is a first Fab, and the second antigen-binding region comprises or is a second Fab, wherein the first Fab is linked at the C-terminus of its CH1 to the N-terminus of the first Fc region (directly or via a linker such as a hinge region), and the second Fab is linked at the C-terminus of its CH1 to the N-terminus of the second Fc region (directly or via a linker such as a hinge region).

34. The bispecific antibody according to claim 33, wherein the bispecific antibody is an IgG-like antibody having a configuration shown in FIG. 3.

35. The bispecific antibody according to claim 33 or 34, comprising a first Fab as a first antigen-binding region specifically binding to CD28 and a second Fab as a second antigen-binding region specifically binding to TAA, wherein the bispecific antibody comprises
heavy chain 1: comprising or consisting of, from N-terminus to C-terminus, a first Fab heavy chain variable region-a heavy chain constant region CH1-a first Fc region, wherein the heavy chain constant region CH1 is linked at its C-terminus to the N-terminus of the first Fc region directly or via a linker (e.g., a hinge region);
light chain 1: comprising or consisting of, from N-terminus to C-terminus, a first Fab light chain variable region-a light chain constant region;
heavy chain 2: comprising or consisting of, from N-terminus to C-terminus: a second Fab heavy chain variable region-a heavy chain constant region CH1-a second Fc region, wherein the heavy chain constant region CH1 is linked at its C-terminus to the N-terminus of the second Fc region directly or via a linker (e.g., a hinge region); and/or
light chain 2: comprising or consisting of, from N-terminus to C-terminus: a second Fab light chain variable region-a light chain constant region;
preferably, each domain is directly linked.

36. The bispecific antibody according to claim 35, wherein the TAA is CLDN18.2, and wherein
the heavy chain 1 comprises or consists of an amino acid sequence set forth in SEQ ID NO: 35 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto;
the heavy chain 1 comprises or consists of an amino acid sequence set forth in SEQ ID NO: 37 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto; or
the heavy chain 1 comprises or consists of an amino acid sequence set forth in SEQ ID NO: 39 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 33 or an amino acid sequence having at least 85%, 90%, 95%, 96%, 97%, 98%, or 99% identity thereto; and the light chain 2 comprises or consists of an amino acid sequence set forth in SEQ ID NO: 34 or an amino acid sequence having at least 85%, 90%, 95%, 96%, 97%, 98%, or 99% identity thereto.

37. The bispecific antibody according to claim 35, wherein the TAA is PSMA, and wherein
the heavy chain 1 comprises or consists of an amino acid sequence set forth in SEQ ID NO: 51 or an amino acid sequence having at least 85%, 90%, 95%, 96%, 97%, 98%, or 99% identity thereto; the light chain 1 comprises or consists of an amino acid sequence set forth in SEQ ID NO: 36 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto;
the heavy chain 1 comprises or consists of an amino acid sequence set forth in SEQ ID NO: 52 or an amino acid sequence having at least 85%, 90%, 95%, 96%, 97%, 98%, or 99% identity thereto; the light chain 1 comprises or consists of an amino acid sequence set forth in SEQ ID NO: 38 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto; or
the heavy chain 1 comprises or consists of an amino acid sequence set forth in SEQ ID NO: 53 or an amino acid sequence having at least 85%, 90%, 95%, 96%, 97%, 98%, or 99% identity thereto; the light chain 1 comprises or consists of an amino acid sequence set forth in SEQ ID NO: 40 or an amino acid sequence having at least 85%, 90%, 95%, 96%, 97%, 98%, or 99% identity thereto; the heavy chain 2 comprises or consists of an amino acid sequence set forth in SEQ ID NO: 48 or an amino acid sequence having at least 85%, 90%, 95%, 96%, 97%, 98%, or 99% identity thereto; and the light chain 2 comprises or consists of an amino acid sequence set forth in SEQ ID NO: 50 or an amino acid sequence having at least 85%, 90%, 95%, 96%, 97%, 98%, or 99% identity thereto.

38. A nucleic acid molecule, encoding any one of the chains of the antibody or the antigen-binding fragment thereof according to any one of claims 1-16 or any one of the chains in the bispecific antibody according to any one of claims 17-37, or consisting of a nucleic acid sequence.

39. An expression vector, comprising the nucleic acid molecule according to claim 38, preferably, the expression vector is pCDNA, such as pCDNA3.1.

40. A host cell, comprising the nucleic acid molecule according to claim 38 or the expression vector according to claim 39, preferably, the host cell is prokaryotic or eukaryotic, e.g., a 293 cell or a CHO cell, such as an Expi293 cell.

41. A method for preparing the antibody or the antigen-binding fragment thereof according to any one of claims 1-16 or the bispecific antibody according to any one of claims 17-37, the method comprising culturing the host cell comprising the nucleic acid molecule according to claim 38 or the expression vector according to claim 39 under conditions suitable for expressing the chains of the antibody, and optionally recovering the antibody from the host cell (or a host cell medium).

42. An immunoconjugate, comprising the antibody or the antigen-binding fragment thereof according to any one of claims 1-16 or the bispecific antibody according to any one of claims 17-37.

43. A pharmaceutical composition or a medicament or a formulation, comprising the antibody or the antigen-binding fragment thereof according to any one of claims 1-16 or the bispecific antibody according to any one of claims 17-37, or the immunoconjugate according to claim 42, and optionally a pharmaceutical supplementary material.

44. A pharmaceutical combination product, comprising the antibody or the antigen-binding fragment thereof according to any one of claims 1-16 or the bispecific antibody according to any one of claims 17-37, or the immunoconjugate according to claim 42, and one or more additional therapeutic agents, wherein preferably, the therapeutic agents are various therapeutic agents for treating tumors, such as chemotherapeutic agents, angiogenesis inhibitors, cytokines, cytotoxic agents, other antibodies, small molecule drugs, or immunomodulatory agents (e.g., immune checkpoint inhibitors or agonists); preferably, the additional antibody is a PD-1 antibody.

45. A method for preventing or treating a tumor in a subject, comprising administering to the subject an effective amount of the antibody or the antigen-binding fragment thereof according to any one of claims 1-16 or the bispecific antibody according to any one of claims 17-37, or the immunoconjugate according to claim 42, or the pharmaceutical composition or formulation according to claim 43, or the pharmaceutical combination product according to claim 44.

46. The method according to claim 45, wherein the tumor is a solid tumor or a hematological tumor, such as a TAA-positive tumor or cancer; optionally, the tumor is a CLDN18.2-positive gastrointestinal tumor, e.g., a gastrointestinal cancer, such as gastric cancer, breast cancer, colon cancer, rectal cancer, or colorectal cancer; or the tumor is a PSMA-positive tumor, e.g., renal cell carcinoma, such as breast cancer, lung cancer, rectal cancer, or colorectal cancer.

47. The method according to any one of claims 45-46, further comprising administering in combination with an additional therapy such as a therapeutic modality (e.g., surgical therapy or radiotherapy) and/or an additional therapeutic agent, preferably, the therapeutic agent is any therapeutic agent for treating a tumor, such as a chemotherapeutic agent, an angiogenesis inhibitor, a cytokine, a cytotoxic agent, an additional antibody, a small molecule drug, or an immunomodulatory agent (e.g., an immune checkpoint inhibitor or an agonist); preferably, the additional antibody is a PD-1 antibody.
